(19) Europäisches Patentamt
European Patent Office
Office européen des brevets

(11) **EP 2 319 439 B1**

(12) **EUROPEAN PATENT SPECIFICATION**

(45) Date of publication and mention
of the grant of the patent:
**26.07.2017 Bulletin 2017/30**

(21) Application number: **10192301.9**

(22) Date of filing: **22.02.2006**

(51) Int Cl.:
*A61L 24/04* (2006.01)    *A61L 27/26* (2006.01)
*C08L 33/12* (2006.01)    *A61B 17/16* (2006.01)
*A61B 17/88* (2006.01)    *B01F 7/30* (2006.01)
*B01F 15/02* (2006.01)    *A61F 2/46* (2006.01)
*B01F 15/00* (2006.01)

(54) **Materials for treating bone**

Materialien zur Behandlung von Knochen

Matières pour traiter un os

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **22.02.2005 US 654495 P**
**01.08.2005 US 194411**
**28.09.2005 US 721094 P**
**28.09.2005 US 720725 P**
**25.10.2005 US 729505 P**
**22.11.2005 US 738556 P**
**26.01.2006 US 762789 P**
**26.01.2006 US 763003 P**
**02.02.2006 US 765484 P**
**31.07.2005 PCT/IL2005/000812**

(43) Date of publication of application:
**11.05.2011 Bulletin 2011/19**

(62) Document number(s) of the earlier application(s) in
accordance with Art. 76 EPC:
**06711221.9 / 1 850 797**

(73) Proprietor: **DePuy Spine, Inc.**
**Raynham, MA 02767 (US)**

(72) Inventors:
• **Beyar, Mordechay**
**38900, Caesarea (IL)**
• **Globerman, Oren**
**46910, Kfar-shmaryahu (IL)**
• **Shavit, Ronen**
**69413, Tel-Aviv (IL)**
• **Wachsler-Avrahami, Hila**
**69400, Tel-Aviv (IL)**

(74) Representative: **Curran, Clair et al**
**Urquhart-Dykes & Lord LLP**
**Arena Point**
**Merrion Way**
**Leeds LS2 8PA (GB)**

(56) References cited:
**WO-A-02/072156    US-A- 4 093 576**
**US-A- 4 341 691**

• **LEWIS GLADIUS: "Properties of acrylic bone
cement: State of the art review", JOURNAL OF
BIOMEDICAL MATERIALS RESEARCH, WILEY,
NEW YORK, NY, US, vol. 38, no. 2, 1 January 1997
(1997-01-01), pages 155-182, XP002432739, ISSN:
0021-9304**

**Description**

FIELD OF THE INVENTION

The present invention relates to viscous materials.

BACKGROUND TO THE INVENTION

A common occurrence in older persons is compression fractures of the vertebrae, causing both pain and a shortening (or other distortion) of stature. One common treatment is vertebroplasty, in which cement is injected into a fractured vertebra. While this treatment fixes the fracture and reduces pain, it does not restore the vertebra and person to their original height. Another problem is that the cement is injected in a liquid phase so that it may be unintentionally injected outside of the vertebra and/or may migrate out through cracks in the vertebra. This may cause considerable bodily harm.

Another common treatment is kyphoplasty, in which the fracture is reduced, for example by first inflating a balloon inside the vertebra and then injecting a fixing material and/or an implant. The problem of cement migration is reduced, but not avoided, as a lower pressure can be used to inject the cement.

In general, polymeric cements becomes more viscous as the polymer chain grows by reacting directly with the double bond of a monomer. Polymerization begins by the "addition mechanism" in which a monomer becomes unstable by reacting with an initiator, a volatile molecule that is most commonly a radical (molecules that contain a single unpaired electron). Radicals bond with monomers, forming monomer radicals that can attack the double bond of the next monomer to propagate the polymer chain. Because radicals are so transient, initiators are often added in the form of an unreactive peroxide form that which is stable in solution. Radicals are formed when heat or light cleaves the peroxide molecule. For applications in which high temperatures are not practical (such as the use of bone cement in vivo), peroxide is cleaved by adding a chemical activator such as N,N-dimethyl-p-toluidine. (Nussbaum DA et al: "The Chemistry of Acrylic Bone Cement and Implication for Clinical Use in Image-guided Therapy", J Vase Interv Radiol (2004); 15:121-126).

Viscous cement is advantageous not only in reducing the risk of its leakage, but also, because of its ability to infiltrate into the intravertebral cancellous bone (inter-digitation) [see G Baroud et al, Injection biomechanics of bone cements used in vertebroplasty, Bio-Medical Materials and Engineering 00 (2004) 1-18]. Baroud also suggests that about 95% of the applied injection pressure is required to overcome friction in the cannula. In addition, viscous material may reduce the fracture.

Examples of commercially available viscous bone cements include, but are not limited to, CMW (trade mark) Nos. 1, 2 and 3 (DePuy Orthopaedics Inc.; Warsaw, IN, USA) and Simplex (trade mark) -P and -RO (Stryker Orthopaedics; Mahwah, NJ, USA). These cements are characterized by a liquid phase after mixing and prior to achieving a viscosity of 500 Pa.s. In a typical use scenario, these previously available cements are poured, while in a liquid phase, into a delivery device.

There have also been attempts to reduce cement migration by injecting more viscous cement, for example, during the doughing time and at the start of polymerization. However, the injection methods suggested require higher pressures for the more viscous material. Also, some types of viscous materials, such as hardening PMMA, have a small workability window at high viscosities, as they harden very quickly once they reach a high viscosity. This has generally prevented very viscous materials and the associated very high pressures from being used. One possible reason is that as pressures increase, the physician is prevented from receiving feedback on the resistance of the body to the injection of the cement. Thus, over-injection can easily occur.

Some fixing materials, such as polymethylmethacrylate (PMMA), emit heat and possibly toxic materials while setting. These may further weaken the bone and possibly cause the cement to loosen and/or the bone to fracture.

It has recently been suggested that some fixing materials, being harder than bone, induce fractures in nearby bones.

It is also known to use bone-like repair materials, such as a slurry of bone chips, which apparently do not induce such fractures. However, injecting such materials is difficult due to their viscosity.

US-4969888, US-5108404, US-6383188, US-A-2003/0109883, US-A-2002/ 068974, US-6348055, US-6383190, US-4494535, US-4653489 and US-4653487disclose various tools and methods for treating bone.

An additional manner to deliver bone cement into the vertebra is using a tamping instrument (US-6241734 and US-6613054), comprising a cannula and a rod, which urges the material within the cannula into the bone.

US-A-2004/260303 teaches an apparatus for delivering bone cement into a vertebra.

Cannulae with working sleeves are described, for example, in US-6241734 and US-6613054. US-4341691 discloses a bone cement comprising a first powder component which contains pre-polymerised beads and a second liquid component which contains a monomer.

## SUMMARY OF THE INVENTION

The invention relates to formulations of bone cement which provide a window of time during which the cement is suitably viscous for injection. More specifically, the invention provides a bone cement according to claim 1. Preferred embodiments are defined by the dependent claims.

## BRIEF DESCRIPTION OF THE FIGURES

Exemplary non-limiting embodiments of the invention will be described with reference to the following description of embodiments in conjunction with the figures. Identical structures, elements or parts which appear in more than one figure are generally labeled with a same or similar number in all the figures in which they appear, in which:

Fig. 1A is a general flowchart of a process of treating a compression fracture;
Fig. 1B is a more detailed flowchart of a process of treating a compression fracture;
Fig. 2 shows a composite tool for accessing a vertebra;
Figs. 3A-3F show stages of a method of treatment according to Figs 1A and 1B;
Figs. 4A and 4B illustrate basic material delivery systems;
Figs. 5A and 5B show details of material extruder tips;
Fig. 5C shows an elongated and curved extrusion of material;
Figs. 6A-6C illustrate narrowing lumen sections of a delivery system;
Fig. 7A illustrates a hydraulic delivery system;
Figs. 7B and 7C show alternative methods of providing hydraulic power to the system of Fig. 7A;
Figs. 7D and 7E illustrate an exemplary hydraulic system including a disposable unit;
Figs. 7F-7G illustrate an exemplary hydraulic delivery system;
Fig. 7H illustrates an exemplary hydraulic delivery system;
Fig. 7I is a cross sectional view of a hydraulic actuator for a delivery system, the inset showing a portion of the actuator in greater detail;
Figs. 7J and 7K are exploded and cross sectional views respectively of a portion of a hydraulic actuator for a delivery system;
Fig. 7L is an exploded view of a pressure release valve for a delivery system;
Figs. 7M, 7N and 7O are cross sectional views and a side view of a pressure release valve for a delivery system in operation;
Figs. 7P and 7Q are cross sectional views of a cement reservoir illustrating a floating piston;
Figs. 7R and 7S are cross sectional views of a cement reservoir illustrating assembly of a distal injection port;
Fig. 7S1 is a cross sectional view of a cement reservoir;
Fig. 7T is a perspective view of a foot operated actuator suitable for use in a delivery system;
Fig. 7U is a cut away view of a foot operated actuator suitable for use in a delivery system;
Figs. 7V, 7W, 7X and 7Y are additional views of a foot operated actuator suitable for use in a delivery system;
Fig. 8A shows a cassette based delivery system;
Fig. 8B is a detail showing the delivery of unit element;
Figs. 9A and 9B show a material pusher with reduced material twisting;
Fig. 10A- 10F show sleeve based material pushers;
Figs. 11A and 11B show squeeze based delivery systems;
Fig. 12A and 12B illustrate a one step access and delivery system;
Fig. 12C shows an over-the-wire delivery system; and
Fig. 13 is a graph showing compressibility of a material;
Figs. 14A and 14B are exploded and perspective views respectively of an exemplary apparatus for mixing viscous material;
Figs. 14C1, 14C2, 14C3 and 14C4 are a series of top views illustrating a travel path of a mixing element within a mixing well of the apparatus of Figs. 14A and 14B;
Fig. 15 is a graph of viscosity (Pa.s) as a function of time (minutes) for a cement according to the invention and an exemplary prior art cement;
Figs. 16 and 17 are graphs indicating viscosity as Newtons of applied force per unit displacement (mm) under defined conditions for exemplary cements according to the invention and illustrate the time window for injection which is both early and long;
Figs. 18, 19, 20 and 21 are perspective views of an embodiment of a transfer apparatus for loading a viscous material into a container; and
Figs. 22, 23, 24, 25 and 26 illustrate an additional embodiment of a transfer apparatus.

DETAILED DESCRIPTION OF EMBODIMENTS

Overview of exemplary process

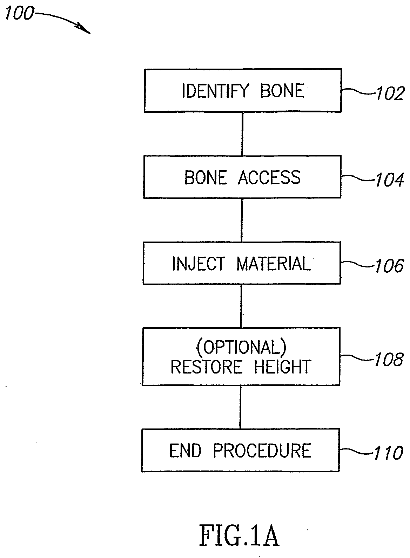

Fig. 1A is a general flowchart 100 of a process of treating a compression fracture, using apparatus of the invention.

At 102, a bone to be treated is identified. In the case of a vertebra, this usually involves X-ray or CT images to identify a vertebra or other bone that is fractured, for example by a compression fracture. The following description focuses on vertebral compression fractures but some embodiments of the invention are not limited to such cases.

In an embodiment of the invention, the access is minimally invasive, for example, only a single channel is formed into the body. Optionally, the procedure is carried out via a cannula having a diameter of, for example of 5 mm, 4 mm or less in diameter is inserted into the body. In some cases, multiple openings into the body are formed. The procedure can also be carried out using a surgical or key-hole incision; however, this may require a longer recuperation period by the patient. Optionally, the cannula (and corresponding length of a delivery tube described below) is at least 50 mm, 70 mm, 100 mm or more or intermediate or smaller values.

At 104, the vertebra is accessed.

At 106, a material, having a high viscosity in some embodiments of the invention, is injected into the vertebra. Optionally, the vertebra is fractured due to weakening caused by osteoporosis or other pathological conditions. At 108, material is optionally provided in a manner and/or amount which restores at least part of the height of the vertebra, for example, 20%, 40%, 50% or intermediate or a higher percentage of a pre-compression height. A particular feature of some embodiments of the invention is that the provided material is of sufficient viscosity or sufficiently solid that leakage from the vertebra is reduced or prevented, as compared to liquid PMMA cement. A pressure used to advance the material may be higher than what is known in the art to match the increased viscosity.

At 110, the procedure is completed and the tube is removed.

Exemplary bone access set

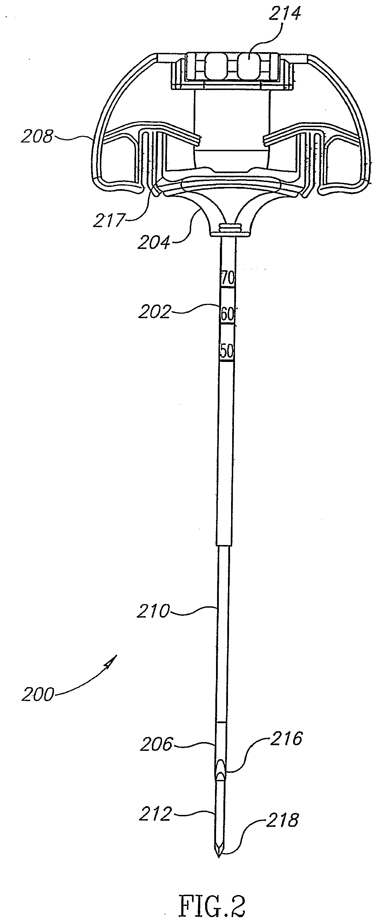

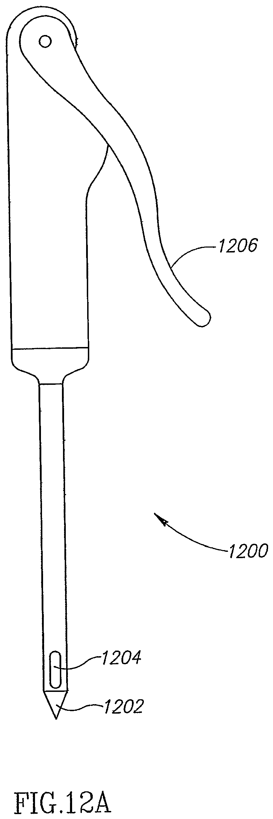

Before going into the details of the procedure, the tools used are first described. Fig. 2 shows a composite tool 200 optionally used for accessing the bone, in accordance with an embodiment of the invention. In an embodiment of the invention, the access tools used comprise a set of component tools that interlock to act, selectively, as a single tool or as separate tools, hi an embodiment of the invention, this composite set/tool serves as a one step access system in which only a single insertion of objects into the body is required. Optionally, as described below, the delivery system is also inserted at the same time. Optionally, a cannula portion of the tool is omitted, for example as described in the embodiments of Figs. 12A-12C.

In an embodiment of the invention, the components of tool 200 are coaxially matched components, which fit one within the lumen of the next.

An optional cannula 202 comprises a handle 204 and a body including a lumen.

An optional drill tool 206 includes an elongate body adapted for drilling and a handle 208. Optionally, handle 208 selectively rotationally locks to handle 204, for manipulation using a single hand, optionally using a snap-lock 217. The body of tool 206 fits in the lumen of cannula 202. Optionally, a section 210 of tool 206 is marked to be visible on an x-ray image, even in contrast to cannula 202. Optionally, this allows the difference in diameters between cannula 202 and drill tool 206 to be minimal. Absent such a marker, in some cases, the difference in diameters may not be visible on an x-ray image and the two tools cannot be distinguished.

An optional guidewire 212 is provided inside a lumen of drill tool 206. Optionally, a knob or other control 214 is provided for selective advancing and/or retracting of guidewire 212 relative to drill 216. The knob may be marked with relative or absolute positions.

Optional depth marking are provided on cannula 202.

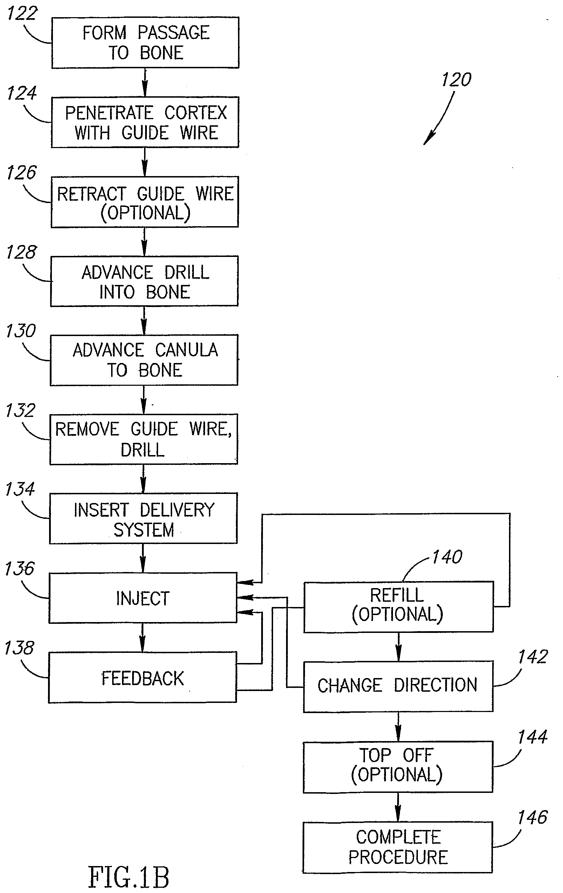

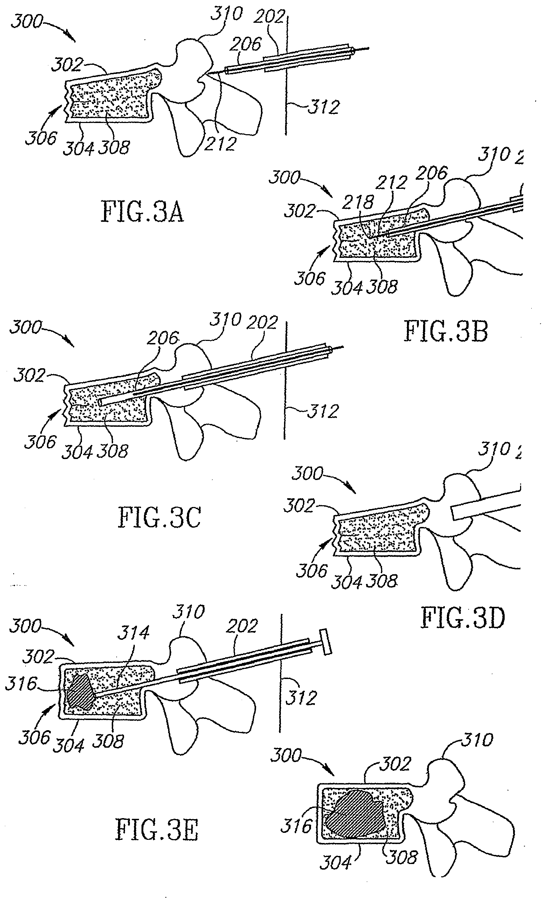

An exemplary use of these tools will be described below, in which Figs. 3A-3F schematically show the progress as a vertebra 300 having a compression fracture 306 is being treated, paralleling a detailed flowchart 120 shown in Fig. 1B.

Penetrate to bone

At 122 (Fig. 1B), a passage is formed to the bone through a skin layer 312 and intervening tissue, such as muscle and fat. Optionally, the passage is formed by advancing composite tool/set 200 until a tip 218 of guidewire 212 contacts the bone. In some embodiments, tip 218 is designed to drill in soft tissue (e.g., includes a cutting edge). Alternatively or additionally, tip 218 includes a puncturing point adapted to form a puncture in soft tissue.

This is shown in Fig. 3A. Also shown are cortical plates 302 and 304 of the vertebra and a cancellous bone interior 308.

A single pedicle 310 is shown, due to the view being cross-sectional. Optionally, the access to the vertebra is via a pedicle. Optionally, the access is via both pedicles. Optionally, an extrapedicular approach is used. Optionally, the access point or points are selected to assist in an even lifting of the vertebra.

Penetrate bone

At 124, tip 218 penetrates through the cortex of the bone being treated (Fig. 3B). In an embodiment of the invention, tip 218 is separately manipulated from the rest of composite tool 200. Optionally, tip 218 is advanced until it contacts a far side of the vertebra.

In an embodiment of the invention, tip 218 of guidewire 212 is formed to drill in bone and is advanced through the vertebral cortex by rotation or vibration. Optionally, it is advanced by tapping thereon or applying pressure thereto.

Optionally, a relative position of the guidewire and the cannula is noted, to assist in determining the inner extent of the vertebra.

At 126, the guidewire is optionally retracted. Optionally, the guidewire is axially locked to drill tool 206. Optionally, guidewire 212 and drill tool 206 align so that tip 218 and a tip 216 of the drill tool form a single drilling tip.

At 128, drill tool 206 is advanced into the bone (Fig. 3C). Optionally, tip 216 of drill tool 206 designed for drilling and/or is advanced, for example, by tapping, rotation and/or vibration. Optionally, the drill tool is advanced to the far side of the vertebra. Optionally, the previous depth marking of the guidewire is used to limit this advance. Optionally, the guidewire is not retracted at 126. Instead, drill tool 206 is advanced over the guidewire until it reaches the end of the guidewire.

At 130, cannula 202 is optionally advanced to the bone over the drill. Optionally, the leading edge of the cannula is threaded or otherwise adapted to engage the bone at or about the bore formed by the drill tool. Optionally, the cannula is inserted into the bone.

At 132, the guidewire and/or drill tool are optionally removed (Fig. 3D). In some embodiments, the cannula is not advanced all the way to the bone. In others, the cannula may be advanced into the bone, for example, to prevent contact between the treatment and cortical bone and/or weak or fractured bone. Optionally, the cannula is advanced past the pedicle and to the vertebral interior 308.

Optionally, a reamer (not shown) is inserted into the cannula and used to remove tissue from interior 308.

Inject material

At 134, a material delivery system 314 is provided into cannula 202 (shown in Fig. 3E). Optionally, the delivery system delivers material to a side thereof (described below).

At 136, system 134 is activated to inject material 316 into interior 308. Fig. 3E shows that when enough material is injected, vertebral height may be partially or completely restored. The injected material may partially or completely compress interior 308.

Feedback

At 138, feedback is optionally provided to an operator, to decide if injection is completed. Optionally, feedback is provided by fluoroscopic imaging of the site. However, other imaging methods may be used.

Optionally, non-imaging feedback is provided, for example a pressure inside the vertebra, using a pressure sensor (not shown), or using an indicator (visual or audio) for the amount of material injected.

Optionally, the feedback is used to decide if the procedure is progressing as desired, e.g., desired amount of height restoration (if any), verify a lack of material leakage, determine symmetry or asymmetry and/or the presence of new fractures in bone.

Repeat and/or change

Optionally, the material is provided in a magazine having a fixed amount (described below). If that magazine is finished and additional material is required, a refill may be provided (140), for example by replacing the magazine with a new one.

Optionally, a property of the delivery of material is changed, for example one or more of a delivery pressure, a delivery rate, an amount of delivery when delivery is in discrete units, a viscosity, composition and/or type of the delivered material, a pre-heating or pre-cooling of the material, a location of provision inside the vertebra, a spatial pattern of provision and/or a direction of provision in the vertebra.

Optionally, the direction of provision of the material is changed (142), for example to assist in maintaining symmetry of lifting or to point in the injection of material away from a fracture or towards an empty space. Optionally, the direction of provision is changed by rotating delivery system 314. Alternatively or additionally, injection is continued through a new access hole in the vertebra. Optionally, the cannula is moved axially.

Optionally, a different material is used to top off the procedure, for example, a cement which sets to a hardened condition (e.g., PMMA) is used to seal the entry hole and/or stiffen the non-hardening material (144).

Complete procedure

At 146, the tools are removed. Fig. 3F shows vertebra 300 after the procedure is completed. Optionally, the entry incision is sealed, for example, using tissue glue or a suture.

Exemplary basic delivery system

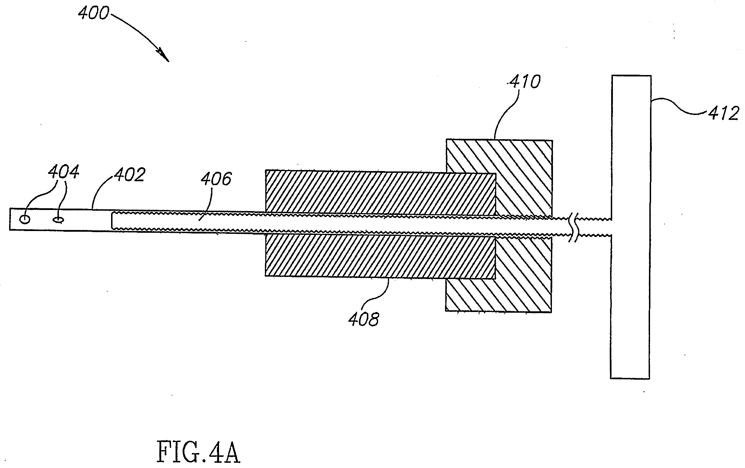

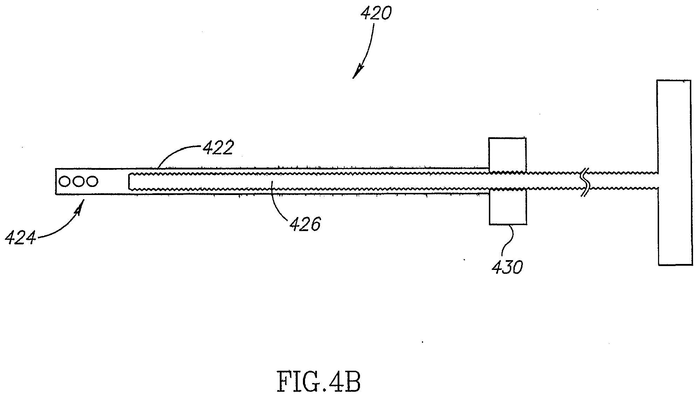

Figs. 4 A and 4B illustrate basic delivery systems, in accordance with embodiments of the invention

Fig. 4A is a cross-sectional view of a delivery system 400, comprising generally of a delivery tube 402 having one or more extrusion apertures 404. Optionally, the distal end of tube 402 is sealed. Alternatively it may be at least partially open, so forward injection of material is provided. It is noted that when the end is sealed, there may be less force acting to retract the delivery system from the vertebra. Material inside tube 402 is advanced by a threaded pusher 406.

In the design shown, tube 402 is attached to a barrel 408 with a permanent or temporary attachment method. Threading (not shown) may be provided inside of barrel 408, to match the threading on pusher 406. Alternatively (not shown), the inner diameter of barrel 408 is greater than that of tube 402. Optionally, barrel 408 and/or tube 402 serve as a reservoir of material.

A body 410 which acts as a nut and includes an inner threading engages pusher 406. In an embodiment of the invention, when a handle 412 of pusher 402 is rotated (while holding on to body/nut 410), pusher 406 is advanced, injecting material out of apertures 404 into the body. Optionally, barrel 408 is detachable from body 410, for example, for replacing barrel 408 with a material-filled barrel, when one barrel is emptied. The coupling can be, for example, a threading or a quick connect, for example, a rotate-snap fit. Optionally, tube 402 is detachable from barrel 408, for example using the same type of coupling.

In an embodiment of the invention, when the distal tip of pusher 406 goes past apertures 404 (in embodiments where it is that long), the passage cuts the material in front of the pusher away from the material exiting the aperture, releasing the exiting material from the delivery system.

Fig. 4B shows an alternative embodiment of a delivery system, 420, in which a different design of apertures 424 is used. In the embodiment, a delivery tube 422 serves as a barrel and storage for the material and is optionally detachable from a threaded nut body 430. Optionally, tube 422 is long enough to include an amount of material sufficient for injection, for example, 8 to 10 cm$^3$. Optionally; body 430 includes a pistol or other grip (not shown) and, as above, may be threaded to engage a pusher 426.

In an embodiment of the invention, the delivery system is made of metal, for example, stainless steel. Alternatively or additionally, at least some of the components are made of a polymer material, for example, PEEK, PTFE, Nylon and/or polypropylene.

Optionally, one or more components are formed of coated metal, for example, a coating with Teflon to reduce friction.

In an embodiment of the invention, the threading of the pusher is made of Nitronic 60 (Aramco) or Gall-Tough (Carpenter) stainless steels.

In an embodiment of the invention, instead of a standard threading, a ball screw is used. Optionally, the use of a ball screw increases energy efficiency and makes operation easier for manual systems as shown in Fig. 4 A and 4B. Optionally, a gasket is provided to separate the balls from the material.

In an embodiment of the invention, the delivered material is provided as an elongate sausage with a diameter similar to that of the delivery tube and/or aperture(s). Optionally, a long delivery tube is provided. Alternatively, a plurality of such strings/sausages are implanted. Optionally, the material is provided in a diameter smaller than that of the delivery tube, for example, 0.1-0.01 mm smaller so that there is reduced friction.

Exemplary extrusion details

Referring back to Fig. 4A, it is noted that the more proximal extrusion aperture 404 is optionally smaller than the more distal one. Optionally, the relative sizes are selected so that the extrusion rate and/or forces at the two holes is the same. Alternatively, the holes are designed so that the rates and/or forces are different. Referring to Fig. 4B, three axially spaced apertures may be provided and the profile of extrusion can be that a greatest extrusion and/or force is applied at the middle hole.

In an embodiment of the invention, the sizes of apertures are selected so that the total amount of material ejected is as desired, taking into account the possible sealing of some of the apertures by the advance of the pusher.

In an embodiment of the invention, the apertures are designed so that the extruded material is ejected perpendicular to the delivery system. Optionally, the delivery system is shaped so that the ejection is at an angle, for example, an angle in the plane of the axis and/or an angle in a plane perpendicular to the axis. Optionally, the angle is selected to offset forces which tend to push the delivery system out of the vertebra. Alternatively or additionally, the angle is selected to match a desired lifting direction of the vertebra or, for example, to prevent direct lifting by the extruded material. Optionally, the delivery system is inserted at a desired angle into the vertebra. Optionally, the angles of different apertures, for example, apertures on opposite sides of the delivery tube, are different, for example, defining a 180° angle between the apertures on opposite sides or a more acute (towards the proximal side) or oblique angle. In an embodiment of the invention, the extrusion angle is 30°, 45°, 60°, 80° or smaller, intermediate or larger angles to the tube axis. Optionally, the material is extruded with a bend radius of 1 mm, 2 mm, 3 mm, 4 mm, 5 mm, 10 mm or intermediate, smaller or larger radii.

The radial arrangement of the extrusion apertures can be of various designs. In one example, for example to ensure even filling of space 308, three, four or more axial rows of apertures are provided. Each row can have, for example, one, two, three or more apertures. In another example, apertures are provided only on opposing sides, so that, for example, a user can select if to extrude towards cortical plates 302 and/or 304, or not.

Rather than rows, a staggered arrangement may be used. One possible advantage for a staggered arrangement is that the delivery tube may be overly weakened by aligned rows of apertures.

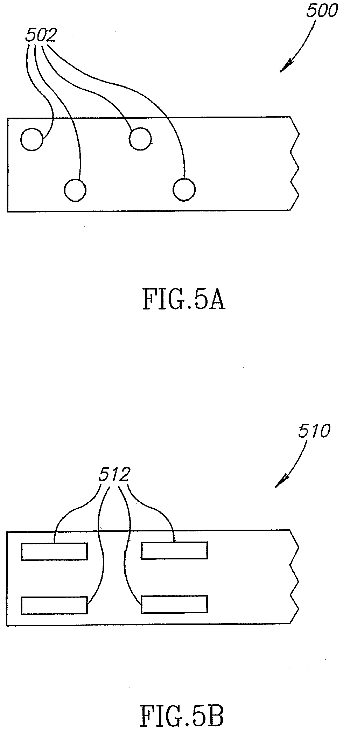

Fig. 5A shows a design of a delivery tip 500 in which round apertures 502 in a staggered row design are used. Fig. 5B shows a design of a delivery tip 510 in which elongated rectangular apertures 512 are arranged in a non-staggered manner.

As shown, the shape of the apertures can be various, for example, round, ellipsoid, rectangular, axially symmetric or asymmetric, parallel to the tube axis or not and/or elongate. Optionally, the edges of the apertures are jagged. Optionally, the shape of the apertures is selected for one or more of the following reasons: shape of extrusion, preventing failure of the aperture and/or preventing failure of the delivery tip. Optionally, the apertures have a lip (optionally pointing inwards), which may assist in shaping the extrusion. For example, the lip may be between 0.1 and 1 mm in width, for example, 0.3 mm or 0.5 mm.

In an embodiment of the invention, the delivery tube is rigid. Optionally, the delivery tube is flexible or is mechanically shaped (e.g., using a vise) before insertion. In an embodiment of the invention, the cannula is flexible and allows the insertion of a delivery tube which is curved at its end.

In an embodiment of the invention, the type of delivery tip used is selected by a user. Optionally, the delivery tip is replaceable, for example attached by a threading to the delivery system.

Optionally, an overtube or ring is selectively provided over part of the delivery system to selectively block one or more of the apertures.

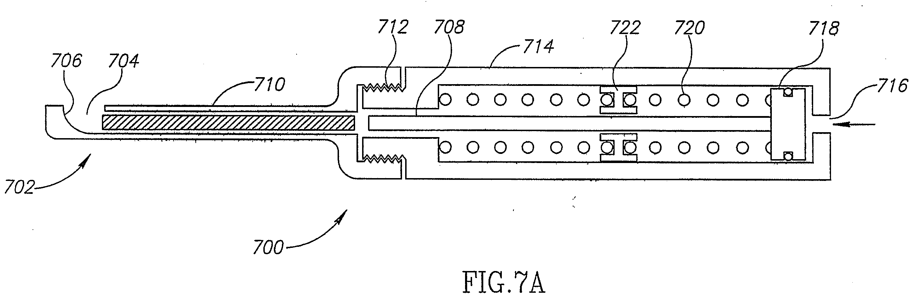

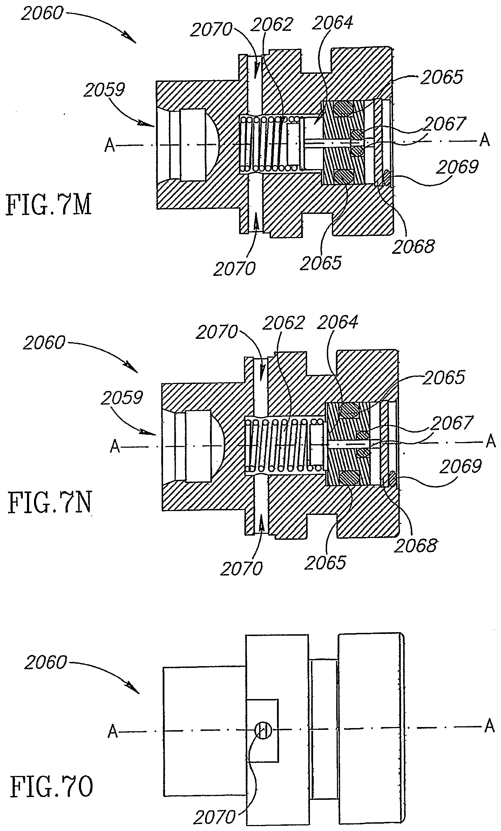

Referring briefly to Fig. 7 A, a closed distal tip 702 of cannula 710 is shown, in which a guiding incline 706 is provided to guide the ejected material out of a lateral aperture 704. Optionally, the use of such an incline reduces turbulence in the flow/distortion of the material and/or may assist in reducing friction and/or improving control over the shape of the extrusion. Also to be noted is that material extrusion is provided on only one side of the delivery system. This may allow better control over the force vectors inside the vertebra, caused by the extrusion. In an embodiment of the invention, the angles defined by the guiding incline (90° and in the plane of the tube axis) help determine the extrusion direction.

Also shown in Fig. 7A is a non-twisting pusher 708, which may reduce turbulence, friction and/or other difficulties in extruding the material, such as voids.

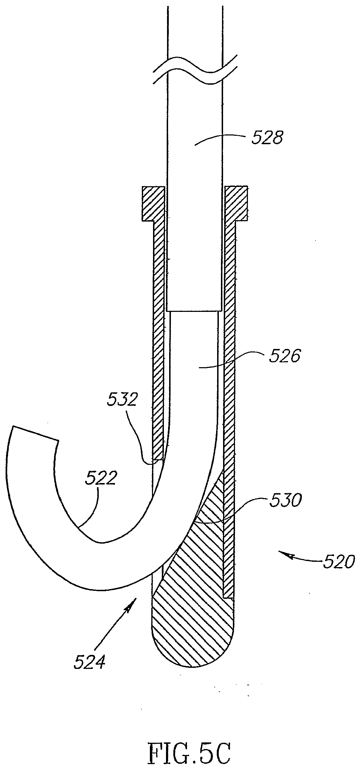

Fig. 5C shows a cannula delivery tip 520, from which a material 526 is extruded by a pusher 528 in a curved extrusion shape 522. In an embodiment of the invention, the curvature is controlled by controlling the relative friction on a proximal side 532 and on a distal side 530 of a lateral aperture 524. Alternatively or additionally, the degree of curvature depends on the size of the aperture and the shape of the incline. Optionally, the material is plastically deformed by the extrusion and may maintain a shape conferred thereby barring contact with a deforming surface (e.g., a bone plate). In an embodiment of the invention, a distal tip of the cannula is closed, optionally permanently closed, so that cement 522 is forced laterally outwards via aperture 524.

Alternatively or additionally, extrusion 522 can be curved or bent due to axial or rotational motion of tip 520. Optionally, the rotation is used to more uniformly fill space 308.

In an embodiment of the invention, the delivery tube moves and/or rotates during delivery. Optionally, a gear mechanism couples movement of the pusher with rotation and/or axial motion of the tube. Optionally, a manual motion is provided by an operator. Optionally, a vibrator is coupled to the delivery system.

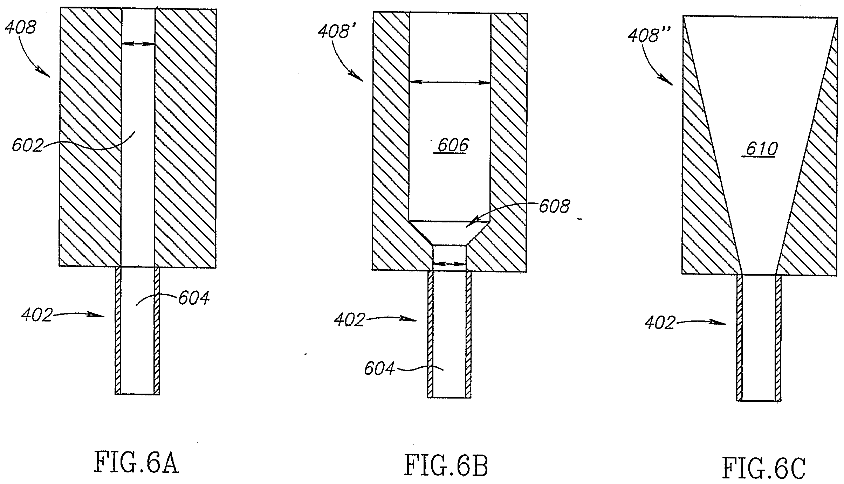

One consideration mentioned above, is that the amount of material in barrel 408 may not be sufficient for a complete procedure. A matching design is illustrated in Fig. 6A, in which the diameter of an inner lumen 602 of barrel 408 is the same as the diameter of an inner lumen 604 of delivery tube 402. A longer delivery tube/barrel maybe required to reduce the number of barrel changes.

Fig. 6B shows an alternative design, in which a barrel 408' has a lumen 606 with a greater inner diameter and thus a greater storage volume. Optionally, the greater diameter provides an additional hydraulic amplification factor as the diameter changes. Optionally, a sudden change in diameter may cause turbulence, resistance and/or void creation. In some materials, diameter change requires compression of the material. Optionally, as shown, a gradual change in diameter is provided, with an intermediate sloped section 608 with an inner diameter varying between the diameters of lumen 606 and 604. Optionally, the pusher has a diameter matching lumen 606 and does not fit into lumen 604. Optionally, an extension is provided to the pusher, which extension does fit in lumen 604.

Referring to Fig. 6C, a gradually changing lumen 610 is provided in a barrel 408". Optionally, the distal end of the pusher is made of a flexible material, which can conform to the change in diameter. Optionally, the flexible material is harder than the injected material. Alternatively or additionally, the distal end of the pusher is shaped to match the geometry of lumen 610.

In an embodiment of the invention, the lumen of the barrel is larger than the diameter of the pusher, at least in a proximal section of the barrel. After the pusher advances an amount of material into the bone, the pusher is retracted and the material remaining in the barrel is rearranged so that the next advance of the pusher will advance it. Optionally, the rearranging is by advancing a second plunger having a diameter similar to that of the barrel. Optionally, this plunger is coaxial with the pusher.

The delivery tube may have various cross-sectional shapes, for example, circular, rectangular, arcuate and/or square. Optionally, the cross-section is matched to the shape of extrusion apertures. Optionally, the inside of the apertures is made sharp to cut the extruded material as it is advanced, instead of or in addition to plastically deforming or shearing it.

Exemplary Viscosity/Plasticity and Pressure

In an embodiment of the invention, the viscosity of the bone cement is 500, optionally 1,000, optionally 1,500, optionally 2,000 Pa.s or lesser or greater or intermediate values at the time of loading to an injection system, optionally 3, or 2 or 1 minutes or lesser or intermediate values after mixing. Optionally, a cement with viscosity in this range is useful in vertebral repair, for example in vertebroplasty and/or kyphoplasty procedures. In an embodiment of the invention, use of a cement which is viscous at the time of injection reduces the risk of material leakage. Reduced leakage optionally contributes to increased likelihood of a positive clinical outcome.

In an embodiment of the invention, cement is sufficiently viscous to move the bone as it is injected. Optionally, moving of the bone contributes to fracture reduction and/or restoration of vertebral height.

In an embodiment of the invention, the provided material has a viscosity of above 600 Pa.s. Optionally, the material is advanced into the body using a pressure of at least 40 atmospheres or higher, for example, 100 or 200 atmospheres or more. If the material is plastic, it may have a hardness, for example, of between 10A shore and 100A shore and/or a Young's modulus higher than 200 MPa.

In an embodiment of the invention, pressure requirements are relaxed at a beginning of a procedure, for example, if a void is created by bone access or by rotation of the delivery system.

In an embodiment of the invention, the outer diameter of the delivery system is, for example, 2 mm, 3 mm, 4 mm, 5 mm or intermediate or smaller or larger diameters. Optionally, the wall thickness of the delivery system is 0.2 or 0.3 mm. Optionally, the wall thickness increases towards the distal tip

It should be noted that the pressure used for delivery may depend on one or more of: the friction between the material and the delivery system, the length of material being pushed, the pressure applied to the material, the pressure desired to be applied by the material to the vertebra, the manner in which the extrusion applies pressure against the vertebra, the viscosity of the material, an area of contact between the material and the cylinder and/or other causes of resistance to motion of the material.

Lower pressures may be used, for example, if it is deemed that the vertebra may be damaged or material leakage possible. The volume injected may be, for example, 2-4 cc for a typical vertebra and as high as 8 to 12 $cm^3$ or higher. Other volumes may be appropriate, depending for example, on the volume of space 308 and the desired effect of the injection.

In an embodiment of the invention, the rate of injection is 0.25 cm3.s$^{-1}$.

Higher or lower rates may be provided, for example, between 25 and 0.1 $cm^3$.s$^{-1}$ or less, and between 25 and 1 $cm^3$.s$^{-1}$ or more. Optionally, the rate is controlled using electronic or mechanical circuitry. Optionally, the rate is decided by an operator responsive to expected or imaged bone deformation in response to the pressure. Optionally, the rate is changed over the length of the procedure, for example, being higher at a beginning and lower at an end. Optionally, the rate of injection is controlled by the operator (or automatically) responsive to a feedback mechanism, such as fluoroscopy.

Fast setting cement

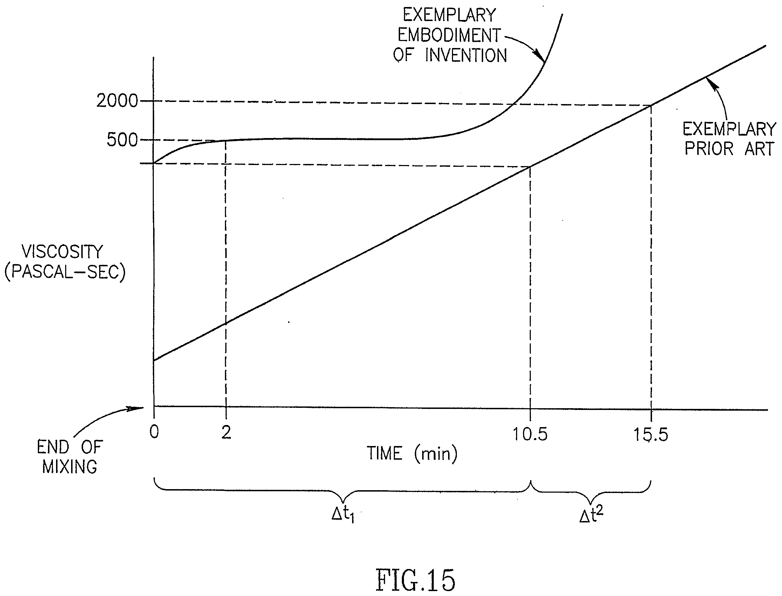

Fig. 15 is a plot of viscosity as a function of time for an exemplary bone cement according to the present invention. The figure is not drawn to scale and is provided to illustrate the principles of the invention. The end of a mixing process is denoted as time 0. In an embodiment of the invention, bone cement according to the invention enters a plastic phase upon mixing so that it has substantially no liquid phase. Optionally, mixture of polymer and monomer components produces a material with viscosity of about 500 Pascal-second or more within 15, optionally 30, optionally 60, optionally 90, optionally 120 seconds or lesser or greater or intermediate times. In an embodiment of the invention, the material reaches a viscosity higher than 500 Pa·s within about 30 seconds after its components mixing. Once a high viscosity is achieved, the viscosity remains relatively stable for 5, optionally 8 minutes or more. In an embodiment of the invention, this interval of stable viscosity provides a window of opportunity for performance of a medical procedure. In an embodiment of the invention, stable viscosity means that the viscosity of the cement changes by less than 200 Pa·s during a window of at least 2 minutes optionally at least 4 minutes after mixing is complete. Optionally, the window begins 1, 2, 3, 6, or 8 minutes after mixing is complete or lesser or intermediate times. In an embodiment of the invention, the viscosity of the cement remains below 1500 Pa·s for at least 4, optionally at least 6, optionally at least 8 optionally at least 10 minutes or intermediate or greater times.

In an embodiment of the invention, an applied injection pressure in the cement reservoir is reduced as the cement flows through the cannula. Optionally, friction between the cement and the cannula walls reduces pressure.

In an embodiment of the invention, injection of cement is continuous. The term continuous as used here indicates that the greatest interruption in a flow of cement exiting a distal tip of the cannula is less than 5, optionally less than 2, optionally less than 1, optionally less than 0.5, optionally less than 0.1 seconds or lesser or intermediate times.

In Fig. 15 the end of a mixing process is denoted as time zero. Mixing is deemed to end when all acrylic polymer beads have been wetted with monomer.

For purposes of comparison, the graph illustrates that an exemplary prior art cement reaches a viscosity suitable for injection at a time of approximately 10.5 minutes post mixing and is completely set by about 15.5 minutes.

A window of opportunity for injection with an exemplary cement according to the invention ($\Delta t_1$) is both longer and earlier than a comparable window for an exemplary prior art cement ($\Delta t_2$). Optionally, ($\Delta t_1$) begins substantially as soon as mixing is complete. In an embodiment of the invention, the cement has substantially no time in a liquid phase before entering a plastic phase.

Viscosity measurements over time for exemplary fast setting cements

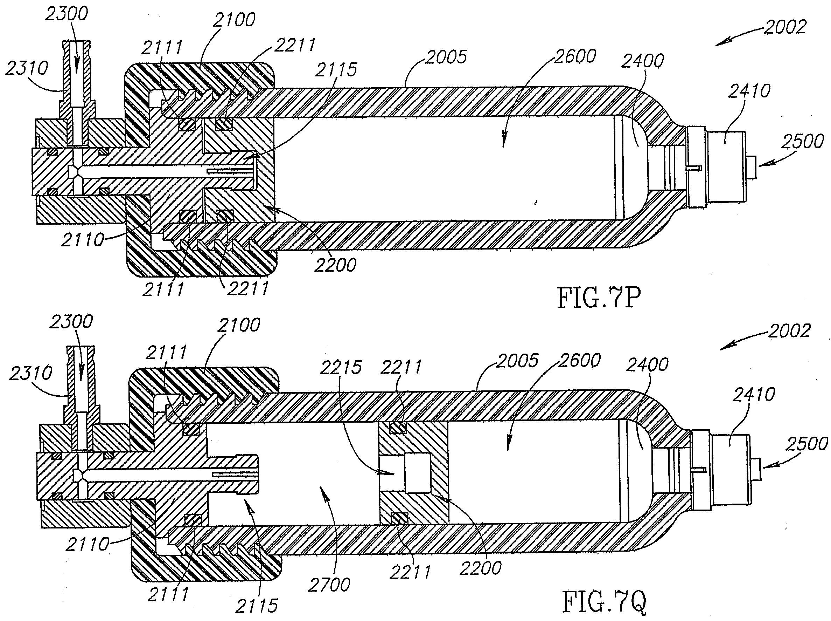

In order to evaluate the viscosity profile of different exemplary batches of cement according to some embodiments of the invention, a bulk of pre-mixed bone cement is placed inside a Stainless Steel injector body (e.g. 2002 of Fig. 7P and 7Q). Krause et al. described a method for calculating viscosity in terms of applied force. ("The viscosity of acrylic bone cements", Journal of Biomedical Materials Research, (1982): 16:219-243).

In the experimental apparatus inner diameter of injection chamber 2600 is approximately 18 mm. The distal cylindrical outlet 2500 has inner diameter of approximately 3 mm and a length of more than 4 mm. This configuration simulates a connection to standard bone cement delivery cannula/Jamshidi needle. A piston 2200 applies force (F), thus causing the bone cement to flow through outlet 2500. Piston 2200 is set to move with constant velocity of approximately 3 mm·min$^{-1}$. As a result, piston deflection is indicative of elapsed time.

The experimental procedure serves as a kind of capillary extrusion rheometer. The rheometer measures the pressure difference from a end to end of the capillary tube. The device is made of a 18mm cylindrical reservoir and a piston. The distal end of the reservoir consist of 4 mm long 3 mm hole. Assuming steady flow, isothermal conditions and incompressibility of the tested material, the viscose force resisting the motion of the fluid in the capillary is equal to the applied force acting on the piston measured by a load cell. Results are presented as force vs displacement. As displacement rate was constant and set to 3 mm·min$^{-1}$, the shear rate was constant as well. In order to measure the time elapses from test beginning, the displacement rate is divided by 3 (jog speed).

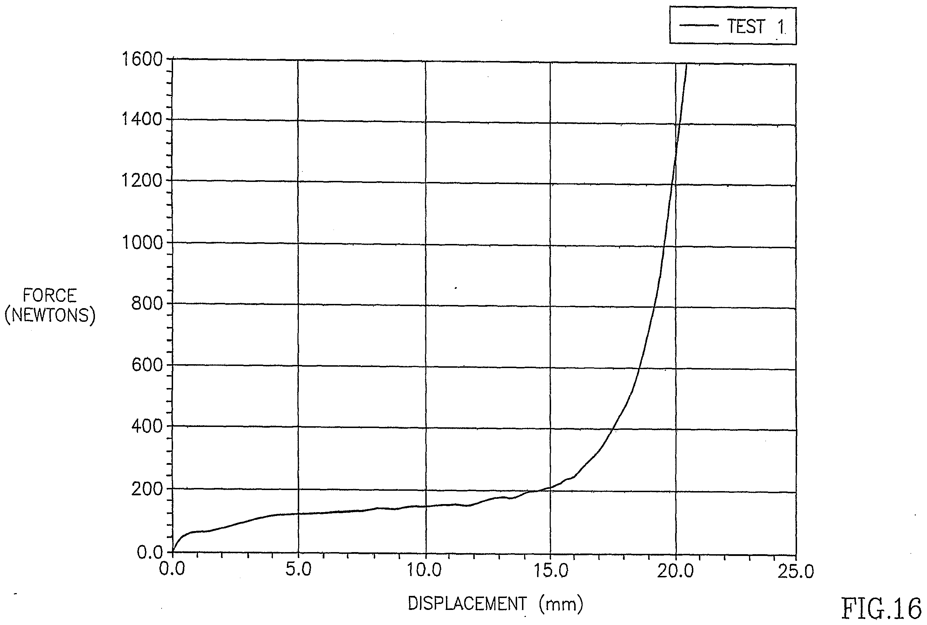

Fig.16 indicates a viscosity profile of a first exemplary batch of cement according to the invention as force (Newtons) vs displacement (mm). The cement used in this experiment included a liquid component containing approximately 98.5% v/v MMA, approximately 1.5% DMPT and approximately 20 ppm of hydroquinone and a powder component containing approximately 69.39% w/w PMMA, approximately 30.07% barium sulphate and approximately 0.54% of benzoyl peroxide. The average molecular weight of the PMMA was approximately 110,000 Dalton. Approximately 1% of the PMMA had a molecular weight of 700,000 to 1,000,000 Dalton. The bead size was in the range of 10 to 200 $\mu$m.

In this test (Average temperature: 22.3°C; Relative Humidity: app. 48%) the cement was mixed for 30-60 seconds, then manipulated by hand and placed inside injector 2002. Force was applied via piston 2200 approximately 150 seconds after end of mixing, and measurements of force and piston deflection were taken.

At a time of 2.5 minutes after mixing (0 mm deflection) the force applied was higher than 30 N.

At a time of 6.5 minutes after mixing (12 mm deflection) the force applied was about 150 N.

At a time of 7.5 minutes after mixing (15 mm deflection) the force applied was higher than 200 N.

At a time of 8.5 minutes after mixing (18 mm deflection) the force applied was higher than 500 N.

At a time of 9.17 minutes after mixing (20 mm deflection) the force applied was higher than 1300 N.

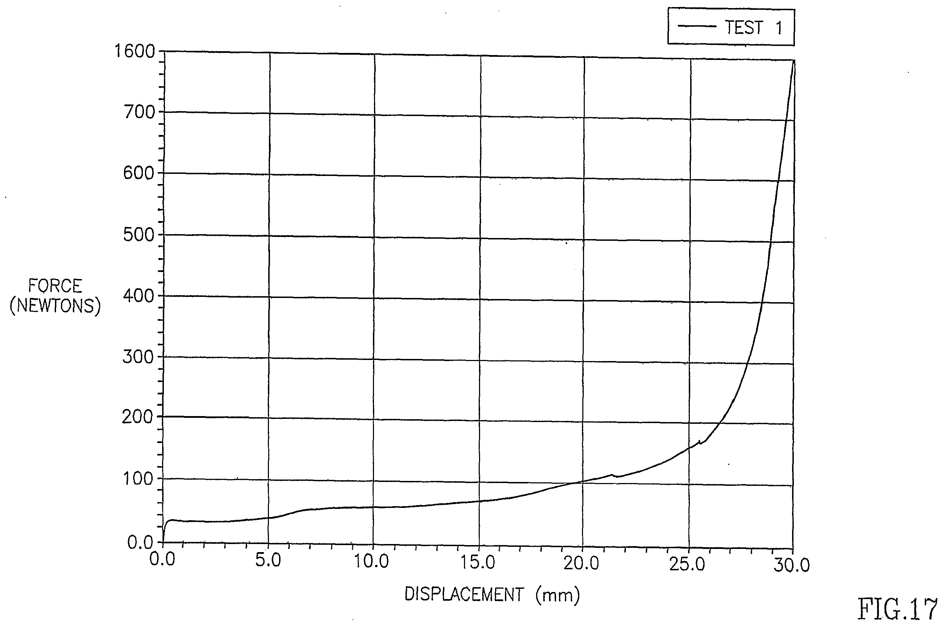

Fig. 17 indicates a viscosity profile of an additional exemplary batch of cement according to the invention as force (Newtons) vs displacement (mm). The cement in this test was prepared according to the same formula described for the experiment of Fig. 16. In this test (Average 21.1 °C; Relative Humidity: app. 43%) the cement was mixed for approximately 45 seconds, then manipulated by hand and placed inside injector 2002. Force was applied via piston 2200 approximately 150 seconds after end of mixing, and measurements of force and piston deflection were taken.

At a time of 2.25 minutes after mixing (0 mm deflection) the force applied was higher than 30 N.

At a time of 8.25 minutes after mixing (18 mm deflection) the force applied was about 90 N.

At a time of 10.3 minutes after mixing (25 mm deflection) the force applied was higher than 150 N.

At a time of 11.4 minutes after mixing (28.5 mm deflection) the force applied was higher than 500 N.

At a time of 12.25 minutes after mixing (30 mm deflection) the force applied was higher than 800 N.

Results shown in Figs. 16 and 17 and summarized hereinabove illustrate that exemplary bone cements according to some embodiments the invention are ready for injection in as little as 2.25 minutes after mixing is completed. Alternatively or additionally, these cements are characterized by short mixing time (i.e. transition to plastic phase in 30 to 60 seconds). The exemplary cements provide a "working window" for injection of 4.5 to 6.3 minutes, optionally longer if more pressure is applied. These times correspond to delivery volumes of 14.9 and 20.8 ml respectively. These volumes are sufficient for most vertebral repair procedures. These results comply with the desired characteristics described in Fig. 15. Differences between the two experiments reflect the influence of temperature and humidity on reaction kinetics.

Hydraulic material provision system

Fig. 7 A shows a delivery system 700 which is powered hydraulically. A cannula 710 is filled with material to be ejected into the body. Cannula 710 is optionally detachable via a connection 712 to a body 714. Optionally, the connection is by threading. Alternatively, a fast connection method, such as a snap connection, is used.

Body 714 converts hydraulic pressure provided via an input port 716 into an advance of a pusher rod 708. Optionally, body 714 is integral with tube 710, but this prevents replacing tube 710 when the material to be ejected is exhausted.

In an embodiment of the invention, incoming hydraulic (or pneumatic) fluid pushes against a piston 718, which advances pusher 708 directly. Optionally, a hydraulic advantage is provided by the ratios of the piston and the pusher. Optionally, a spring 720 is provided for retracting pusher 708 when the fluid pressure is released.

Optionally, one or more spacers 722 are provided surrounding pusher 708, to prevent buckling thereof. Optionally, the spacers are mounted on spring 720. Optionally, spacers are provided at several axial locations. Alternatively to spacers, fins may extend from pusher 708 to body 714.

Optionally, in use, when material is used up, pressure is reduced, pusher 708 retracts and delivery tube 710 is replaced. Optionally, a barrel filled with material for injection, separate from tube 710 is provided, so that tip 702 does not need to be removed from the body.

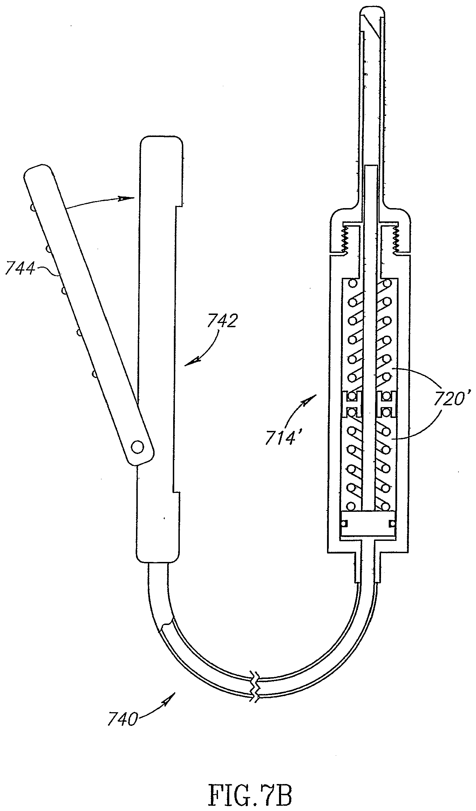

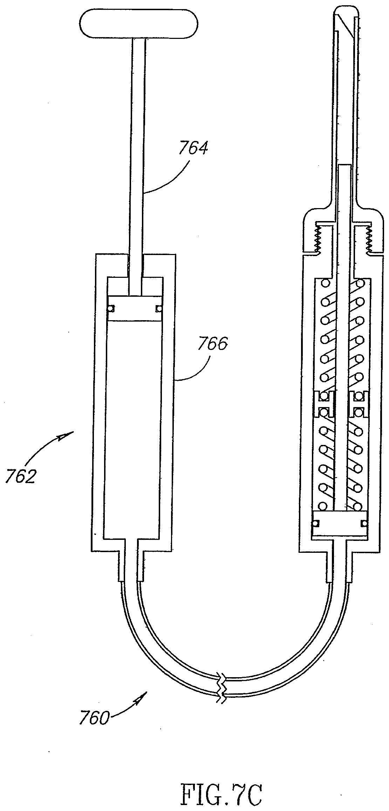

Figs. 7B and 7C show two alternative methods of providing hydraulic power. In Fig. 7B, a foot pedal pump 740 is used, in which a user places his foot on a pedal 744 and depresses it against a plate 742. Various foot pumps are known in the art. Optionally, a long press releases the pressure. Optionally, the hydraulic subsystem is a sealed system which is provided ready to use (e.g., including fluid) to the user and/or distributor. Exemplary lengths of the flexible tubing are between 0.2 and 3 metres, for example, between 1 and 2 metres. However, greater lengths can be used as well.

In an embodiment of the invention, a foot operable actuator employs a 1.5m, optionally 2m, optionally 2.5 m tube or a tube of lesser or intermediate or greater length (see Fig. 7B).

In an embodiment of the invention, a hand operable actuator employs a 0.25 m, optionally 0.5 m, optionally 1.0 m tube or a tube of lesser or intermediate or greater length.

Also shown in Fig. 7B is a variant of body 714, indicated as 714'. Instead of a single spring 720, two springs 720' are shown, with the spacer(s) between the springs. Optionally, the use of multiple springs helps maintain the spacers near a middle (or other relative length unit) of the pusher in danger of buckling.

Fig. 7C shows an alternative embodiment, in which a hand pump 760 is used, which pump can be of any type known in the art, for example, a mechanism 762 comprising a piston 764 and a cylinder 766. Optionally, the pumping is by rotating piston 764 elative to cylinder 766, which components include matching threading. Alternatively, linear motion is used. Optionally, a hydraulic gain is achieved between the pump and the delivery mechanism, for example a gain of 1:3, 1:5, 1:10 or any smaller, intermediate or greater gain.

In an embodiment of the invention, the hydraulic system is provided as a disposable unit, with a non-disposable (or a disposable) foot pump.

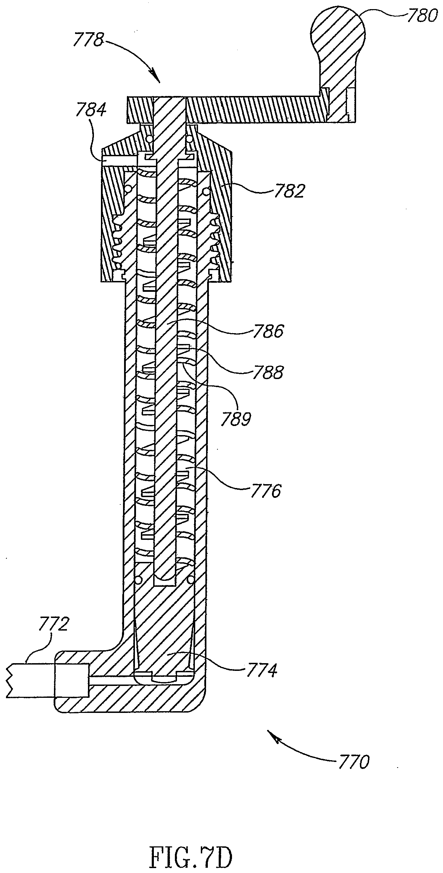

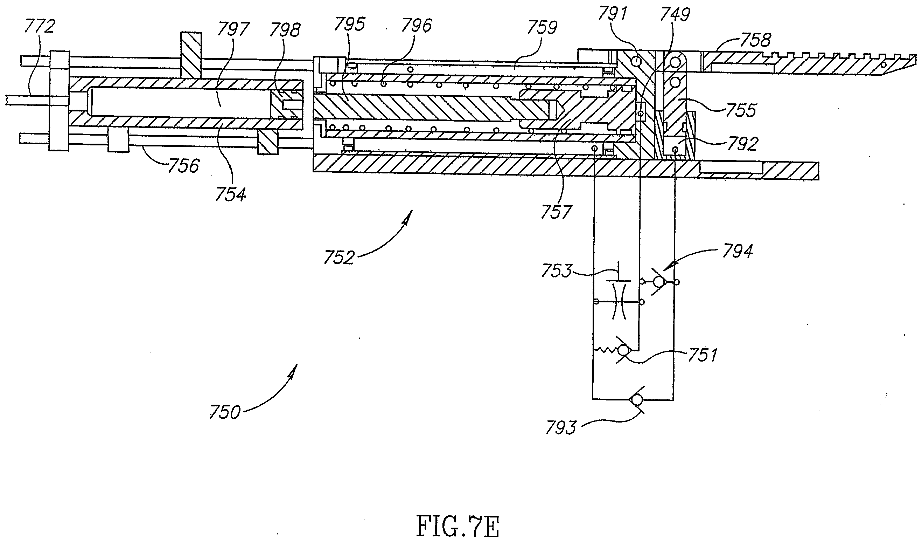

Fig. 7D shows a disposable mixing and storage chamber 770 and Fig. 7E shows a reusable pump 750 with a disposable hydraulic capsule 754.

Referring to Fig. 7D, a same capsule 770 is optionally used both for mixing and for storage/delivery of a material. Optionally, the material is a setting cement such as PMMA. In the embodiment of a hydraulic delivery stream, a flexible tube 772 is optionally permanently connected to a pump (Fig. 7E). When fluid is provided through tube 772, a piston 774 moves through a cylinder volume 776 and pushes the material out (e.g., and into a delivery system). In the figure, the capsule is shown loaded with a mixer 778. Optionally, materials are provided into volume 776 using a detachable funnel (not shown) and then the funnel is removed and mixer 778 inserted instead. In the exemplary mixer shown, a cap 782 covers cylinder 776. When mixing is completed, this cap may be replaced by a fitting adapted to couple to the delivery tube.

In use, a handle 780 is rotated, rotating a shaft 786 having a rotor 788 defined thereof, for example, as a helix. An optional stator 789 is provided. An optional vent 784 may be connected to a vacuum source, to suck out toxic and/or bad smelling fumes caused by the setting of the material. Optionally, a viscosity of the materials is estimated by the difficulty in turning the handle. Optionally, the handle includes a clutch (not shown) that skips when a desired viscosity is reached. Optionally, the clutch is settable. Optionally, a viscosity meter is used or viscosity is estimated based on temperature, formulation and time from mixing.

Cap 782 optionally includes a squeegee or other wiper, to wipe material off of mixer 778 when it is removed from capsule 770.

Referring to Fig. 7E, tube 772 connects to a capsule 754 which includes a piston 798 and a volume 797, pre-filled with fluid. In an embodiment of the invention, a frame 756 is provided attached to pump 750 for selectively receiving capsule 754.

Pump 750 is, for example, a hydraulic oil based pump-mechanism 752 that extends a pushing rod 795 which advances piston 798.

In the embodiment shown, a foot pedal 758, attached to an axis 791, forces a piston 755 into a cylinder 792. A one way valve 794 allows the fluid in cylinder 792 to flow into a volume 749 where it pushes against a piston 757. When pedal 758 is released, a spring (not shown) pulls it back to an upward position and allows a hydraulic fluid to flow from a storage chamber 759 (e.g., which surrounds the pump) through a one way valve 793 into cylinder 792.

A pressure relief valve 751 is optionally provided to prevent over pressurizing of cylinder 749. In an embodiment of the invention, a spring 796 is provided to push back piston 757 and pusher 795 with it, when pressure is released. Optionally, pressure is released using a bypass valve 753, which is manually operated. Once pusher rod 795 is retracted, capsule 740 is optionally removed.

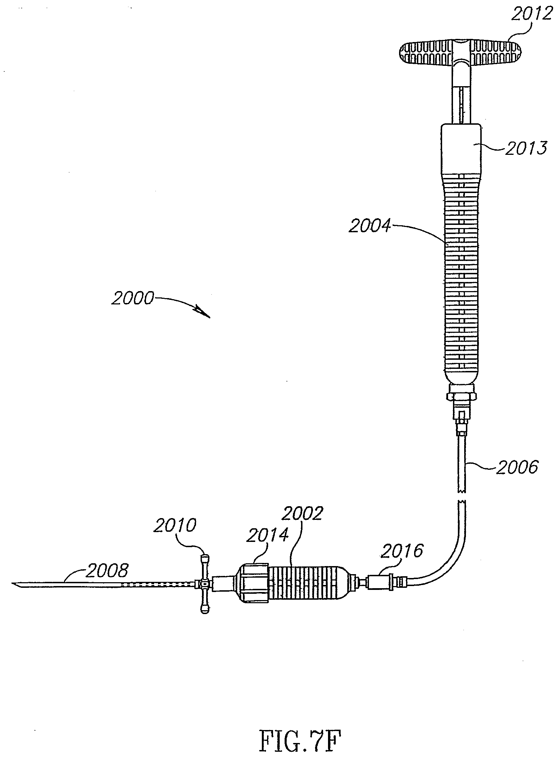

Figs. 7F (side view) and 7G (cross section) illustrate a hydraulic delivery system 2000, comprising a reservoir 2002 for material (e.g. bone cement) and a pressure source 2004. Optionally a flexible tube 2006 which connects reservoir 2002 and pressure source 2004 is included in system 2000. Reservoir 2002 is connectable to a cannula 2008. Cannula 2008 can optionally be introduced into a bone prior to injection of material from reservoir 2002. Reservoir 2002 and cannula 2008 may be connected using, for example, a luer lock connector 2010 and/or threaded connector 2014. Alternately, reservoir 2002 and cannula 2008 may be fashioned as a single piece. Optionally, cannula 2008 is compatible with a stylet (not shown).

In an embodiment of the invention, cannula 2008 is a plastically deformable cannula, optionally a slitted cannula. Optionally, use of a plastically deformable cannula 2008 facilitates positioning of reservoir 2002 outside of an X-ray imaging field.

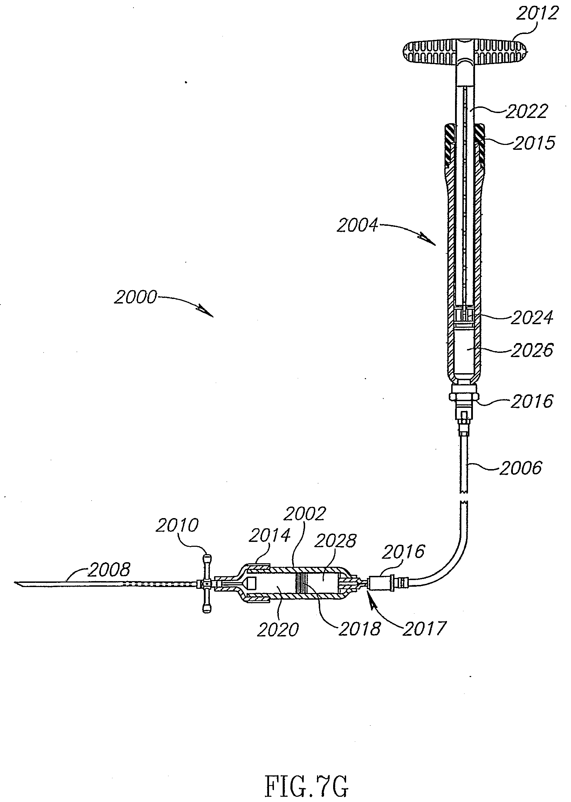

In an embodiment of the invention, pressure source 2004 is a hydraulic pressure source. Pressure source 2004 may be filled with any liquid. In an embodiment of the invention, pressure source 2004 is filled is filled with a sterile liquid 2026 (Fig. 7G), such as saline or water. Optionally, filling is via flexible tube 2006. Optionally, an outlet of tube 2006 is immersed in the liquid, and hydraulic actuator 2012 is operated to fill pressure source 2004 with liquid. In the embodiment of Figs. 7F and 7G, the actuator is depicted as a ball screw connection 2013 equipped with a rotating handle 2012. According to this embodiment, handle 2012 is rotated to fill pressure source 2004 with liquid.

In an embodiment of the invention, pressure source 2004 is provided as a prefilled unit. Optionally the prefilled unit includes body 2004, tube 2006 and/or cap 2013 and/or handle 2012 with drive shaft 2022. Optionally the prefilled unit includes body 2004, optionally with tube 2006 and is provided with temporary seals at each end. In an embodiment of the invention, the seals are removed or broken when additional system components are connected.

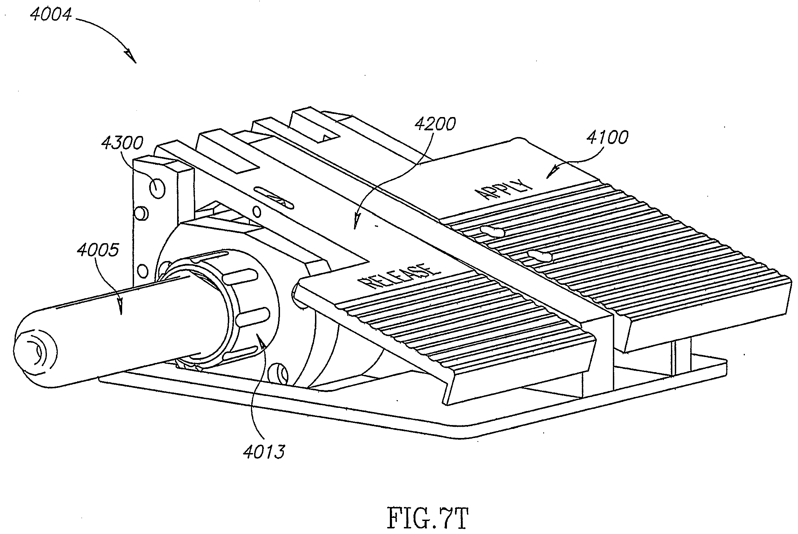

Although an exemplary manually operable actuator is pictured, hydraulic actuator 2012 may be operable by, for example, a foot pedal (see Fig. 7T described hereinbelow) or an electric actuator, such as a linear actuator or a motor. Electric operation may be achieved, for example, by employing a battery (as described hereinbelow) and/or mains supply. In an embodiment of the invention, the hydraulic actuator causes pressures source 2004 to generate a pressure of 50, optionally 100, optionally 150, optionally 200, optionally 300 atmospheres or lesser or greater or intermediate values.

Optionally, a safety mechanism (described in greater detail hereinbelow) limits pressure. In an embodiment of the invention, the safety mechanism includes a valve with a defined pressure threshold. In an embodiment of the invention, the threshold is set to prevent injection of cement after it has solidified and/or to protect the system from being damaged by attempts to inject solidified cement.

For example if a particular cement formulation is flowable at a pressure of 150 atmospheres during the "working window", a valve threshold may be set at 170 atmospheres. In such a case, the system would be designed to withstand a greater internal pressure, such as 200 atmospheres. This arrangement reduces the chance that the system will be damaged when cement solidifies.

Optionally, the pressure threshold is 150 or 210 atmospheres or lesser or greater or intermediate values. In an embodiment of the invention, the safety mechanism may be used to release trapped gases (e.g. air) and/or cement. In an embodiment of the invention, each activation of the hydraulic actuator (e.g., handle rotation, foot pedal pressing) results in injection of defined amount of cement. Optionally, the hydraulic actuator provides pressure amplification.

Optionally, air is removed from pressure source 2004 and/or tube 2006 prior to connection of pressure source 2004 to reservoir 2002. One or more connectors 2016 can be optionally employed to connect of reservoir 2002 to pressure source 2004, optionally via tube 2006. Connectors 2016 may be, for example, luer lock connectors, quick release connectors or threaded connectors.

In an embodiment of the invention, delivery system 2000 is employed to deliver a viscous material, optionally a viscous bone cement. In an embodiment of the invention, cement components (e.g. powder and liquid) are mixed. The mixture is loaded into reservoir 2002, optionally via a reservoir cap 2014. Optionally cap 2014 is unscrewed and reservoir 2002 is filled with bone cement 2020 (Fig. 7G). According to various embodiments of the invention, cement 2020 may be inserted into reservoir 2002 manually or by use of a tool or filling device. In an embodiment of the invention, cement 2020 is sufficiently viscous that it may be preformed to a size and shape which permit easy insertion into reservoir 2002. After cement 2020 is inserted in reservoir 2002, cap 2014 is replaced. Pressure source 2004 is connected to reservoir 2002, optionally via flexible tube 2006, via connector(s) 2016. In an embodiment of the invention, pump 2004, tube 2006, connector 2016 and fluid 2026 are provided as a pre-assembled sterile unit. Optionally, air is removed from the system via a pressure release valve 2017, optionally on connector 2016. Optionally rotation of handle 2012 increases pressure in the system and drives air out. Optionally, air is released through valve 2017 or is expelled via cannula 2008 prior to insertion of the cannula in the body.

Optionally, mixing of cement components is performed under vacuum to prevent air bubbles from being entrapped in the cement.

Reservoir 2002 is connected to cannula 2008 via connector 2010. In an embodiment of the invention, connector 2010 serves as an orientation marker which indicates an ejection direction of cement injected through the cannula. Operation of handle 2012 delivers cement 2020 from reservoir 2002 to cannula 2008. In an embodiment of the invention, rotation of handle 2012 rotates pump thread 2022 and advances piston 2024. Advancing piston 2024 applies pressure to liquid 2026 within pressure source 2004 and causes liquid 2026 to advance into reservoir 2002, optionally via tube 2006. Liquid 2028 in reservoir 2002 applies pressure to a piston 2018 within reservoir 2002. As piston 2018 advances through reservoir 2002, it causes cement 2020 to advance and exit reservoir 2002 via the cannula 2008.

According to various embodiments of the invention, 2008 cannula may be equipped with one or more apertures for cement delivery. Optionally, these apertures are located at a distal end and/or near the distal end of cannula 2008. Optionally, the apertures face axially and/or radially with respect to the cannula. Optionally, the cannula distal end is closed. In an embodiment of the invention, one or more lateral openings on the cannula permit sideways injection to a desired target in a bone from a cannula with a permanently closed distal tip.

In an embodiment of the invention, the injection procedure is monitored by a medical imaging system (e.g. fluoroscopy). When a desired amount of cement 2020 has been delivered through cannula 2008, injection is stopped. Optionally, reservoir 2002 is disconnected from cannula 2008 and/or tube 2006 and/or pressure source 2004. Cannula 2008 is removed from the bone, and the operation site is closed.

In an embodiment of the invention, cannula 2008 and/or cement 2020 are composed of biocompatible materials. In an embodiment of the invention, components of system 2000 which contact cement 2020 are not adversely affected by the cement. For example if MMA is employed as a component of the cement, reservoir 2002 may be constructed of an MMA monomer resistant polymer/Plastic while cannula 2008 may be constructed of Stainless Steel. In various embodiments of the invention, reservoir 2002 may be made of, for example, nylon, pressure source 2004 may be made of, for example, metal and/or plastic (e.g. polycarbonate), and the flexible tube 2006 is made of, for instance, nylon or Teflon (trade mark).

In an embodiment of the invention, reservoir 2002 and/or pressure source 2004 are constructed of Amorphous Nylon (e.g. Nylon Nos. 6, 6/6 or 12, e.g. Grilamid 90 or Durethan) and/or of a cyclic olefin copolymer (COC) (e.g., Topas (trade mark); Ticona GmbH, Kelsterbach, Germany). These materials are resistant to cement components, including the monomer component.

In an embodiment of the invention, the reservoir 2002 is designed to withstand pressures in the range of 100 to 300 atmospheres. Optionally, a reservoir with an internal diameter of 18 mm wis constructed with a wall thickness of 5mm so that the outer diameter is 28mm. Optionally, the walls are ribbed to increase strength and/or reduce weight.

The pressure source 2004 will come in contact only with the hydraulic fluid, such as water or a saline solution. Optionally, pressure source 2004 is constructed of polycarbonate and/or polysulphone and/or PEEK or other materials which are not corroded by the hydraulic fluid.

Optionally, system 2000 employs a pressure of at least 100, optionally 150, optionally, optionally 200, optionally 300 atmospheres or lesser or intermediate or greater values to inject cement 2020. In an embodiment of the invention, system 2000 is constructed to withstand these operational pressures. The actual pressure which accumulates in the system may vary, for example as the viscosity of cement 2020 varies. Various types of connectors and/or pressure sources and/or reservoirs and/or cannulae may be employed depending upon an anticipated pressure. One of ordinary skill in the art will be able to select commercially available components such as connectors, tubing and O-rings which are suitable for use in construction of a system 2000 with a given anticipated operating pressure. In some embodiments of the invention, pressure is provided by a tamping instrument including a rod adapted to comply with a lumen of the cannula (see Fig. 7A). In other embodiments of the invention, pressure is provided by a piston (e.g. 2018 in Fig. 7G) with a diameter wider than the cannula lumen.

It is stressed that the combination of high viscosity cements employed in the context of the invention and the small diameter of the cannula to be filled renders standard syringes or other non-amplifying pressure sources ill suited for cannula filling. Optionally, the viscous cement is manually manipulated to facilitate loading of the delivery device reservoir as described below under "Transfer of viscous materials".

In those embodiments of the invention in which a tamping rod is employed, it may optionally be introduced into the vertebra via a working sleeve characterized by a slightly larger diameter than the cannula diameter.

Optionally, reservoir 2002 is transparent to permit visualization of cement 2020. In an embodiment of the invention, a transparent reservoir 2002 is marked with graduations indicating the amount cement 2020 in the reservoir. Optionally, this permits a user to ascertain how much cement is being injected.

In an embodiment of the invention, pressure source 2004, optionally attached to tube 2006, are provided filled with liquid. Optionally, air has already been flushed from these components. Such an embodiment may expedite operating room procedures. According to this embodiment, a quick connector 2016, connecting the reservoir 2002 to the flexible tube 2006, is equipped with a uni-directional valve 2017 that seals the tube 2006 until it is connected to the reservoir 2002.

Optionally, a uni-directional valve (not shown in the Figures) is incorporated into the reservoir piston 2018, and is opened/released toward reservoir portion that does not contain cement 2028. For example, systems of the type illustrated in fig 7 G, the cement is loaded from a distal side (covered by cap 2014) of container 2002. Prior to attaching connector 2016, air can be drained by a one-way valve in piston 2018, so entrapped air can flow from container 2020 through the one-way valve in piston 2018, and through escape valve 2017.

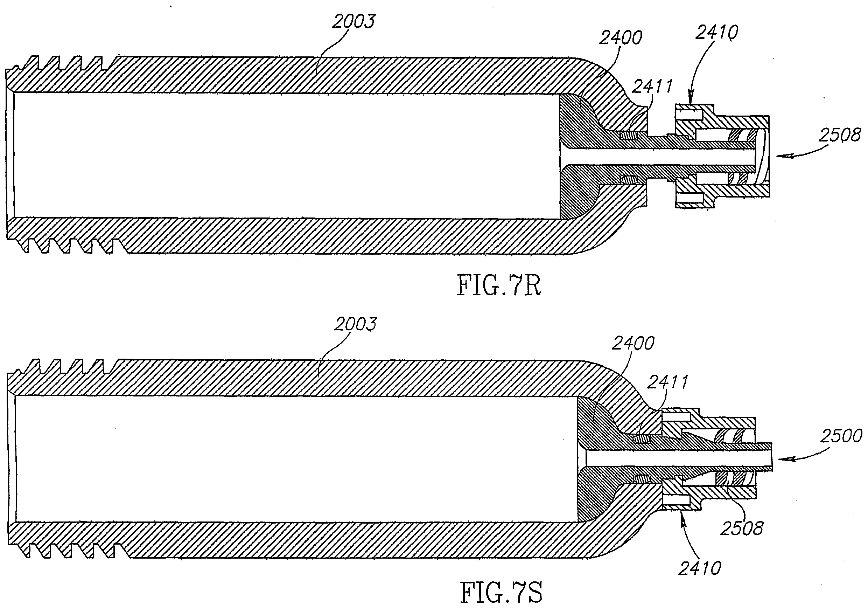

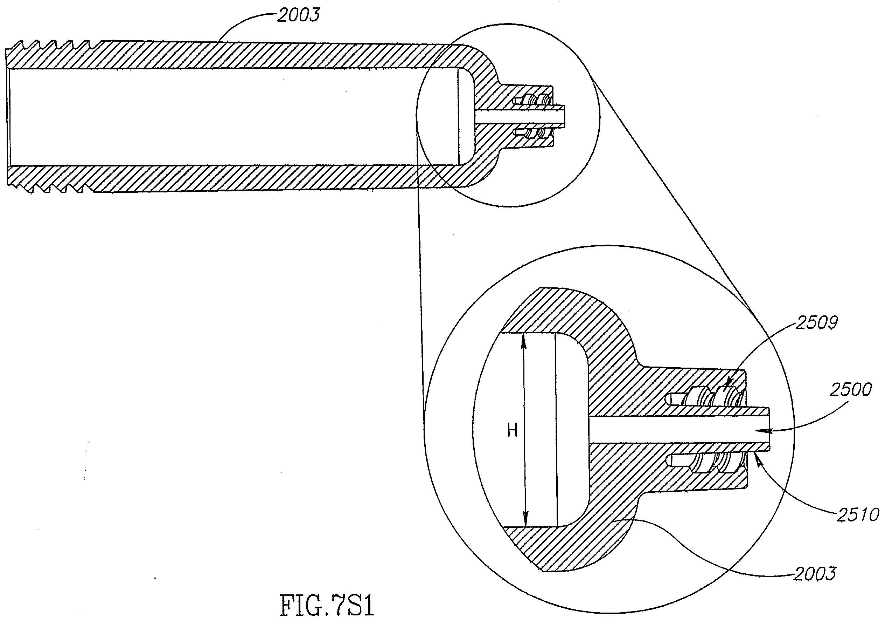

In an embodiment of the invention, (Figs. 7P; 7S) air escapes from opening 2500 directly. Optionally, no valves are provided.

Although a hand operated handle is pictured, according to additional embodiments of the invention, handle 2012 may be replaced by a foot pedal that is used to actuate piston 2024. Alternatively or additionally, pressure source 2004 may rely upon electric power for actuation. Electric power may be supplied, for example, by a battery. In an embodiment of the invention, a battery powered motor turns screw threads 2022 to advance piston 2024.

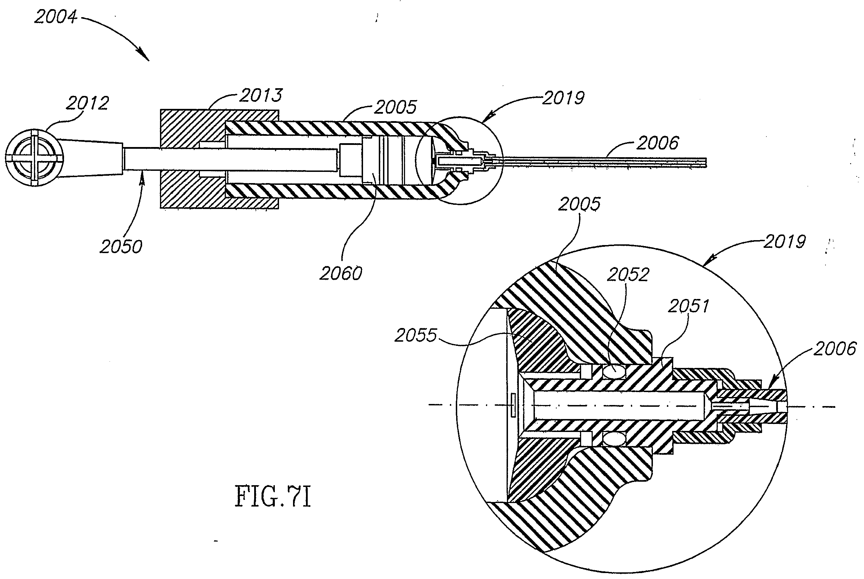

The construction and operation of exemplary hydraulic pressure sources for use in systems of the general type depicted in Figures 7F and 7G is depicted in greater detail in Figs. 7I through 7O.

Exemplary pressure source

Fig. 7I illustrates a hydraulic pressure source 2004 including an actuation handle 2012 connected to a drive shaft 2050 and piston 2060. Drive shaft 2050 passes through hydraulic chamber cap 2013 and is insertable in hydraulic pressure body 2005. Piston 2060 is connected to, or constructed as part of, a distal end of drive shaft 2050. Piston 2060 forms a circumferential seal with respect to an inner surface of a lumen of hydraulic pressure body 2005. In an embodiment of the invention, hydraulic pressure body 2005 is constructed of amorphous nylon or polycarbonate.

In an embodiment of the invention, a small step of the threading (eg 1 mm/rotation), a small radius of bolt (eg 4 mm) and materials employed in construction for bolt and nut (eg Nylon for nut 2013 and stainless steel for bolt 2050) produce a low friction coefficient.

Amorphous nylon provides the requisite strength to resist high internal pressures with a low weight when compared to previously available steel hydraulic pressure sources. In an embodiment of the invention, an amorphous nylon reservoir with an OD of 20, optionally 25, optionally 30, optionally 35 mm (or lesser or intermediate or greater values) resists an internal pressure of as much as 300 atmospheres or more. Optionally, it can be transparent. Optionally, a transparent pressure body 2005 allows an operator to observe progress of piston 2060. In an embodiment of the invention, progress of the piston is gauged against calibration markings on body 2005.

In an embodiment of the invention, pressure body 2005 is connectable to a flexible tube 2006. Optionally, tube 2006 is glued to reservoir 2002 directly (eg by UV curing glue). The inset 2019 shows an embodiment of this connection in greater detail. A funnel 2055 is seated in a distal aperture of hydraulic pressure body 2005 and sealed by means of an O-ring 2052 and adapter plug 2051. According to this embodiment of the invention, as pressure on hydraulic fluid in hydraulic pressure body 2005 increases, funnel 2055 is more firmly seated against the distal aperture of hydraulic pressure body 2005. Optionally, this arrangement spreads the stresses radially over a distal end of the reservoir. Optionally, this arrangement prevents leaks at operating pressures of 100 to 300 atmospheres. The liquid is forced through tube 2006 as the pressure increases. In an embodiment of the invention, a hydraulic unit including body 2005, cap 2013, drive shaft 2050 and piston 2060 is provided as a unit pre-filled with a sterile liquid. Optionally, walls 2005 are transparent so that piston 2060 is visible to an operator of the unit, hi an embodiment of the invention, transparent walls 2005 are marked with graduations which indicate an injected volume of cement.

In an embodiment of the invention, specific materials for the cover 2013 and drive shaft 2050 are chosen to reduce the friction coefficient ($\mu$). Optionally, cover 2013 is made of a polymer/plastic while shaft 2050 is made of steel. Optionally, Radius of Friction (R) is reduced by reducing the thread diameter on shaft 2050 and/or cap 2013.

$$M = R \cdot f$$

$$f = \mu \cdot f$$

so

$$M = R \cdot \mu \cdot f .$$

Therefore, a reduction in the moment the moment needed for every normal force F is optionally achieved by reducing R and/or $\mu$.

Working moment (M) is the product of R and the radial force (f) of the hydraulic liquid. The force (F) between cap 2013 and shaft 2050 is the axial force applied on the piston 2060.

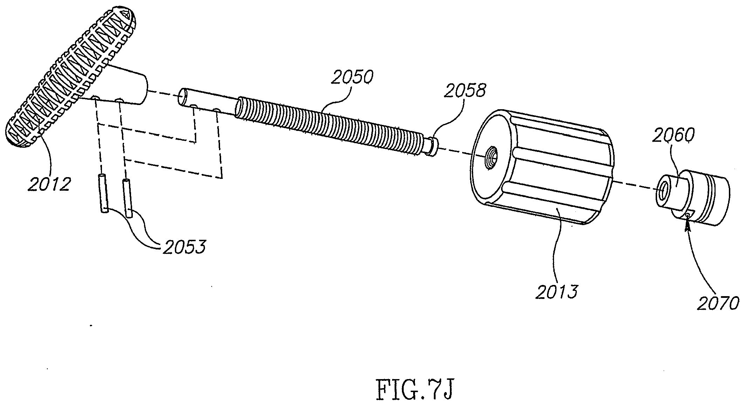

Fig. 7J is an exploded view of a handle and piston assembly as depicted in the embodiment of Fig. 7I. According to this embodiment, pins 2053 anchor handle 2012 to drive shaft 2050. Optionally pins 2053 are designed to break if excessive torque is applied. In an embodiment of the invention, breaking of pins 2053 is a safety feature. Cap 2013 and shaft 2050 are each threaded so that rotation of handle 2012 can cause shaft 2050 to advance or recede through cap 2013. Optionally, cap 2013 is constructed of a plastic polymer. Optionally, shaft 2050 is constructed of metal, for example stainless steel. In an embodiment of the invention, threaded shaft 2050 is constructed of stainless steel and has a diameter of 6 mm, optionally 5 mm, optionally, 4 mm or lesser or greater or intermediate diameters. Optionally, stainless steel threads mated to plastic polymer threads provide a low friction connection which makes it easy to operate handle 2012 manually. In an embodiment of the invention, a narrow diameter drive shaft 2050 is employed as a means of reducing the amount of friction against threads of cap 2013. hi an embodiment of the invention, threads on shaft 2050 and 2013 each engage a set of ball bearings to provide a ball screw mechanism. In this figure, an optional pressure release port 2070 on piston 2060 is visible.

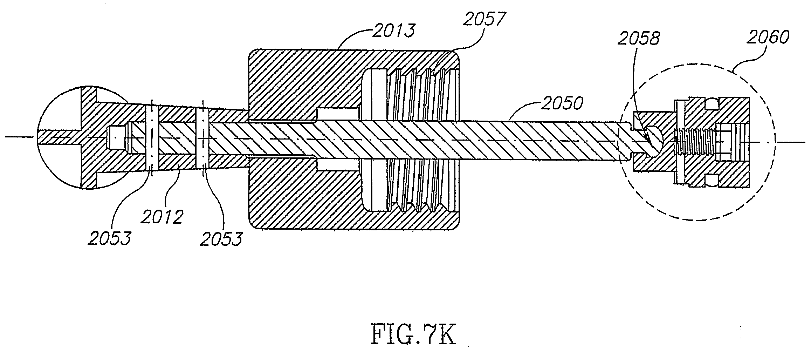

Fig. 7K is a cross sectional view of a handle and piston assembly as depicted in Fig. 7J Fig. 7K shows a second set of threads 2057 on cap 2013. Threads 2057 are one exemplary means to engage cap 2013 to hydraulic pressure body 2005. Once cap 2013 is engaged to hydraulic pressure body 2005, operation of handle 2012 will cause advancement of piston 2060 in hydraulic pressure body 2005. Optionally, threads 2057 are in the same direction, as threads on shaft 2050 to prevent disassembly. Optionally, threads 2057 are locked once the cap 2013 is completely on.

Fig. 7K also shows an exemplary engagement mechanism by which drive shaft 2050 may be coupled to piston 2060. The pictured engagement mechanism relies upon a ball 2058 which snaps into a matching socket (2059; Fig. 7M) in piston 2060. In an embodiment of the invention, this arrangement assures that piston 2o60 remains attached to ball 2058 as shaft 2050 advances. Optionally, a ball/socket connection of this type permits piston 2060 to advance without rotating as bolt 2050 rotates. In an embodiment of the invention, this lack of piston rotation increases valve life and improves sealing. Optionally, the ball/socket type connection provides a sufficiently strong engagement force so that back turning of shaft 2050 will retract piston 2060. Optionally, a ball socket connection is economical yet reliable.

In an embodiment of the invention, operation of handle 2012 in an opposite direction will cause retreat of piston 2060 in hydraulic pressure body 2005. Optionally, operation in a reverse direction ceases injection of cement.

### Pressure source safety valve

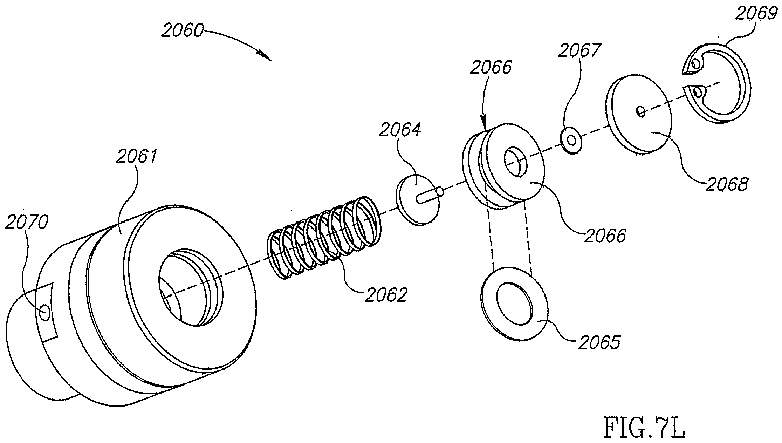

Fig. 7L is an exploded view of a piston 2060 including a pressure release valve which can relieve excess pressure of hydraulic fluid by venting hydraulic fluid through one or more release ports 2070 on piston 2060. In an embodiment of the invention, the pressure relief valve includes a spring 2062, a pressure release aperture element 2064, and a sealing gasket 2067. In the figure, optional washers 2065, 2066, 2068 and 2069 are depicted. In an embodiment of the invention, the pressure release valve is triggered at a pressure of 160 Atmospheres. Optionally, a threshold pressure of 160 atmospheres protects a system designed to withstand 200 atmospheres.

Figs. 7M and 7N are cross sectional views of the valve of Fig. 7L in an open and a closed operational state respectively. Fig. 7N shows spring 2062 in a fully extended configuration. As distal end 2058 of drive mechanism is forced into receptacle 2059, piston 2060 advances through hydraulic pressure body 2005. Advancement of piston 2060 causes an increase in pressure of a fluid residing in hydraulic pressure body 2005. Advancement of piston 2060 causes an increase in pressure of a fluid residing in hydraulic pressure body 2005. When the pressure inside the hydraulic pressure body 2005 reaches a predeterminedjhreshold (e.g., 150, 160 or 210 atmospheres), the retraction of spring 2062 causes apertured element 2064 to protrude through seal 2067. Apertured element 2064 provides a channel of fluid communication between pressurized fluid 2020 (Fig. 7G) and lower pressure compartment (2028). This permits hydraulic fluid to flow to the rear chamber 2028 behind piston 2060 via release ports 2070. Release of hydraulic fluid causes the pressure to drop below the threshold, spring 2062 to expand and seal the valve. In an embodiment of the invention, released hydraulic fluid is visible in transparent pressure body 2005.

Fig. 70 is a side view of an embodiment of piston 2060 showing release port 2070. In an embodiment of the invention, opening of the valve reduces the hydraulic pressure to the threshold pressure. 9

In an embodiment of the invention, pressure at the defined threshold, indicates that the procedure should be stopped because the cement has solidified. If the physician wants to continue with the procedure, replacement of reservoir 2002 is indicated.

### Injection Reservoir

Figs. 7P and 7Q are side cross-sectional views of an injection reservoir 2002 according to an embodiment of the invention. Reservoir 2002 may be constructed of, for example, amorphous nylon e.g. Durethan (trade mark) (LANXESS, Leverkuzen, Germany), Grilamid (trade mark) (EMS-Grivory, Reichenauerstrasse, Switzerland) or Topas (trade mark) (Ticona GmbH, Kelsterbach, Germany). In an embodiment of the invention, materials for construction of reservoir 2002 are selected so that they are not corroded by the relevant cement. In an embodiment of the invention, the amorphous nylon is transparent. In an embodiment of the invention, the thickness of walls 2003 of reservoir 2002 is greater than 3 mm, optionally greater than 4 mm, optionally greater than 5 mm optionally greater than 6 mm or intermediate or greater values. In an embodiment of the invention, reservoir 2002 is characterized by a wall thickness to internal diameter ratio of about 0.23, optionally 0.25, optionally, 0.27, optionally 0.29 (e.g., wall thickness of about 5 mm and ID of about 18 mm), This ratio provides sufficient strength to withstand pressures of 100 to 300 atmospheres.

In an embodiment of the invention, walls 2003 of reservoir 2002 are transparent and marked with a scale indicating volume. Optionally, this permits an operator of the system to ascertain how much cement has been injected at any given moment. Optionally, ribs provided to add strength serve as a scale indicating volume.

In an embodiment of the invention, cement reservoir 2002 is loadable with sufficient cement to treat at least one vertebra with a single injected aliquot. Optionally, 5 ml optionally 10 ml or intermediate or greater volumes are typically employed to treat a single vertebra. Optionally, cement reservoir 2002 is loadable with sufficient cement to treat at least two vertebrae, optionally at least 3, optionally at least 4 vertebrae without re-filling. Optionally, this reduces a number of access procedures for each vertebra, optionally to a single access procedure. In an embodiment of the invention, a single access procedure is employed to treat at least two locations in a vertebra.

In an embodiment of the invention, reservoir 2002 is loaded with a cement characterized by a long "working window" during which the cement is characterized by a viscosity above 500 Pa.s but is not yet solidified. Optionally, the working window is greater than 5, optionally 8, optionally 12, optionally 15 minutes or intermediate or greater times.

In an embodiment of the invention, reservoir 2002 serves also as a mixing chamber. Optionally, a polymer component and a monomer component of the cement are mixable in reservoir 2002. Alternatively, mixing is performed in a separate mixing apparatus and cement is transferred to the reservoir after mixing is complete.

### Reservoir assembly

Fig. 7P illustrates assembly of an exemplary injection reservoir for injection of bone cement. While this reservoir is suited for use in a system of the general type depicted in Figs. 7F and 7G, the embodiment of Fig. 7P features a proximal reservoir cap 2100 as opposed to the distal reservoir cap of Figs 7F and 7G.

A reservoir cap 2100 includes a connector plug 2110 equipped with a tube connection 2310 to facilitate connection to tube 2006 which contains hydraulic fluid. Plug 2110 and tube connection 2310 form a contiguous lumen 2300 which facilitates delivery of hydraulic fluid from tube 2006. Plug 2110 optionally rotates within cap 2100 so that an angle of tube connection 2310 with respect to reservoir 2002 can be adjusted.

In an embodiment of the invention, the angle of connection 2310 with respect to reservoir 2002 is adjusted so that tube 2006 is out of the field of view of an X-ray imaging device. In an embodiment of the invention, rotation of plug 2110 makes connection of 2006 more convenient. Plug 2110 optionally includes a coupling portion 2115 which mates with a complementary coupling portion 2215 (Fig. 7Q) on delivery piston 2200. Optionally, piston 2200 is mounted on plug 2110 during assembly by snap coupling portions 2115 and 2215.

Cement reservoir 2600 is optionally filled with cement prior to closing of reservoir body 2003 with cap 2100. Cap 2100 can then be attached to body 2003 by means of, for example, mated threads on the two pieces. At this stage, both plug 2100 and piston 2200 are sealed to an inner side of walls 2003, for example by O-rings 2211. During operation, hydraulic actuator 2004 causes a fluid to flow through tube 2006 under pressure and enter lumen 2300. As the applied pressure increases, this fluid accumulates in portion 2700 of reservoir 2002 (Fig. 7Q). In an embodiment of the invention, plug 2100 and piston 2200 disengage when the fluid pressure developed in 2700 supplies enough force to advance piston 2200 against resistance supplied by cement in portion 2600 of reservoir 2002.

At the distal end of reservoir 2002, an optional inner plug 2400 engages an outer connector 2410 and holds outer connector 2410 to the distal end of the injection reservoir. Outer connector 2410 is constructed to engage an injection cannula and to remain engaged at a relevant operating pressure of the delivery system. Inner plug 2400 and outer connector 2410 form a channel of fluid communication 2500 which can facilitate a flow of cement from reservoir 2600 to an inner lumen of a cannula connected to connector 2410. The function of plug 2400 during filling of reservoir 2002 is described below with reference to Figs. 7R and 7S.

Fig. 7Q shows that fluid 2700 accumulates within 2005 and pushes piston 2200 away from plug 2110 and towards plug 2400. As the volume of fluid 2700 increases, the volume of cement 2600 decreases and the cement is pushed outwards through channel 2500 into a cannula (not shown in this figure). In an embodiment of the invention, piston 2200 is a floating piston which is moved by a column of fluid 2700. Optionally, walls 2005 are transparent so that piston 2200 is visible to an operator of the unit, for example as an indicator of cement volume. In an embodiment of the invention, transparent walls 2003 are marked with graduations which indicate an injected volume of cement.

In an embodiment of the invention, cement reservoir 2600 is supplied as a separate unit comprising walls 2003, inner plug 2400 (Fig. 7R) and outer connector 2410. Figs. 7R and 7S illustrate one embodiment of this exemplary type. Plug 2400 contains an aperture 2500 which is initially covered by connector 2410 during filling of the reservoir with cement and subsequently opened to permit attachment of a cannula.

Fig. 7R illustrates outer connector 2410, inner plug 2400 and walls 2003 in cross section. In this figure, connector 2410 is only partially pressed onto a protruding portion of plug 2400. Seal 2508 remains unbroken. O-ring 2411 provides a tight seal between walls 2003 and plug 2400. This arrangement permits introduction of cement into the reservoir from a proximal end before cap 2100 (Fig. Q) is attached. Once the reservoir is filled, cap 2100 may be applied as described above. Optionally, this arrangement permits a more efficient seal to a cannula and/or provides the possibility to rotate the cannula with respect to the reservoir In an embodiment of the invention, the cannula may be deformed from a straight line and/or be directional.

Once the reservoir is filled and capped, seal 2508 may be broken (Fig. 7S) by advancing connector 2410 towards plug 2400. Breaking of seal 2508 creates channel of fluid communication 2500 which can facilitate a flow of cement outwards from the reservoir into a cannula (not shown in this figure) connected to connector 2410. In an embodiment of the invention, breaking of seal 2508 exposes a threaded and/or luer connection which is adapted to engage a cannula.

Fig. 7S1 depicts an embodiment of reservoir 2600 in which walls 2003 are formed as a contiguous unit which includes some of the functional characteristics of outer connector 2410, inner plug 2400 in Fig. 7S. In the embodiment of Fig. 7S1, aperture 2500 is not sealed. Walls 2003 include threads 2509 and/or luer connector 2510 for attachment to a cannula.

Foot operable actuator

Fig. 7T is a perspective view of a foot operable hydraulic actuator 4004. A foot operable actuator may be used in place of a hand operable actuator. In an embodiment of the invention, a foot operated actuator permits an operator to employ both hands for other tasks and/or reduces the need for an assistant. In the figure, hydraulic reservoir 4005 is functionally similar to hydraulic reservoir 2005 as described above. Locking ring 4013 is functionally analogous to reservoir cap 2013 as described above. The pictured foot operable actuator contains a drive pedal 4100 which moves angularly with respect to an axle 4300. Each depression advances a hydraulic piston in hydraulic reservoir 4005 by a fixed increment. After pedal 4100 is depressed for actuation, it returns to a pre- actuation position automatically. This return may be achieved, for example, by use of a spring which provides a resistive force. Optionally, actuator 4004 includes an additional pedal 4200 for pressure release which moves angularly with respect to an axle 4300. In an embodiment of the invention, pedals 4100 and 4200 are clearly marked so that an operator can distinguish between them easily. Markings may be, for example, in printed symbols and/or by pedal colour and/or by pedal size and/or by pedal shape and/or by pedal position.

In an embodiment of the invention, walls of hydraulic pressure chamber 4005 are transparent and marked with a scale. Optionally, the scale indicates indicating volume. Optionally, this permits an operator of the system to ascertain how much cement has been injected at any given moment. Optionally, ribs provided in the walls to add strength serve as a scale indicating volume.

Pressure inducing levers may apply increments of hydraulic pressure using any clutch/drive mechanism known in the art to advance a hydraulic piston within hydraulic reservoir 4005. Examples of suitable drive mechanisms include, but are not limited to ratchet pawl mechanisms, sprag clutches, roller ramp clutches and mechanical diodes, cam followers and roller bearing cam followers. Sprag clutches are commercially available (e.g. from JTEKT Co.; Osaka/Nagoya; Japan). Mechanical diodes are available from, for example, Epilogics (Los Gatos; CA; USA). One of ordinary skill in the art of mechanical engineering will be able to select a suitable commercially available drive mechanism, or construct a suitable drive mechanism from commercially available parts in consideration of desired performance characteristics for any particular embodiment of the invention.

In an embodiment of the invention, release pedal 4200 is provided to release some or all applied hydraulic force. Such a release may be desired, for example to change or replace cement reservoir 2004, at the end of a surgical procedure and/or in the event of an emergency. Optionally, release pedal 4200 may open a valve which vents hydraulic fluid from reservoir 4005. Alternatively or additionally, release pedal 4200 may act by permitting a threaded drive shaft to move backwards so that a hydraulic piston in reservoir 4005 is retracted.

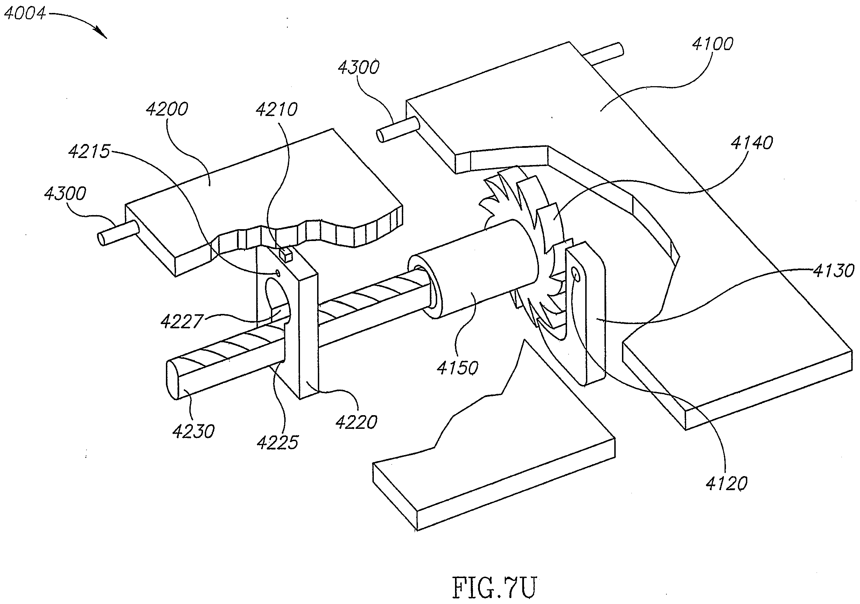

Fig. 7U illustrates one embodiment of a pedal operated drive mechanism with a companion release pedal.

When drive pedal 4100 is depressed by a foot, it rotates with respect to axle 4300 and depresses drive arm 4130. Drive arm 4130 engages gear 4140 causes it to rotate angularly by a single increment. The increment may be varied by varying the number of teeth on gear 4140. Gear 4140 is mounted on a drive nut 4150 so that rotation of gear 4140 causes rotation of drive nut 4150. Drive nut 4150 is equipped with an inner threading mechanism (not visible in the figure) which can advance driveshaft 4230 without rotating a distal end thereof. As drive nut 4150 is rotated it operates the inner threading mechanism and drives drive shaft 4230 outwards through a narrow portion 4225 of a hole in clutch plate 4220. Drive arm 4130 and gear 4140 are depicted to generally indicate the presence of a ratcheted gear or functionally similar mechanism and their pictured shapes should not be viewed as a limit of the invention.

When drive pedal 4100 is released, it is raised by a spring (not shown). Drive arm 4130 disengages from gear 4140. Drive arm axle 4120 permits drive arm 4130 to rotate slightly as it is raised and lowered so that it disengages and re-engages teeth of gear 4140.

A distal end of drive shaft 4230 pushes a piston (not shown in this view) in hydraulic pressure reservoir 4005 (Fig. 7T). Pressure in the reservoir tends to force drive shaft 4230 to return towards drive nut 4150.

A narrow aperture 4225 in clutch plate 4220 prevents rotation of drive shaft 4230 while drive arm 4130 is disengaged from gear 4140 and prevents the shaft from retuning towards nut 4150. Clutch plate 4220 is optionally attached to pedal 4200 by rod 4210 and pin 4215.

When release lever 4200 is depressed, it lowers clutch plate 4220 so shaft 4230 passes through a wide portion 4227 of the hole. Shaft 4230 is then free to retract towards nut 4150 because it can rotate in wide portion 4227 of the hole. Optionally, shaft 4230 is constructed with sectorial threading (not pictured in this view) so that when clutch plate 4220 descends shaft 4230 rotates against a nut that defines a desired resistance.

Optionally, operation of lever 4200 releases all of the pressure in the system or only a portion of it. Optionally, release may be sudden or gradual.

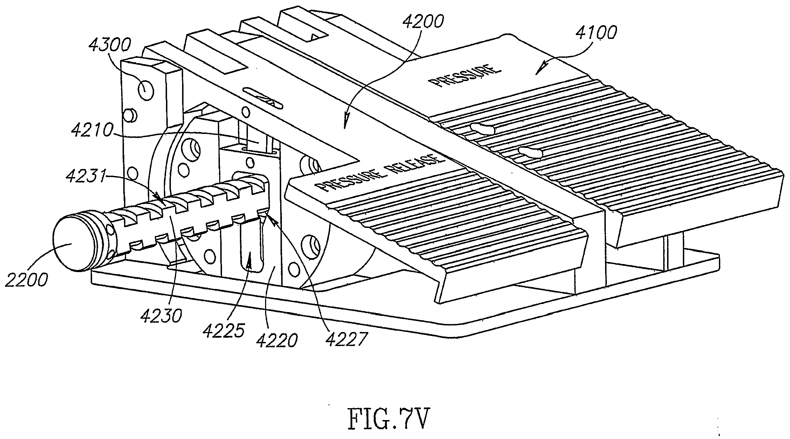

Fig. 7V illustrates a foot operable hydraulic actuator as seen in Fig. 7T with hydraulic reservoir 4005 removed. In this view, drive shaft 4230 is characterized by optional sectorial threading 4231. Shaft 4230 is pictured in wide portion 4227 of a hole in clutch plate 4220 so that it can retract. A distal end of shaft 4230 is fitted with a piston 2200. As shaft 4230 advances it drives piston 2200 into pressure reservoir 4005 (not pictured in this view) and increases the hydraulic pressure.

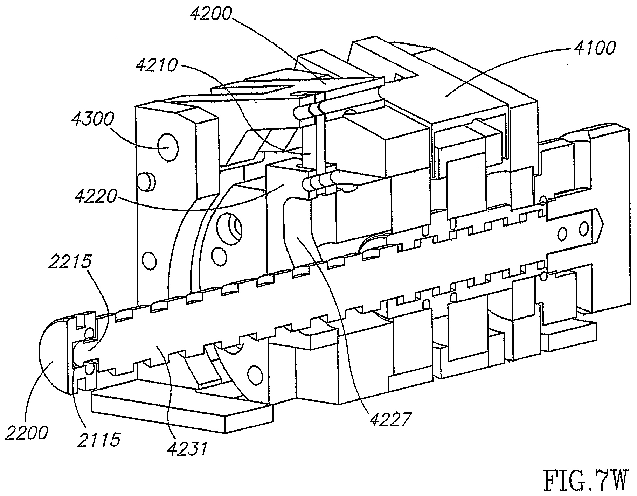

Fig. 7W is a cross sectional view of the exemplary actuator depicted in Fig. 7V. In this view release lever 4200 is raised so that wide portion 4227 of the hole in clutch plate 4220 is raised off of sectorial threads 4231 on shaft 4230. This causes the clutch plate to engage the drive shaft and prevent retraction of the drive shaft.

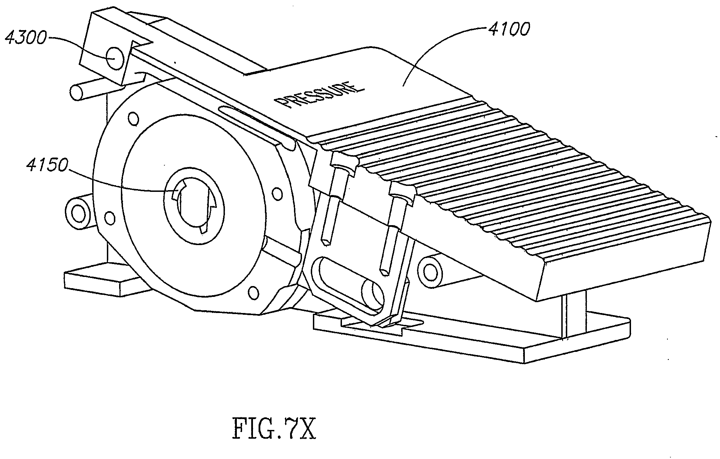

Fig. 7X illustrates an embodiment of drive nut 4150 adapted to engage and rotate sectorial threads such as those illustrated in Figs. 7 V and 7 W.

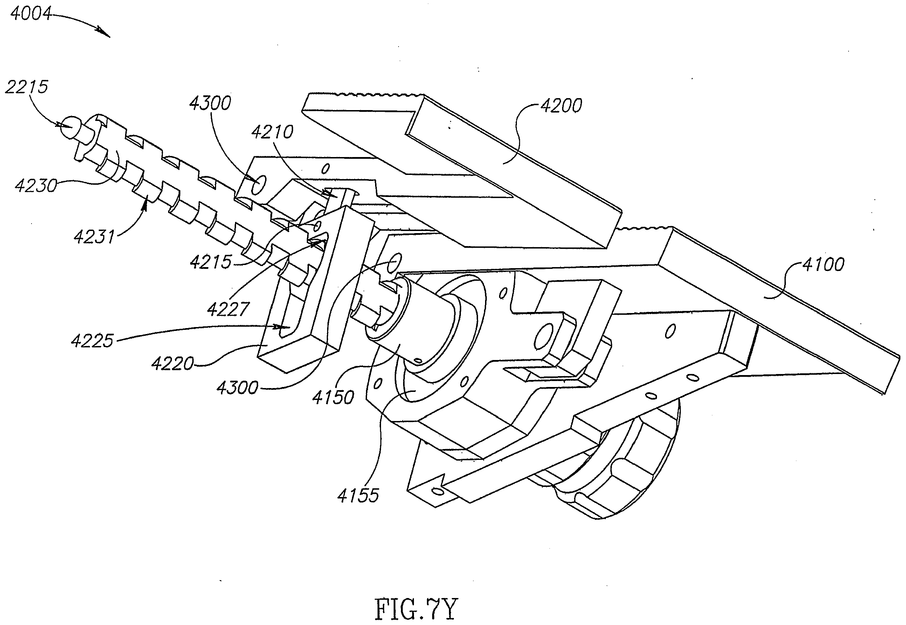

Fig. 7 Y is a perspective view of the exemplary foot activated actuator depicted in Figs. 7V, 7W and 7X from below. This view shows a housing 4155 of the inner threading mechanism which advances shaft 4230 by engaging and turning sectorial threads 4231.

Additional exemplary hydraulic mechanism

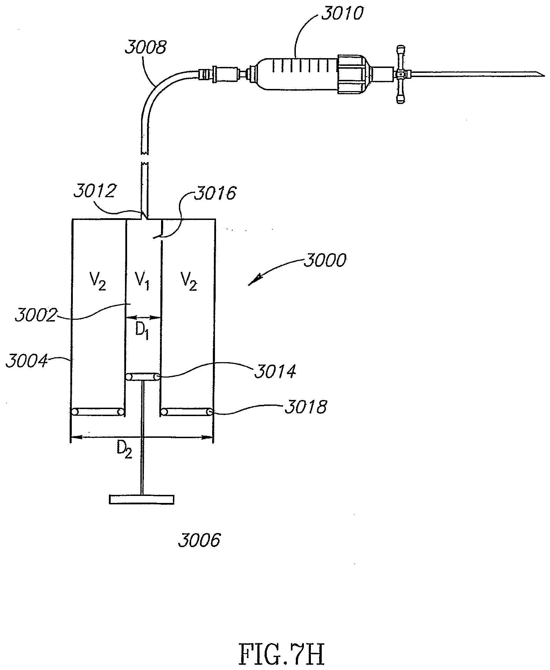

Fig. 7H illustrates an additional embodiment of a hydraulic delivery system according to the invention. In the pictured embodiment, a hydraulic pump 3000 includes a smaller syringe 3002 within a larger syringe 3004. Smaller syringe 3002 having a first volume ($V_1$) volume and a first diameter ($D_1$), and larger syringe 3004 having a second volume ($V_2$) volume and a second diameter ($D_2$), where $V_1 \ll V_2$, and $D_1 \ll D_2$.

In one embodiment of the invention, each activation of an actuator 3006 (e.g., handle rotation or foot pedal pressing) results in injection of defined amount of liquid from smaller syringe 3002 into reservoir 3010, optionally via flexible tube 3008. In an embodiment of the invention, a uni-directional valve 3012, located at the distal end of the small syringe 3002, assures that liquid flows only towards reservoir 3010. When activation of the actuator ceases, piston 3014 of the small syringe 3002 automatically returns to its original position. The automatic return of piston 3014 may be achieved, for example by use of a spring or an elastic band which applies force in a direction opposite to a direction of actuation. A second uni-directional valve 3016 is located in the wall between smaller syringe 3002 and larger syringe 3004. When piston 3014 returns to its original position, a vacuum is created inside syringe 3002. The vacuum and/or a force and/or a spring that presses piston 3018 opens valve 3016 and liquid from larger syringe 3004 flows into the barrel of the small syringe 3002. According to this embodiment of the invention, liquid in larger syringe 3004 serves as a reservoir for the refilling of the small syringe 3002. In an embodiment of the invention, a piston 3018 of larger syringe 3004 advances as V2 decreases. Optionally, this embodiment can provide force amplification (if D1 is greater than an ID of 3010; see detailed explanation below) and/or facilitates delivery of small and/or defined aliquots of liquid upon each activation of actuator 3006.

In many embodiments of the invention, the system is designed to assure that the hands of an operator is outside an X-ray radiation zone of an imaging or monitoring system employed in conjunction with the cement delivery system.

Pressure Amplification

Referring again to Fig. 7G, piston 2024 of pressure source 2004 is characterized by a first diameter (Dl) and piston 2018 of cement reservoir 2002 is characterized by a second diameter D2.

If hydraulic fluid is present in 2026 and in 2028, the two chambers function as a single chamber and no hydraulic amplification is achieved even if D1 and D2 are different.

In an embodiment of the invention, piston 2018 is pushed by a drive shaft (not shown) instead of by hydraulic fluid in 2028. According to this embodiment, hydraulic amplification may be calculated as follows:

The applied force (F) supplied by each piston can be calculated from relevant pressures (P) and diameters (D):

$$F = P*A;$$

where

$$A = \pi * D^2 / 4;$$

thus

$$F = P * \pi * D^2 / 4.$$

If pressure amplification is defined as pressure in cement reservoir 2002 (P2) divided by pressure in pressure source 2004 (P1), the pressure amplification is equal to $(D1/D2)^2$.

In an embodiment of the invention, pressure source 2002 is a designed to be held in one hand, while a second hand operates handle 2012. This design is compatible with an internal diameter D1 of 5 cm. A typical cement reservoir has in internal diameter of 1.8 cm. This exemplary configuration produces a pressure amplification of 7.72.

For foot operated embodiments, D1 maybe considerably larger (e.g. 10 cm, 15 cm, 20 cm or intermediate or larger sizes) and a greater pressure amplification may be achieved.

Alternatively or additionally, mechanical amplification applies as in any manual device that uses a rotational drive with a lever arm (e.g. handle 2012 and cap 2013 in Fig. 7G or lever 4100 and gear 4140 in Fig 7U). These mechanical amplifiers further reduce the amount of input force required to achieve a large force to drive the piston in the cement reservoir (e.g. 2002 or 4005).

Unit material provision system

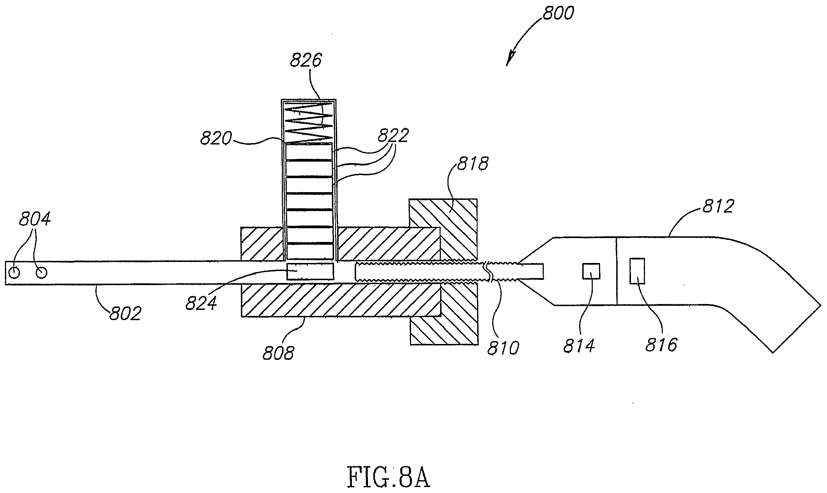

Fig. 8A shows a delivery system 800 in which material is provided as discrete units, each of which is of relatively small volume, for example, 1/2, 1/4, 1/7, 1/0 or less of the amount required for treatment. One potential advantage of working in units is that an operator is more aware of the effect of his/her actions as each action can only inject one unit. Another potential advantage of working in units is that units with different material properties may be provided during a procedure. Another potential advantage is that units being small will generally exhibit a smaller friction with the delivery system.

System 800 comprises a delivery tube 802 having one or more extrusion apertures 804 at its tip. A barrel 808 on which tube 802 is mounted, also includes an optional magazine 820, described below. A body 818 with an optional nut threading is optionally attached to barrel 808. A pusher 810 lies within delivery tube 802 and/or barrel 808.

In an embodiment of the invention, a handle 812 is provided which includes a battery powered mechanism for advancing pusher 810. A hydraulic mechanism such as described above may be used instead. Optionally, one or more switches are provided, for example, an on/off switch 816 and a direction switch 814. Optionally, when pusher 810 completes its forward motion, it is automatically retracted. Optionally, only a single switch is needed, activation of which causes extrusion of one unit, hi an embodiment of the invention, handle 812 is rotationally locked to body 818, for example using one or more guide pins.

In an embodiment of the invention, handle 812 comprises a motor and a battery that rotate pusher 810. An alternative mechanism is described below.

Referring to magazine 820, in an embodiment of the invention, the magazine comprises discrete units 822 of material (a unit 824 is shown inside tube 802). Optionally, a spring 826 is used to push the units towards tube 802. Optionally, the magazine is filled with a contiguous mass of material and the units are defined by the cutting action caused by pusher 810 pushing a unit of material away from the magazine.

In an embodiment of the invention, a magazine is prepared ahead of time, for example, by a manufacturer, who fills the magazine with a non-setting material.

In an embodiment of the invention, the magazine is loaded with a series of units of different properties, for example, responsive to an expected progress of a procedure, for example, first providing a soft material and then providing a harder material, or vice versa. Alternatively, a rotating magazine is used, in which a user can select which of several compartments will load barrel 808 next. This allows fine control over the injected material. In an embodiment of the invention, an operator can remove magazine 820 at any time and replace it with a different magazine. Optionally, this is done while pusher 810 is forward, so that there is no danger of backflow from the body.

Optionally, one or more of the units comprises or is an implant device (rather than an amorphous and/or homogenous mass), for example, an expanding implant or an implant whose geometry does not change. Optionally, one or more of the units comprises a crosslinked material.

In an embodiment of the invention, the delivery system used comprises two or more delivery tubes (optionally the combined geometry has a cross-section of a circle or of a figure eight). Optionally, each tube has a separate pusher mechanism and/or a separate material source (e.g., a magazine). Optionally, the two tubes are used simultaneously. Optionally, an operator can selectively use one tube. Optionally, the materials provided in each tube are components that react chemically one with another. Optionally, electronic control is provided to control the relative provision rates of the two tubes. Optionally, this allows control over the final material properties. Optionally, the use of two or more tubes allows a layered structure to be built up in the body. Optionally, one of the tubes delivers a setting material and the other tube delivers a non-setting material. In an alternative embodiment, each tube is used to provide a different component of a two component material. Optionally, the two tubes meet at their distal end, to ensure mixing of the components.

In an embodiment of the invention, the delivered material is CORTOSS by Orthovita inc. (US)5 a composite of Bis-GMA, Bis-EMA and TEGDMA. This material is optionally mixed along the path in the delivery tube.

In an embodiment of the invention, instead of the units being provided by a magazine or by a cutting mechanism, a partial unit behavior is provided by the motor of handle 812 stopping after every "unit" advance. Optionally, mechanical stops are provided for a hydraulic mechanism, if used. Optionally, instead of stopping, a sound is provided when a unit is injected or based on a different logic, for example, when 50% or another percentage of planned amount of material is provided. Optionally, a CPU is provided which analyzes an image provided by an imaging system and generates a signal when a sufficient and/or near sufficient and/or over-load amount of material is provided. Other circuitry may be used as well.

Optionally, circuitry is provided for controlling the rate and/or pressure of material provision. Optionally, the circuitry stops advancing if a sudden change in resistance is perceived.

In an embodiment of the invention, the delivery system includes pre-heating or pre-cooling of the injected material and/or of tube 802. In an embodiment of the invention, a Peltier cooler and/or a resistance heater are provided in barrel 808. Other cooling or heating methods, such as based on chemical reactions or phase changing materials, may be used.

In an embodiment of the invention, the magazine is a long coiled magazine. Alternatively or additionally, the deformable material is folded in the magazine. Optionally, the magazine is elongated. Optionally, separate loading and pushing mechanism are provided. In an embodiment of the invention, for loading, a unit is inserted through a slot in the side of the barrel. For pushing, the unit is advanced under a low pressure past the slot (or the slot is sealed) and only then is significant pressure required to advance the unit, for example, once the leading edge of the unit reaches the extrusion apertures.

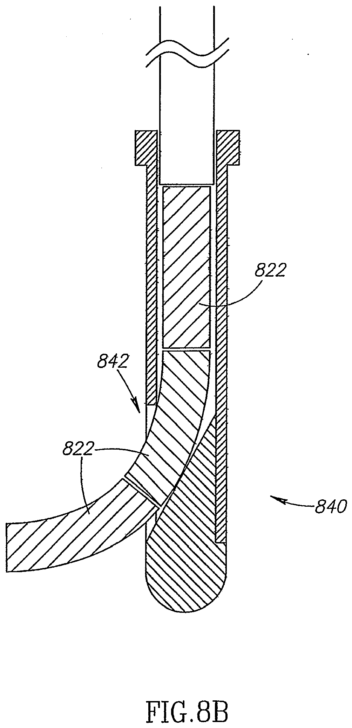

Fig. 8B shows the implementation of a unit delivery method even without a cassette. A delivery tip 840 of the cannula is shown with a lateral aperture 842 through which multiple units 822 are shown exiting. Optionally, an indication is provided to the user as a unit exits, for example, based on motion of a pusher used. Optionally, the system of Fig. 8A is used to load a series of units 822 into the barrel, for example, pulling back the pusher after each unit is advanced past the cassette. In an embodiment of the invention, a distal tip of the cannula is closed, optionally permanently closed, so that cement 822 is forced laterally outwards via aperture 842.

Battery powered pusher

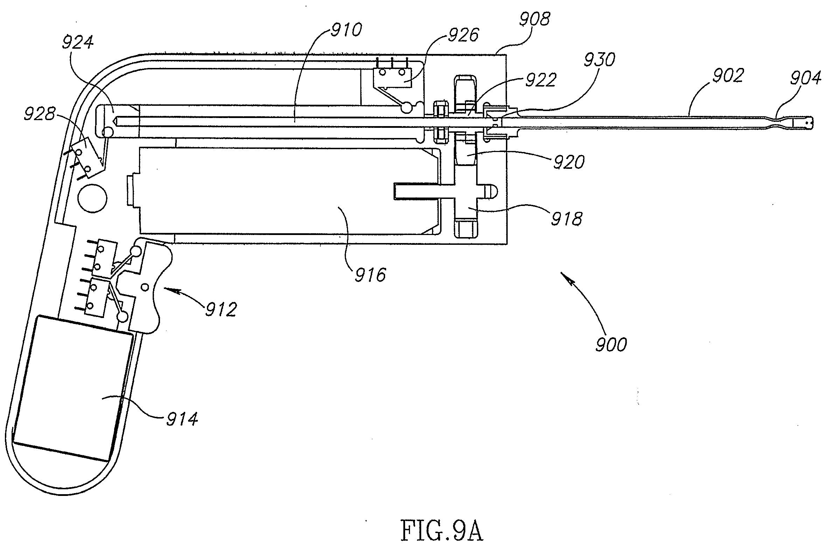

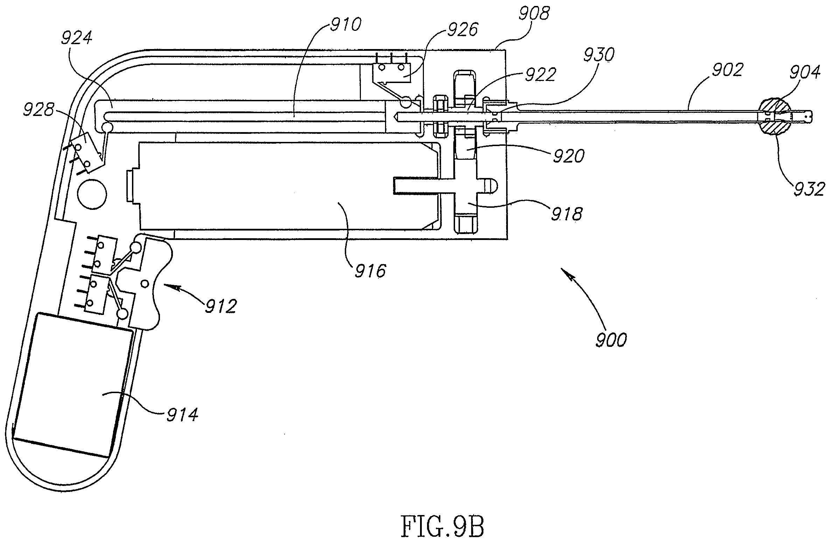

Figs. 9A and 9B show a material pusher 900 with reduced material twisting, in accordance with an embodiment of the invention.

As in the delivery systems described above, pusher 900 comprises a delivery tube 902 having one or more apertures 904 near its end. Optionally, an offset is provided between the apertures and the far tip of tube 902, for example, to ensure centring (or other positioning) of the extruded material, for example preventing the material from being provided too close to a far end of the vertebra, if the delivery system is pushed forward.

Tube 902 is mounted (e.g., optionally replaceably) to a body 908. A pusher 910 is used to advance material through tube 902.

In an embodiment of the invention, in use, an operator presses a switch 912, for example, to select between forward, backwards and no motion of pusher 910. Power from a battery 914 (or a hydraulic or other source) is conveyed to a motor 916. Rotation of the motor causes a nut 922 to rotate relative to pusher 910. Optionally, a series of gears are used which may or may not provide a mechanical advantage, depending on the implementation. In an embodiment of the invention, motor 916 rotates a gear 918 that rotates a gear 920, which rotates nut 922 which is coaxial thereto. Optionally, a rotation preventing element 924, for example, a rectangular element 924 is mounted on pusher 910 and prevents rotation thereof.

Optionally, one or more sensors are used to detect the extremes of positions of pusher 910, when it is advanced and when it is retracted. In the example shown, a micro-switch 926 and a micro-switch 928 detect the ends of motion of pusher 910, for example, using a bump or electrically conducting section 930 (depending on the sensor type used). Alternatively or additionally, a positional encoder is used, for example, by counting rotation, or a separate encoder as known in the art of encoders.

Fig. 9B shows system 900 after extrusion is effected, showing extrusions 932. Optionally, extrusions 932 are an extension to tube 902, prior to them being cut off by pusher 910. In an embodiment of the invention, rotation of tube 902 causes extrusions 932 to act as a reamer. In an embodiment of the invention, the viscosity and shear strength of the material are selected to effect a desired limitation on the reaming abilities, for example, to prevent damage to bone.

Optionally, one or more gears are provided to rotate and/or oscillate the delivery tube as the material is advanced. Optionally, periodic or ramp axial motion is provided, by motor means. Optionally, the distal tip of the delivery tube is made soft, for example by attaching a soft tip thereto, to reduce or prevent damage to the vertebra.

Sleeve provision system

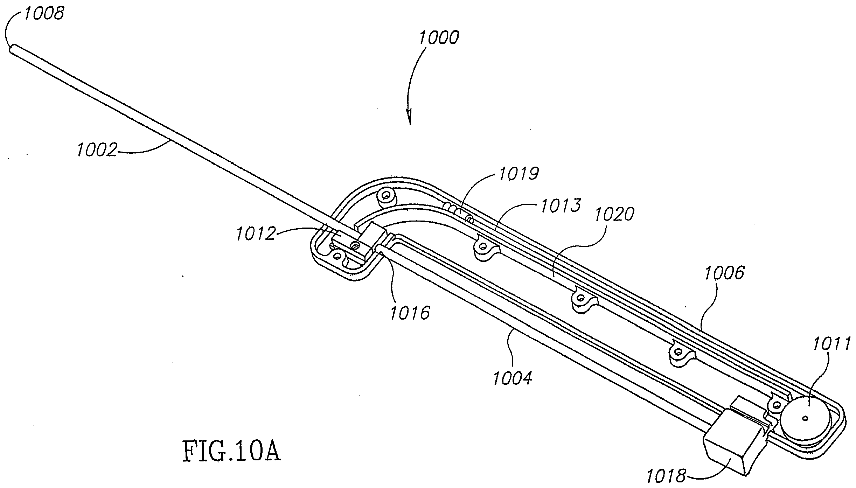

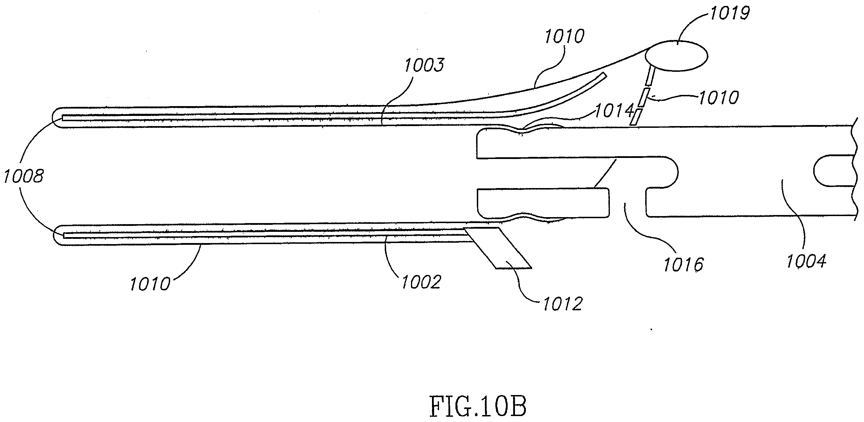

Figs. 10A and 10B shows a sleeve based delivery system 1000, in accordance with an embodiment of the invention. Fig. 10A is a general cut-open view of system 1000, in which a sleeve 1010 is not shown. Fig. 10B shows the distal portion of system 1000, including sleeve 1010 mounted thereon.

The embodiment of Figs. 10A-10B also illustrates a refilling mechanism by which the delivery tube includes a port to which a refill system can be connected to refill the delivery tube with material to be injected into the body.

A pusher 1004 pushes material that is found inside a delivery tube 1002. In the embodiment shown, the material is ejected past a tip 1008 of delivery tube 1002. A sleeve 1010 is provided so that the sleeve lies between the material and delivery tube 1002. An optional tube cutter 1012, such as a knife is shown to optionally split the tube after it exits the body. A pulley system 1011 for collecting the split tube is also shown.

In operation, an amount of material is either provided in tube 1002 or is injected into it, for example, via a port 1016 in pusher 1004. Advancing of pusher 1004, for example, by applying force to a knob 1018 attached thereto, for example manually, using a motor or using other mechanisms described herein, pushes against the material in tube 1002. At the same time, sleeve 1010, which is attached to pusher 1004, for example, by a crimping 1014, is pulled along with the material. Portions of sleeve 1010 reaching distal tip 1008 of tube 1002, fold back towards a body 1006 of delivery system 1000. When sleeve 1010 reaches knife 1012, it is optionally split so that it can pass over tube 1002 and pusher 1004. A thread or wire or other coupling 1013 is attached to the proximal (split) side of sleeve 1010 (e.g., via a connector 1019) and via a pulley 1011 is pulled as pusher 1004 advances. A slide 1020 is optionally provided to guide the motion of the split sleeve It should be appreciated that such a sleeve system can also be used for delivering implants rather than material. In one example, a compressed plastic implant, for example, polyurethane, which is compressed radially (and extended axially) is advanced using a sleeve system, to reduce friction. Optionally, the sleeve material is selected according to the material being used and/or the tube material. In another example, the sleeve system is used to deliver a self-expanding implant, for example, as described in WO-00/44319 or in WO-2004/110300.

It is noted that a sleeve system may also be flexible. Optionally, the sleeve is formed of a chain-link or a knitted material, rather than an extruded plastic polymer tube. Optionally, the sleeve is formed of multiple layers of materials, for example by extrusion or by lamination. Optionally, fibers or other strengthening means are provided to reduce elongation. Optionally, the sleeve is formed of a material that withstands heat and/or chemical byproducts caused by PMMA. Optionally, the sleeve is preformed to elastically expand when it exits the delivery tube. Optionally, the sleeve is perforated or includes a plurality of apertures therein.

Optionally, the sleeve elutes one or more treatment materials. Optionally, the sleeve elutes one or more catalysts or catalysis retarding materials, for example, to prevent or to slow down reactions in the delivery system and/or speed them up out of the delivery system.

Optionally, a layer of oil or other lubricant is provided in addition to or instead of the sleeve.

Optionally, the sleeve remains inside the body, for example, being formed of a bio-degrading materials or maintaining its form. Optionally, when degrading, strengthening fibres or other elements remain to enhance the strength of the extruded material or implant.

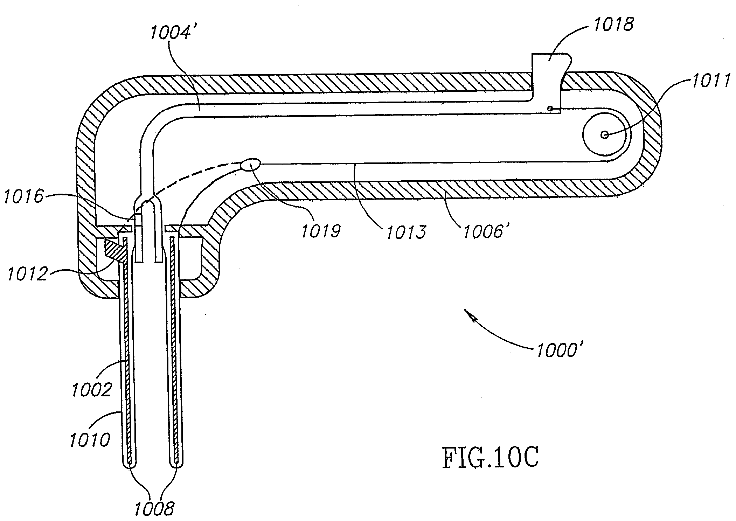

Fig. 10C is a cross-sectional view of a variant system 1000' in which a pusher 1004' is flexible enough to bend. This allows a body 1006' of the device to be manufactured in a nonlinear shape, for example, in the shape of revolver, which may be easier to hold. Optionally, one or more wheels, bearings or slides (not shown) are used to guide pusher 1004'. Optionally, pusher 1004' can be made more flexible as some of the motive force used to move the material is provided by the sleeve pulling the material forward. Alternatively or additionally, some reduction is supported by the reduced friction.

Optionally, a sleeve system is used with a magazine system, for example, the units being provided through port 1016.

Optionally, the sleeve is pre-split and includes an overlap to prevent friction in the delivery tube. Optionally, this allows a magazine to load the sleeve from the side.

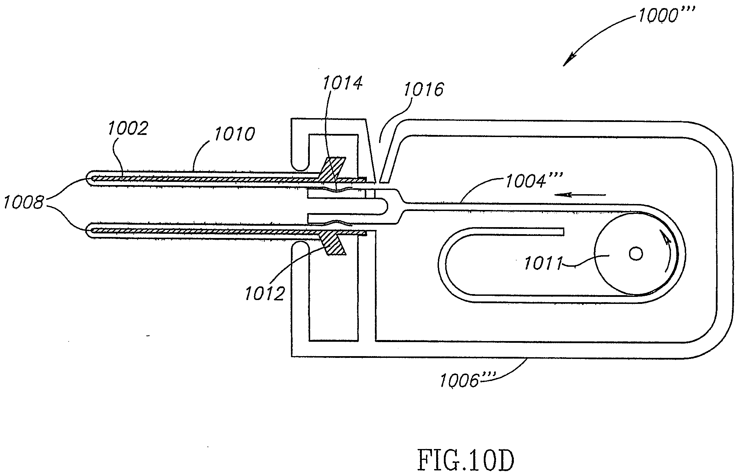

Fig. 10D shows a further, compact, variant 1000" in which a pusher 1004" is made flexible enough to fold over itself, so body 1006" can be of smaller dimensions. It should be noted that these more compact and/or non-linear embodiments can also be practiced without the sleeve feature. The sleeve pullback mechanism is not shown here.

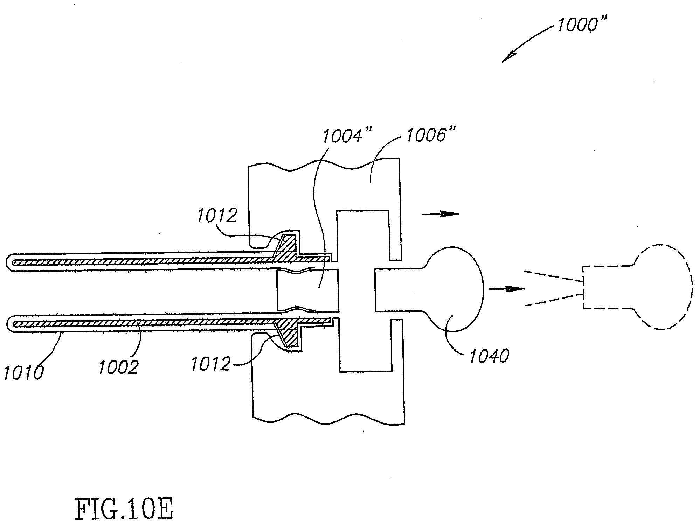

Fig. 10E shows a variant system 1000"' in which a pusher 1004"' is reduced in size axially. In this design the motive force is provided by pulling back the cut sleeve 1010 using a knob 1040 (or a motorized or mechanical gain or other means). This pulling back advances a shortened pusher 1004"'. Optionally, pusher 1004"' is provided as a sealed end of sleeve 1010. A body 1006"' of the system can be very compact, depending on the method of pulling back on knob 1040. Op two or more symmetrically positioned knifes 1012 are provided, to allow for proper mechanical support of tube 1002 by body 1006"'. Optionally, the tube is precut.

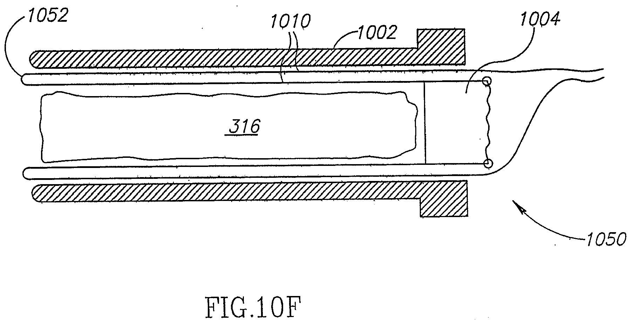

In an embodiment of the invention, it is noted that pusher 1004 is separated from the injected material by the sleeve. Optionally, a hydraulic system is used to advance the pusher, for example (in Fig. 10F) attaching a flexible tube to pusher 1004"' in tube 1002. In an embodiment of the invention, sleeve 1010 is used to isolate the body itself from the hydraulic system, possibly allowing for a system with a higher probability of leaking.

In the embodiments shown, the material exited from the distal end 1008 of tube 1002. Optionally, a stop is provided at the end, so that the material is forced sideways. Optionally, the stop is not attached to tube 1002 at end 1008 thereof. Rather a thread, running through tube 1002 and/or outside thereof (or more than one thread) attaches the stop to the body of device 1000. Optionally, the thread runs through a narrow lumen formed in pusher 1004.

Alternatively, one or more elements which attach the stop to tube 1002, serve to split sleeve 1010, at tip 1008 of tube 1002. In an embodiment of the invention, the stop is attached to tube 1002 after the sleeve is mounted thereon. Alternatively, the sleeve is pre-split, pulled through tube 1002, past the elements and attached to connector 1019.

In an alternative embodiment of the invention, the sleeve is provided totally within the delivery tube. In one embodiment (not shown), the delivery tube comprises two coaxial tubes and the inner tube serves as shown by tube 1002 in Figs. 10A-10E.

In another embodiment, the fact that the delivery tube is full of material is taken advantage of, in that the material (316) serves to prevent the tube from collapsing when it is simultaneously pushed from one end and pulled from the other. This may depend on the viscosity of the material and/or on the shape of the distal tip of the delivery system. Optionally, the distal end is slightly flared to define a folding over location for the sleeve.

Fig. 10F shows such an embodiment, of a delivery system 1050, in which sleeve 1010 is provided within delivery tube 1002. As can be seen a folding over location 1052 for the sleeve is provided past the end of tube 1002. In an embodiment of the invention, a ring (not shown) is provided past the end of tube 1002 and around which the sleeve is folded. This ring serves as a scaffold for the folding, but due to its having a diameter greater than an inner diameter of tube 1002 (or at least being misaligned if the ring and/or tube are not circular in cross-section), cannot be pulled into the tube by retraction of sleeve 1010.

In an alternative embodiment of the invention, sleeve 1010 does not fold back towards system 1000. Rather, the sleeve is pushed into the vertebra with the material. Optionally, once out of the confines of tube 1002, the material can tear the tube. In an alternative embodiment, the sleeve remains intact and encloses the material, sausage-like, in the body. The sleeve may be formed of biocompatible, bioabsorbable and/or implant grade material.

Squeeze based material provision

In an embodiment of the invention, the material is squeezed out of the delivery system rather than pushed. Fig. HA shows a squeeze based system 1100, in which a delivery tube 1102 is made out of a squeezable material, such as a polymer or annealed metal.

A pair of rollers 1104 (or one roller and an opposing anvil, not shown) advance towards the distal side of tube 1102, squeezing it flat and forcing material that fills the tube to migrate distally. Various motion mechanism can be used. In the figure, the motion mechanism is a linear gear 1108 which engages a gear 1106 that is coaxial with roller 1104. When the roller is rotated, the linear gear advances the roller. Various power sources may be used, for example, electric motors and hydraulic power. Also, other power trains may be used. The rollers are optionally made of stainless steel.

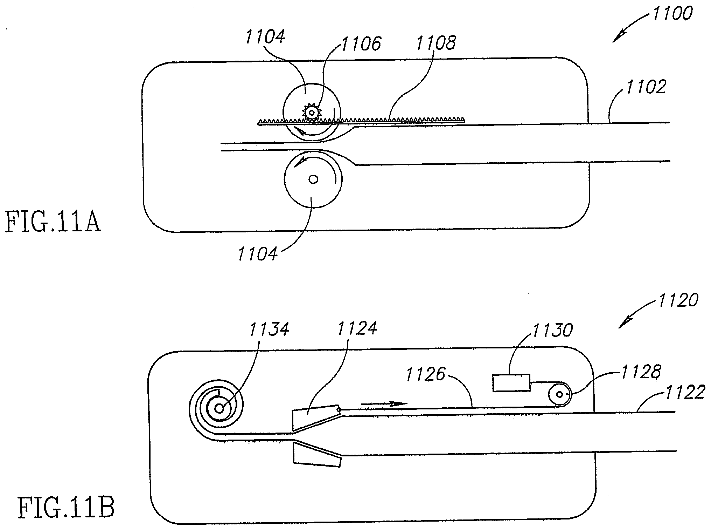

Fig. 11B shows a delivery system 1120, in which a squeeze element 1124 slides rather than rolls against a delivery tube 1122. Tube 1122 is optionally rolled around a pin 1134. Various mechanisms can be used to move squeeze element 1124, for example a motor 1130 attached to a cable 1126 via an optional pulley 1128.

Tamping method

In an embodiment of the invention, friction is reduced by reducing the length of motion of the material inside a delivery tube. In one method, a small amount of material is provided into a distal side of a delivery tube (while outside the body). Then the distal part is inserted into the body and a tamping tool is provided into the proximal part.

This process may be repeated several times until a desired amount of material is provided into the body.

Penetrating delivery system

In some embodiment of the invention, the delivery system also penetrates to the bone and/or penetrates the bone. Optionally, this obviates the need for a separate cannula and/or may simplify the procedure. Optionally, the delivery tube is kept in the body when it is being refilled with material to be injected.

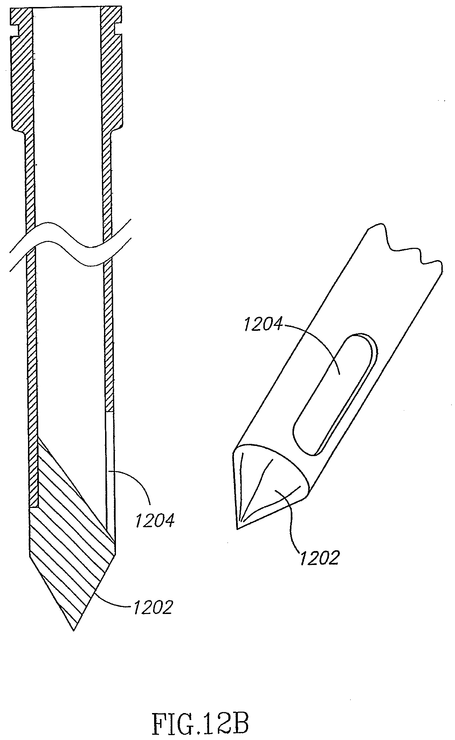

Fig. 12A shows a penetrating delivery system 1200. A distal tip 1202 is formed in a manner suitable for drilling in bone. This is shown in greater detail in Fig. 12B which illustrates one embodiment of a bone cement cannula with a lateral ejection aperture 1204 and a permanently closed distal tip 1202.

A hydraulic pump or mechanical ratchet advance mechanism is optionally used, with a handle 1206 used for pumping shown.

A potential advantage of a one piece system is that fewer parts are needed. If the system is preloaded with all the material needed, for example, at a manufacture, no equipment changes are needed. Optionally, the use of a side aperture 1204 allows the tip to be a drilling tip. Optionally, the use of smaller diameter tubes allows fewer parts to be used, as drilling is simplified.

Optionally, the proximal end of system 1200 is adapted for tapping with a mallet.

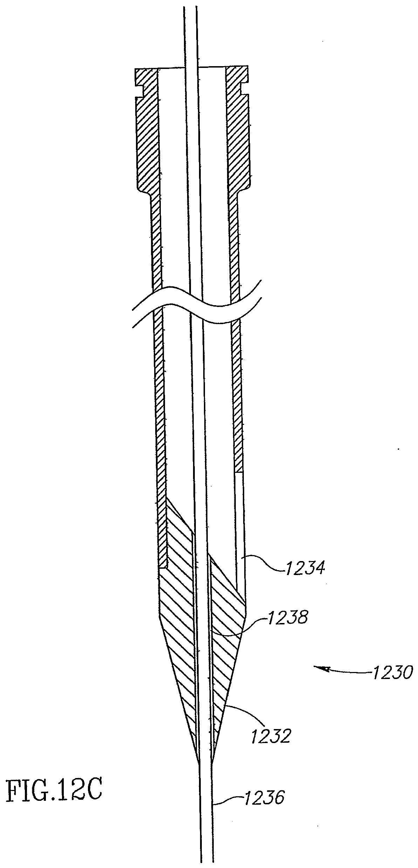

Fig. 12C shows an alternative embodiment of a system, 1230, in which the system is adapted to ride on a guidewire 1236, for example, a K-wire. In an embodiment of the invention, a bore 1238 is formed in a drilling section 1232 of system 1230. Alternatively, the bore is to the side of the drilling head, for example, exiting through an aperture 1234 which may also be used for extruding material. Optionally, the pusher (not shown) is drilled as well. Optionally, the diameters of the drilled holes are too small for the material to exit through. Alternatively, bore 1238 is used for extruding material, after the K-wire is removed.

In an embodiment of the invention, the material is predrilled with a bore, to allow passage of the guidewire therethrough. Optionally, this bore is provided with a sleeve. It is noted that absent axial pressure on the material, the material will generally not flow into the drilled bore. Alternatively or additionally, the guidewire is coated with a suitable friction reducing coating, solid or fluid.

Optionally, the delivery tube is loaded after the delivery tube is guided into the body (and the guidewire removed), for example using a barrel storage means or a unit magazine as described above.

Optionally, a separate lumen is defined for a K-wire. Optionally, that lumen is a collapsible lumen. However, until pressure is applied to the material to be delivered, it remains un-collapsed. Once the guidewire completed its task, it is removed and pressure applied to the material, collapsing the guidewire channel and improving the flow characteristics (by increasing effective inner diameter of the delivery tube.

In an embodiment of the invention, a cannula is not needed, for example, if the delivery system rides on the guidewire or if the delivery system is used to directly penetrate the bone. Optionally, the delivery tube of the delivery system is not removed once inserted into or to the bone, for example, using a barrel or pumping mechanism as described above to reload the delivery mechanism if required. Once the system is reloaded, the pusher can advance the material into the delivery tube where it can then be advanced into the bone.

Mixing Apparatus

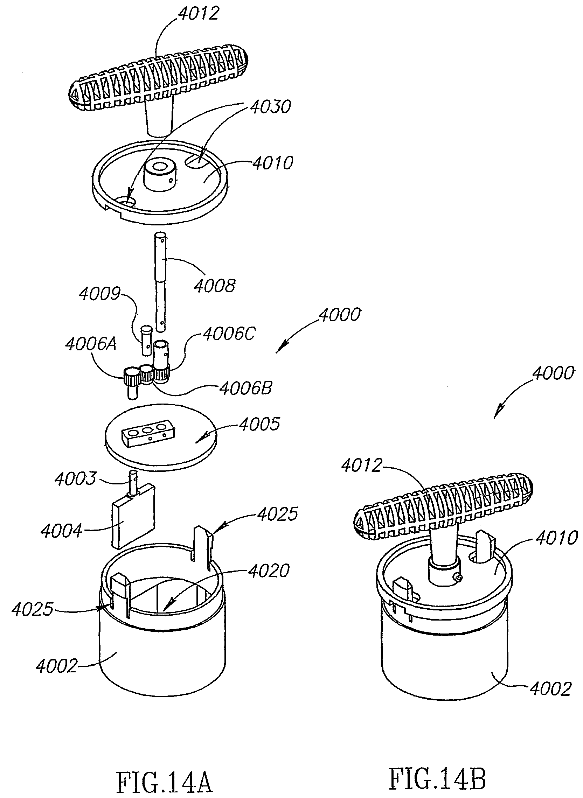

Figs. 14A-14B illustrate an exemplary high shearing force mixing apparatus 4000 adapted for mixing a viscous mixture according to an embodiment of the invention.

Fig. 14A is an exploded view of apparatus 4000 and Fig. 14B is a perspective view of the same apparatus after assembly. In an embodiment of the invention, apparatus 4000 is used for mixing the components of high viscosity bone cement.

In an embodiment of the invention, mixing apparatus 4000 comprises a container 4002, a mixing paddle 4004, a revolving plate 4005 , gears 4006, axles 4008 and 4009, a cover 4010, and a handle 4012. In an embodiment of the invention, mixing element 4004 has a large surface area of 400, optionally 600, optionally 800, optionally 1000 mm$^2$ or intermediate or greater values. Optionally, mixing paddle 4004 is slotted or has holes distributed on its surface. Optionally, during operation mixing implement 4004 applies large shearing forces to a viscous mixture in well 4020. Optionally the large shearing force assures complete mixing of a liquid phase and a solid phase (e.g. powder or beads).

In an embodiment of the invention, paddle 4004 is "wiped" on walls of container 4020. Optionally, shearing forces and stresses may vary with velocity of the revolving and/or paddle surface area and/or cement volume and/or cement viscosity.

In an exemplary use scenario of mixer 4000, the cement components are inserted into a mixing well 4020 of container 4002. The cement components will typically initially include a solid phase (e.g. polymer beads or powder) and a liquid phase.

In an embodiment of the invention, closing cover 4010 by lowering it onto container 4002 so that tabs 4025 are engaged by slots 4030 prevents rotation of cover 4010 with respect to container 4002. Optionally, other rotational locking means are employed.

Revolution of handle 4012 turns axle 4008 and causes revolution of gears 4006A, 4006B and 4006C. In an embodiment of the invention, axle 4008 is rotated by an electric motor, optionally a battery powered motor.

Mixing element 4004 is attached via its axle 4003 to gear 4006A located on revolving plate 4005. When axle 4008 is turned, it causes revolution of gears 4006A, 4006B and 4006C. Revolution of revolving plate 4005 causes axle 4003 of mixing element 4005 to revolve about a center of mixing well 4020. The revolution without rotation of mixing paddle 4004 causes the mixing element to press the mixture against each of the four wells of mixing well 4020 in turn. In an embodiment of the invention, this mixing pattern reduces an amount of un-mixed material on inner walls of well 4020.

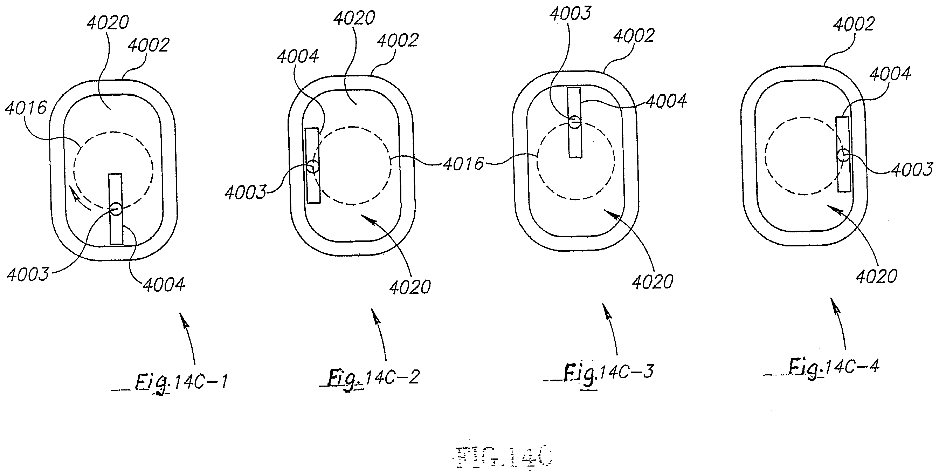

Figs. 14C1, 14C2, 14C3 and 14C4 are top views of mixing well 4020. The described sequential views of paddle 4004 describe how the apparatus successively presses material against walls of the mixing well. In an embodiment of the invention, axle 4003 moves along round path 4016. Gears 4006 A, 4006B and 4006C ensure that paddle 4004 does not rotate around axis 4003. Thus, each of the four sides of the paddle 4004 always faces the same direction (relative to walls of mixing well 4020). In an embodiment of the invention, paddle 4004 revolves without rotation because gears 4006A and 4006C each have the same number of teeth. Gear 4006B is interposed between gears 4006 A and 4006C to cause them to turn in a same direction. Optionally, gear 4006B has any desired number of teeth.

As illustrated in Fig. 14C1, as the paddle 4004 moves from the bottom wall towards the left wall, it applies pressure to a portion of the mixture located near the left wall presses it against the left wall of mixing well 4020(Fig. 14C2). The material being mixed tends to escape towards the upper and lower walls of well 4020. As paddle 4004 continues along its path (Figs. 14C3) it contacts the upper wall of well 4020 and then presses the mixture against the right wall (Fig. 14C4) of well 4020. This mixing pattern provides constant flow of the material being mixed and homogeneous mixing of the mixture components, even at high viscosity.

Mixing apparatus 4000 may be constructed of a wide variety of materials. A choice of construction materials optionally considers the particular type of bone cement to be mixed, its chemical characteristics and/or viscosity. In an embodiment of the invention, mixing well 4020 and/or container 4002 are constructed at least partially of polypropylene and/or nylon. In an embodiment of the invention, paddle 4004 and/or axle 4003 are constructed of stainless steel. Gears 4006A, 4006B and 400C are optionally constructed of plastic and/or metal.

Once mixing is complete, cover 4010 can be opened and the mixed contents can be removed from mixing well 4020.

### Transfer of viscous material

In an embodiment of the invention, mixed viscous bone cement is removed from mixing well 4020 and transferred to a reservoir of a delivery system. Optionally, the reservoir is a cement reservoir as described hereinabove. Optionally, the mixing well serves as a cement reservoir.

In an embodiment of the invention, viscous bone cement is manually manipulated into a delivery system reservoir. Optionally, manual transfer includes shaping. In an embodiment of the invention, viscous bone cement is manually shaped so that it roughly conforms to a configuration of a delivery reservoir. For example, the viscous material may be rolled into a roughly cylindrical form with a diameter slightly smaller than a delivery reservoir into which the material is to be introduced. In an embodiment of the invention, manual transfer includes use of a tool. For example, viscous bone cement is packed into a delivery reservoir using a tool. Optionally, the tool is a rod.

In an embodiment of the invention, viscous bone cement is transferred to a delivery reservoir via an aperture in mixing well 4020. Optionally, the aperture is a lateral aperture in a wall of well 4020. Optionally, the same aperture is used to introduce cement into well 4020. In an embodiment of the invention, the aperture includes a connector connectable to the delivery system reservoir. Optionally, the connector connects the mixing apparatus to the delivery system reservoir while the mixing apparatus operates.

### Transfer apparatus

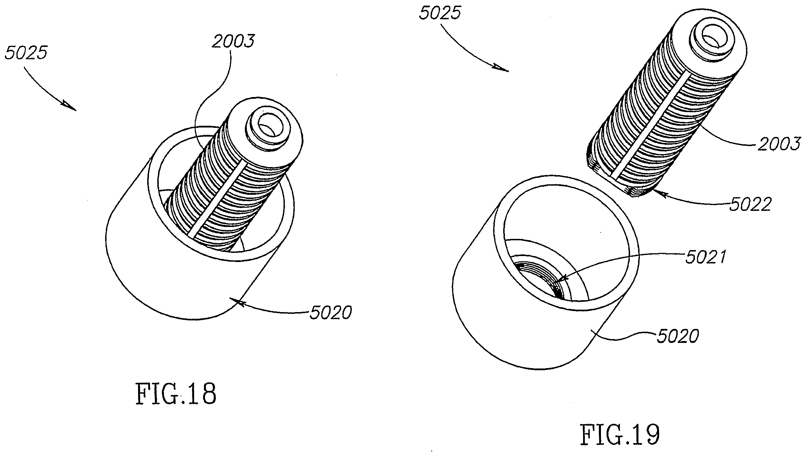

Figs. 18, 19, 20 and 21 illustrate an embodiment of a transfer apparatus 5000 for loading a viscous material into a container. In an embodiment of the invention, the container is a cement reservoir and the material is a viscous bone cement.

Fig. 18 illustrates cement reservoir 2003 assembled. into a transfer piston 5020 to form a transfer assembly 5025.

Fig. 19 illustrates that assembly of cement reservoir 2003 and transfer piston 5020 is optionally by means of matched threads 5021 and 5022.

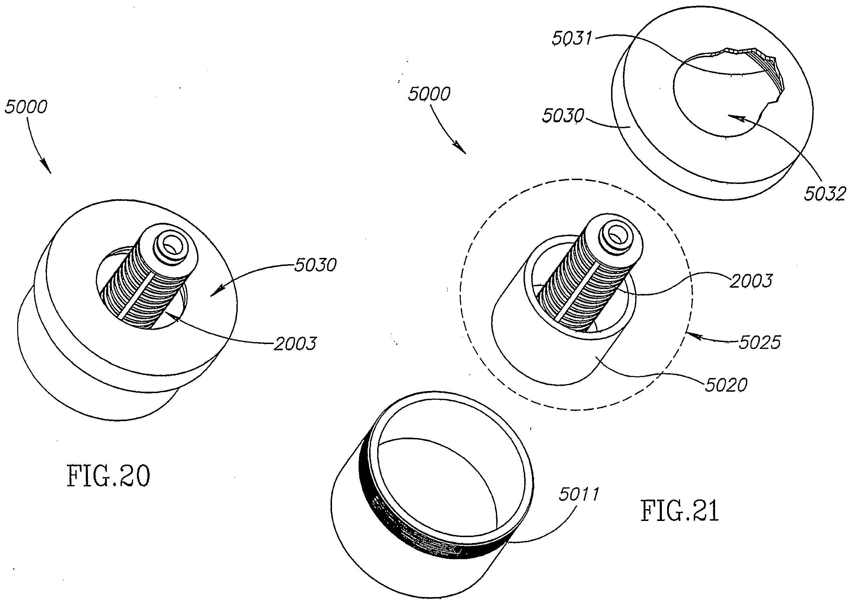

Fig. 21 illustrates assembly of transfer assembly 5025 into a container 5011 of a mixing apparatus. Not pictured is the viscous material (optionally bone cement) in container 5011.

Transfer assembly 5025 is seated in container 5011 so that reservoir 2003 faces outwards. Cover 5030 is optionally applied to container 5011, for example using threads 5031 so that reservoir 2003 protrudes from hole 5032 as seen more clearly in Fig. 20.

Application of pressure to reservoir 2003 and/or an upper edge of piston 5020 causes piston 5020 to descend into container 5011. In an embodiment of the invention, cover 5030 applies pressure to upper edge of piston 502o as it is attached to container 5011. Cement in container 5011 is displaced upwards into reservoir 2003. When the reservoir is sufficiently filled, it is removed from piston 5020. In an embodiment of the invention, the reservoir is transferred to a delivery system as described above.

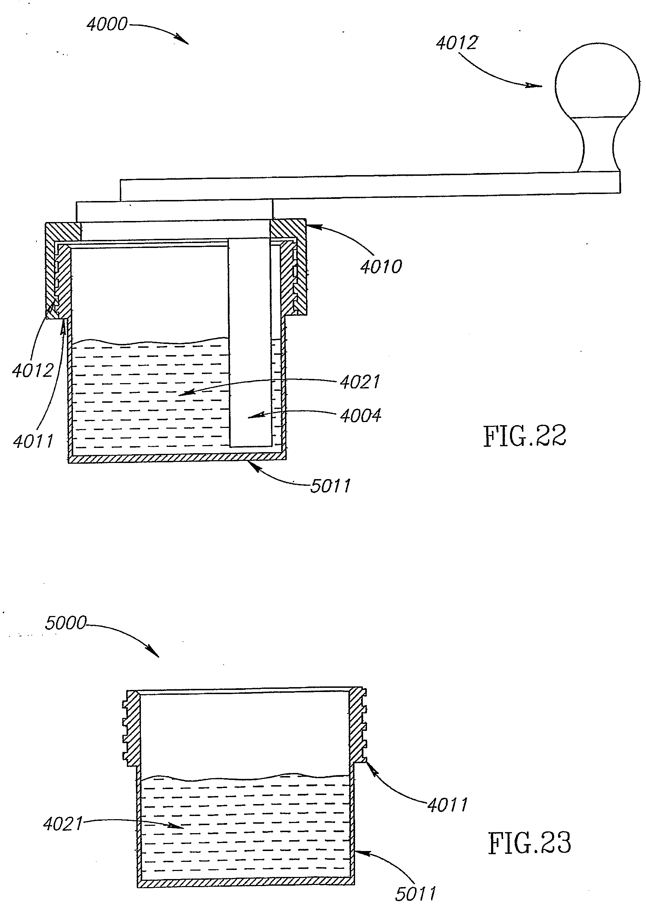

Figs. 22, 23, 24, 25 and 26 illustrate an embodiment of the invention in which container 5011 is a mixing well of a mixer 4000.

Fig. 22 is a cross sectional view of the mixer containing cement 4021. Cover 4010 is held in place by mated threads 4011 and 4012.

Fig. 23 is a cross sectional view of the mixer containing cement 4021 with cover 4010 removed. Mixing well 5011 becomes the basis for transfer apparatus 5000.

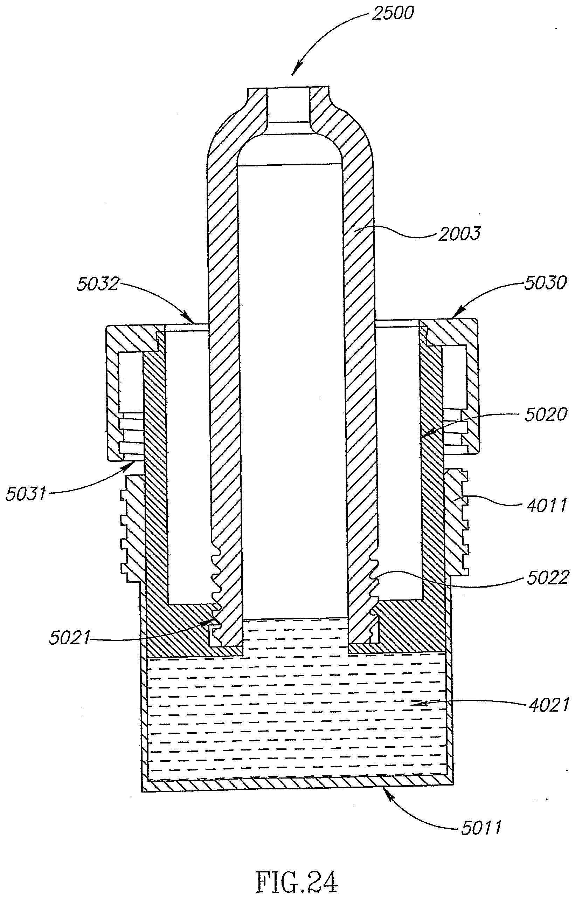

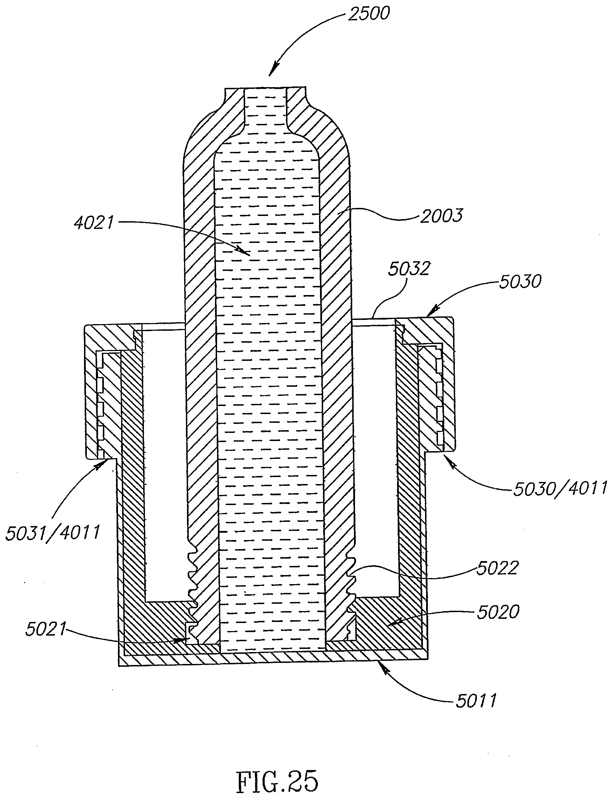

Figs. 24 and 25 are cross sectional views illustrating assembly of cement reservoir 2003 into transfer piston 5020. Optionally, assembly is via mated threads 5022 of reservoir 2003 and 5021 of transfer piston 5020. Cover 5030 is optionally employed to force transfer piston 5020 downwards into mixing well 5011. Cover 5030 may be threaded onto mixing well 5011 via complementary threads 5031 and 4011. In an embodiment of the invention, cover 5030 is screwed onto well 5011 to force piston 5020 downwards onto cement 4021.

Fig. 25 illustrates that downward motion of piston 5020 forces cement 4021 to rise upwards into reservoir 2003 towards aperture 2500.

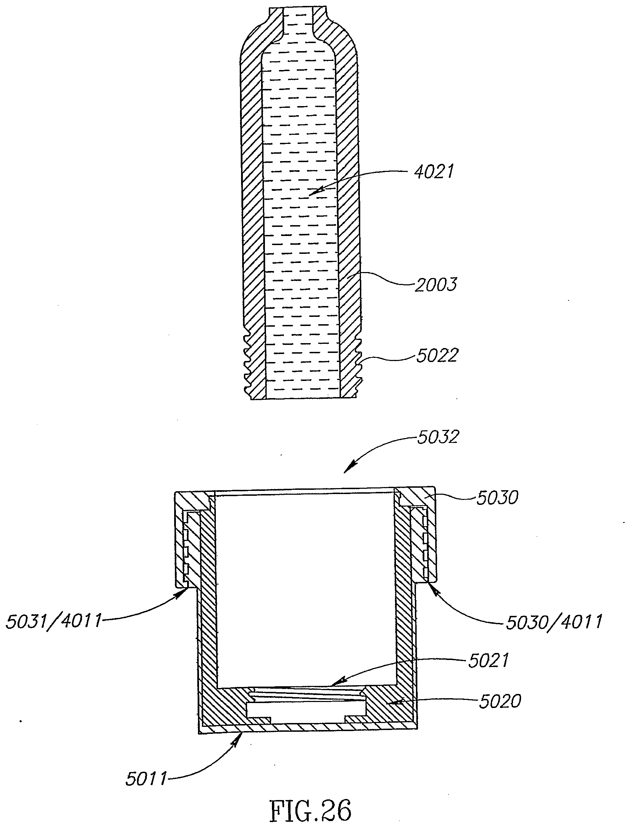

Fig. 26 illustrates removal of reservoir 2003 filled with cement 4022 by disengagement of threads 5022 from matching threads 5021. Optionally, a floor of mixing well 5011 and/or a base of transfer piston 5020 are not straight. In an embodiment of the invention, this reduces an amount of residual cement in well 5011 after transfer.

Optional additional therapy

In an embodiment of the invention, the provision of material is enhanced by additional therapy. Optionally, the additional therapy comprises thermal therapy. Optionally, the material is pre-heated or pre-cooled. Optionally, the pre-heating or pre-cooling also serves a purpose of controlling the material properties and/or setting behaviour.

In an embodiment of the invention, the heating is by contact heat (conduction) or by radiofrequency energy or light, for example a flash lamp or a laser source. Alternatively or additionally, the delivery system radiates heat. Optionally, a microwave or other wireless heating method is used.

Optionally, heating is provided separately from material provision. In one example, a heated guidewire is provided into the vertebra. Optionally, the guidewire extends one or more protrusions, to guide thermal energy into the nearby tissue. Optionally, a thermal sensor is provided to control the temperature in the vertebra and/or prevent over heating.

In an embodiment of the invention, temperature control is applied to increase the handling and/or working time of the bone cement. Optionally, a temperature control unit operates on cement in an external reservoir and/or cement in a delivery system reservoir. In an embodiment of the invention, the temperature control unit includes a resistive coil powered by an electric power source, optionally a battery.

Exemplary Materials

Various materials are suitable for use with embodiments of the invention. Some of the materials which can be used in some embodiments of the invention are known materials, for example, PMMA, however, they may be used at unusual conditions, for example at a semi-hardened condition. Also, while putty materials may be known, they are not typically used for injection through a small bore into bone.

It should be noted that while specific examples are described it is often the case that the material composition will be varied to achieve particular desired mechanical properties. For example, different diagnoses may suggest different material viscosities.

In an embodiment of the invention, for non-hardening materials, the material can be allowed to set outside the body. After such setting the material may be washed or ventilated. In this manner, some materials with potentially hazardous by-products can be safely mixed and then used in the body. Optionally, a material is tested to make sure toxic byproducts are removed to below a safety threshold. Optionally, a testing kit is provided with the delivery system.

In an embodiment of the invention, the material is selected so that its mechanical properties match the bone in which it will be implanted. In an embodiment of the invention, the material is matched to healthy or to osteoporotic trabecular bone. Optionally, the mechanical properties of the bone are measured during access, for example, based on a resistance to advance or using sensors provided through the cannula or by taking samples, or based on x-ray densitometers measurements.

In general, PMMA is stronger and has a higher modulus than trabecular bone. For example, Trabecular bone can have a strength of between 3-20 MPa and a Young's modulus of 100-500 MPa. Cortical bone, for example, has strength values of 170-190 GPa and Young's modulus of 13-40 GPa. PMMA typically has values about half of cortical bone.

In an embodiment of the invention, the material is selected to be less than 120% as strong and/or Young's modulus as the expected bone to be treated. Optionally, the values of one or both of strength and Young's modulus are 10%, 20%, 30%, 40% or less reduced from that of trabecular bone. It should be noted that if less of the vertebra is filled, the injected material will be supported, at least in part, by trabecular rather than cortical bone, depending for example on the method of filing of interior 308.

Exemplary non-hardening material

In an embodiment of the invention, the material used is a putty like material. One example of a putty-like material is a hydroxyapatite with an increased ratio of sodium alginate. For example, the increased ratio can be 8% or 10%. While this material does harden in the body, it does not set to a hardened condition absent humidity. Thus it can be prepared ahead of time and pre-stored in a delivery system, for example by a manufacturer. In an embodiment of the invention, the added material slows down water absorption so that while sufficient water enters the material to initiate setting, not enough enters to cause dissolution. An example of this material is described in Ishikawa et al., "Non-decay fast setting Calcium phosphate cement: Hydroxyapatite putty containing an increased amount of sodium alginate", J Biomed Mater Res 36 1997, 393-399. More details maybe found in "Effects of neutral sodium hydrogen phosphate on setting reaction and mechanical strength of hydroxyapatite putty", by Kunio Ishikawa, Youji Miyamoto, Masaaki Takechi, Yoshiya Ueyama, Kazuomi Suzuki, Masaru Nagayama and Tomohiro Matsumura, in J Biomed Mater Res, 44, 322-329, 1999.

Other calcium derivative cements, bone chips and/or fillers may be used as well. Bone chips, depending on processing may have a limited shelf life. Some of these materials generally harden (or combine with bone growth) after a relatively long time, such as more than a week, more than a month or more than 3 months.

Additional exemplary non-hardening material

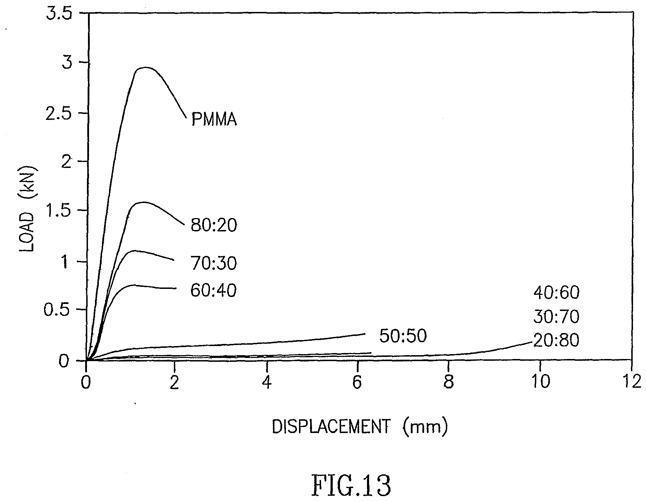

In an embodiment of the invention, the material used is a mixture of LMA (lauryl methacrylate) and MMA (methyl methacrylate). Depending on the ratio used, different mechanical properties and viscosities can be achieved. Fig. 13 is a graph showing the relative viscosities of PMMA and various ratios of the copolymer material. In the example shown, as the ratio of LCA decreases, viscosity goes down.

Diblock copolymers of MMA and LMA were synthesized by anionic polymerization using DPHLi as initiator in THF at -40 [deg.]C with the sequential addition of monomers. The molecular weight distribution of the polymers was narrow and without homopolymer contamination when LMA was added to living PMMA chain ends.

In an embodiment of the invention, the ratio used are 80:20, 70:30, 60:40, 50:50, 30:70, 20:80 or intermediate, smaller or larger ratios (by volume).

Experiment: Materials and Methods

Starting materials

Medicinal distillate methyl methacrylate and lauryl methacrylate stabilized with 10-100 ppm of the monomethyl ether of hydroquinone were used as received from Fluka, Germany. Benzoyl peroxide (BPO) was purchased from BDH Chemicals, England. N barium sulphate (BS) was obtained from Sigma-Aldrich (Israel). All solvents were analytical-grade from Biolab (Jerusalem, Israel) and were used as received.

Polymerization

Polymerization reactions were carried out in a single necked round bottom flask equipped with a magnetic stirrer. In a typical reaction, 60ml MMA (0.565 mol), 50 ml LMA (0.137 mol), 220 mg of benzoyl peroxide (0.9 mmol), and 100 ml THF were transferred. The amount of BPO was adjusted for each of the compositions according to the total amounts of the monomers. The amount of the THF was equal to the total volume of the monomers (table 1). The content was heated to a polymerization temperature of 70-75°C for 20 hours, then the solution was precipitated in sufficient amount of methanol and left to mix for four hours. Finally, the polymer was dried in an oven at 110°C under vacuum.

Table 1: copolymers composition

| Copolymer (MA:LMA) | MA (ml.mol) | LMA (ml.mol) | BPO (mg.mol) | THF (ml) |
|---|---|---|---|---|
| 100:0 | 100 (0.94) | 0 (0) | 285 (1.18) | 100 |
| 80:20 | 80 (0.75) | 20 (0.07) | 258 (1.06) | 100 |
| 70:30 | 70 (0.66) | 30 (0.10) | 239 (0.99) | 100 |
| 60:40 | 60 (0.56) | 40 (0.14) | 220 (0.9) | 100 |
| 50:50 | 5 (0.47) | 50 (0.17) | 201 (0.83) | 100 |
| 40:60 | 40 (0.38) | 60 (0.20) | 182 (0.75) | 100 |
| 30:70 | 30 (0.28) | 70 (0.24) | 163 (0.67) | 100 |
| 20:80 | 20 (0.19) | 80 (0.27) | 144 (0.6) | 100 |
| 0:100 | 0 (0) | 100 (0.34) | 107 (0.44) | 100 |

The dried polymer was milled to a fine powder (Hsiangtai Sample mill, model sm-1, Taiwan) and mixed with barium sulphate (30%w/w). The mixture was heated in a glass inside a sand bath to 140°C, until melting of the polymer. The mixture left to cool, and milled again. This procedure was repeated at least three times, until a homogeneous off-white polymer was received, which could be melted into loadable slugs for the delivery systems and magazines described above.

Characterization

Molecular weight and polydispersity were analyzed by Gel permeation chromatography, GPC system consisting of a Waters 1515 isocratic HPLC pump with a Waters 2410 refractive-index detector and a Rheodyne (Coatati, CA) injection valve with a 20 μl loop (Waters Ma). The samples were eluted with $CHCl_3$ through a linear Ultrastyragel column (Waters; 500 Å pore size) at a flow rate of 1 ml/min.

$^1$H-NMR spectra were recorded on a Varian 300MHz instrument using $CDCl_3$, as solvents. Values were recorded as ppm relative to internal standard (TMS). A Cannon 1C A718 Ubbelhold viscometer was used for the viscosity measurements of the polymer. The measurements were performed at 30°C with toluene as a solvent.

Water Absorption Capacity

Swelling behaviour of acrylic bone cements was carried out from accurately weighed films of 0.8 mm thickness. Films were introduced in 0.9 wt% NaCl solution (20 ml) and kept at 37°C. The water sorption kinetics in 20 ml saline solution were evaluated in two specimens of each bone cement (containing 30% barium sulphate).

Equilibrium gain was determined gravimetrically at different periods of time. The uptake of water was recorded at 30 min intervals in the beginning and spacing out these intervals until the equilibrium was attained. At appropriate times, the samples were removed, blotted with absorbent paper to remove the water attached on its surface and weighed. The percentage of Equilibrium gain was obtained from each specimen using the following expression:

$$Hydration\ Degree\ (\%) = \frac{Weight\ of\ swollen\ specimen - initial\ weight\ of\ specimen}{Initial\ weight\ of\ specimen} \times 100$$

Results:

100% PMMA: Average 1.845% (+0.045)

Initial weight (g): 0.2156 and 0.2211
Weight of specimen at equilibrium (g): 0.2195 and 0.2253
Equilibrium gain (%):1.8 and 1.89;

60% PMMA, 40% PLMA: Average 1.65 %(+0.235)

Initial weight (g): 0.1161 and 0.1402
Weight of specimen at equilibrium (g): 0.1183 and 0.1422
Equilibrium gain (%): 1.42 and 1.89;

50% PMMA, 50% PLMA: Average: 1.02 % (+0.28)

Initial weight (g): 2700 and 0.2371
Weight of specimen at equilibrium (g): 0.2720 and 0.2400
Equilibrium gain (%): 0.74 and 1.3;

Compression Testing

These tests were conducted using an Instron 4301 universal testing machine provided with a load cell of 5 kN, and at a cross-head speed of 20 mm/min. A known weight of polymer was melted in a glass inside a sand bath. The bath was heated at 150°C for two hours, and then barium sulphate was added (30% w/w) and mixed well several times, until homogenous dough was received. Cylindrical specimens of 6 mm in diameter and 12 mm high were prepared by forcing the melted copolymers into the holes of a Teflon mould. One side of the mould was covered with Teflon plates and secured with clamps. The specimens were cooled for 20 minutes in the mould, then the upper side was cut to the mold shape, and the specimens removed from the mould, finished to a perfect cylindrical shape. The test took place at least 1 week after aging in air at 23±1°C. For each cement composition, six specimens were tested. The elastic modulus and the maximal strength force were obtained.

Results:

Molecular Weights and Viscosity Measurement

The number and weight average molecular weights of poly (La-MA), poly (MMA) and their copolymers were obtained from gel permeation chromatography. The polydispersity index varies in the range of 1.6 to 2.87. The viscosities of the polymers are obtained using toluene as solvent at 25°C. The intrinsic viscosities ($\eta$) were obtained by extrapolating $\eta_{sp}\, C^{-1}$ to zero concentration. The molecular weights and viscosities are presented in Table II, below:

| Feed ratio MMA:LMA | NMR analysis | GPC analysis of polymers | | | |
|---|---|---|---|---|---|
| Vol % (mol %) | [MMA]:[LMA] | $M_n$ | $M_w$ | Polydispersity | [$\eta$] |
| 100:0 (100:0) | 100:0 | 65190 | 119544 | 1.833 | 0.544 |
| 8:2 (91.5:8.5) | 88:12 | 69118 | 119194 | 1.724 | 0.421 |
| 7:3 (87:13) | 87:13 | 63006 | 112442 | 1.78 | 0.393 |
| 6:4 (84:16) | 84:16 | 73295 | 118384 | 1.615 | 0.366 |
| 1:1 (74:26) | 69:31 | 94167 | 135880 | 1.44 | 0.351 |
| 4:5 (69:31) | 70:30 | 55455 | 104711 | 1.888 | 0.316 |
| 4:6 (64:36) | 62:38 | 75648 | 134745 | 1.781 | 0.305 |
| 3:7 (56:44) | 56:44 | 35103 | 79986 | 2.27 | 0.221 |
| 2:8 (40:60) | 40:60 | 23876 | 68720 | 2.87 | 0.178 |
| 0:100 (0:100) | 0:100 | 27350 | 75146 | 2.74 | 0.083 |

Compressive Test

The results of the compressive test are collected in Table III below as a function of compressive strength and modulus. The influence on the mechanical behaviour of adding lauryl methacrylate monomers can be clearly observed. The introduction of higher percentages produces a decrease that is more pronounced at 50% (v/v) LA. The compressive modulus shows a drastic decrease as the content of LA increases. This drop may be related to the structure modification of the matrix by the introduction of LMA. This drop may also limit the use of some compositions for some applications.

| Composition MA:LA (v%) | Max strength (MPa) | Modulus (MPa) |
|---|---|---|
| 1:0 | 106.8 (9) | 247 (220) |
| 8:2 | 82.5 (17.1) | 1100.7 (129) |
| 7:3 | 63.3 (13.2) | 634.5 (116) |
| 6:4 | 48 (11) | 550 (250) |
| 5:5 | 18.9 (4.5) | 69.6 (20) |
| 4:6 | 1.9 (0.2) | 49.5 (11.8) |
| 3:7 | 19.19 (3.42) | 8.3 (1.2) |
| 2:8 | 0.253 (0.06) | 1.71 (0.417) |

Material modifications

Optionally, various additives are added to the materials described herein, to modify their properties. The adding can be before setting or after setting, depending on the material. Exemplary materials that can be added include fibres (e.g., carbon nanotubes or glass fibres) of various lengths and thicknesses, aggregates and/or air bubbles.
In an embodiment of the invention, if the material is manufactured to be anisotropic, it can be advanced into the body in a desired direction, for example, by selecting a delivery path (e.g., storage, tube, aperture) to reduce twisting and/or deformation. Optionally, such materials are provided as short units (Fig. 8).

Softening and semi-hardening materials

In an embodiment of the invention, the material used softens after provision into the body. In an embodiment of the invention, the material comprises an additive that disperses or weakness in water or body fluids, for example, salt. A softening material may be useful if the forces required for height restoration are smaller than the forces required for maintaining height. Softening times are optionally controlled by mixing in a gel material which slows down water penetration into the extruded material.

Semi-hardening materials

In an embodiment of the invention, the material used sets to non-hardened condition. In an embodiment of the invention, the material comprises MMA, LMA and NMP. NMP solvates in water, allowing the material to set somewhat. In an embodiment of the invention, a hardened condition is avoided, possibly preventing the induction of fractures in nearby vertebra.

Use of hardening materials

In an embodiment of the invention, the above described devices (e.g., delivery) are used with a material which sets to a hardened condition, for example, PMMA or other bone cements and fillers. In an embodiment of the invention, the material is provided in a kit that includes a timer and/or a viscometer, so that an operator can estimate the workability and viscosity of the material and its usefulness for height restoration without leakage. Optionally, the time includes a temperature senor and provides an estimate of workability time based on the temperature and the time the components of the PMMA were mixed.

In an embodiment of the invention, the cement includes an acrylic polymer, such as polymethylmethacrylate (PMMA). The polymer is supplied as beads. Optionally, styrene may be added. A monomer (e.g. methylmethacrylate ; MMA) is mixed with the polymer beads.

In general bone cements polymerize by radical- initiated addition reactions. In an embodiment of the invention, the cement is prepared from two separate components: a powder component containing pre-polymerised beads (e.g. of PMMA or a PMMA/styrene copolymer) and a liquid component containing monomers (e.g. MMA).

In an embodiment of the invention, an initiator (e.g. benzoyl peroxide (BPO) is incorporated into the powder and a chemical activator (e.g. DMPT) is incorporated into the liquid. Optionally, an easily oxidized molecule (e.g. hydroquinone) is added to the liquid component to prevent spontaneous polymerization during storage.

Optionally, cement may be rendered radiopaque, for example by adding a radio-opaque material such as adding barium sulphate and/or zirconium compounds to the powder and/or liquid component.

Optionally, the average molecular weight of PMMA in all beads is 80,000, optionally 100,000, optionally 120,000, optionally 140,000, optionally 160,000, optionally 180,000 Dalton or intermediate or lesser or greater values. In an embodiment of the invention, the average molecular weight PMMA in all beads is approximately 110,000 Dalton. Optionally, at least some of the beads include styrene. In an embodiment of the invention, styrene is added to PMMA beads in a volumetric ratio of 5-25%. At least some beads contain polymer (e,g. PMMA and/or styrene) with a higher molecular weight, Optionally, the higher molecular weight is 600,000, optionally 900,000, optionally 1,100,000 Dalton or intermediate or lesser or greater values. Optionally, polymer beads (e,g. PMMA and/or styrene) with the higher molecular weight comprise 0.25%, 0.5%, 1%, 2%, 3%, 4%, 5% or intermediate or lesser or higher of the total bead population. In an embodiment of the invention, this type of formulation provides a cement characterized by a short mixing time and/or a cement which achieves a viscosity of 500 to 900 Pa.s in 2 to 3 minutes from the beginning of mixing and/or which remains sufficiently flowable for injection for at least 6 to 10 minutes.

In an embodiment of the invention, higher molecular weight PMMA in the polymer beads cause the mixture to achieve a flowable plastic phase earlier than previously available cements and/or to remain in the flowable plastic phase longer than previously available alternatives. Optionally, altering the percentage of higher molecular weight PMMA in the polymer beads alters a viscosity profile of the resultant mixture.

Optionally, at least one bead of PMMA has molecular weight in the range of 700,000 Dalton to 1,000,000 Dalton. In an embodiment of the invention, approximately 3% of the beads have PMMA characterized by a molecular weight in this range.

In an embodiment of the invention, a setting material is formulated to have a high viscosity for a working window of significant duration, for example, 5, 8, 10 or intermediate or more minutes.

In an embodiment of the invention, the following formulation is used: a set of beads formed of PMMA/styrene of diameter 10-200 μm and an amount of 20 cc MMA per 9.2 grams beads. In an embodiment of the invention, MMA solvates and/or encapsulates the beads and the viscosity of the mixture remains high, at the beginning due to the solvation and friction between the beads and later, as the beads dissolve, due to the progressing of polymerization. The beads may also be provided in a mixture comprising a range of sizes. It should be noted that the properties of the materials may be selected to improve a viscosity working window, even if strength of the final cement is compromised.

In an embodiment of the invention, the working viscosity is set by selecting the bead size and/or material ratios and/or molecular weights of polymer provided in the beads.

In an embodiment of the invention, the working viscosity is influenced by the presence of particles of hardened acrylic polymer added to the mixture.

Mechanical viscosity increasing agents

In an embodiment of the invention, the cement includes particles characterized by a large surface which do not participate in the polymerization reaction. Examples of materials suitable for use as particles characterized by a large surface are which do not participate in the polymerization reaction include, but are not limited to Zirconium, hardened acrylic polymer and bone. Optionally, the particles characterized by a large surface which do not participate in the polymerization reaction are not X-ray transparent so that they aid in visualization of injected cement. In an embodiment of the invention, the large surface area particles impart added viscosity to the cement mixture independent of polymerization. Optionally, the added viscosity comes from friction of particles against one another in the cement.

Polymerization Reaction Kinetics

In an embodiment of the invention, mixture of polymer and monomer components produces a material with a viscosity in the range 500 to 900 Pa.s within 120, optionally within 100, optionally within 60, optionally within 30, optionally within 15 seconds or lesser or greater or intermediate times. In an embodiment of the invention, once a high viscosity is achieved, the viscosity remains stable for 5 minutes, optionally 8 minutes, optionally 10 minutes or lesser or intermediate or greater times. In an embodiment of the invention, stable viscosity indicates a change of 10% or less in two minutes and a change of 20% or less in 8 minutes. The time during which viscosity is stable provides a window of opportunity for performance of a medical procedure.

Material with a glass transition temperature

In an embodiment of the invention, a bone cement includes a material characterized by a glass transition temperature higher than 37°C. Heating such a material above its glass transition temperature, weakens the material. The weakening transforms the material to a dough-like or putty-like state. In an embodiment of the invention, the dough-like material is suitable for delivery using a delivery system as disclosed herein. After delivery, the dough cools to 37°C and hardens. Examples of materials with a glass transition temperature above 37 degrees include, but are not limited to, polycaprolactone (PCL) and/or Polylactic acid (PLA). Polymers with glass transition temperatures suitable for use in the context of the invention are commercially available, for example "Lactel absorbable polymers" (Curect Corp.; Pelham, AL, USA).

In an embodiment of the invention, a material with a glass transition temperature above 35, optionally 40, optionally 45, optionally 50, optionally 55, optionally 60°C is selected.

Optionally, the delivery system is heated to maintain the material above the glass transition temperature. In an embodiment of the invention, a heating element is provided in and/or adjacent to the cement reservoir and/or the cannula.

Use of bone in bone cement

In an embodiment of the invention, the bone cement or other dough-like material includes processed bone (from human or animals origin) and/or synthetic bone. Optionally, the cement has osteoconductive and/or osteoinductive feature. Optionally, the processing of the bone includes grinding. One of ordinary skill in the art will be capable of processing bone using known methods for use in the context of the present invention.

In an embodiment of the invention, the bone cement comprises 50%, optionally 60 % optionally 70% or intermediate or greater percentages of bone powder and/or granules and/or chips.

Additional implant devices

Optionally, an implant is also injected into the vertebra, for example, before, during or after injection of the material. Exemplary implants are metal or polymer cage or intra ventricular devices and enclosing mesh or solid bags or balloons. Optionally, bone graft is injected. Optionally, where an implant is provided, the material is extruded through the implant, for example from an axial section thereof in a radial direction.

Optionally, devices such as, for example, those described in PCT applications PCT/IL00/0458, PCT/IL00/0058, PCT/IL00/0056, PCT/IL00/0055, PCT/IL00/0471, PCT/IL02/0077, PCT/IL03/0052, and PCT/IL2004/00508, PCT/IL2004/00527 and PCT/IL2004/00923 are used.

Optionally, the material is extruded into a performed cavity, for example a cavity formed using an inflatable balloon. Optionally, the material is extruded into an inter-vertebral space, for example a disc-space.

Optionally, a material which sets to a hardened condition, for example, PMMA is co- extruded with or extruded before or after material which does not so set. Optionally, the setting material comprises less than 60% of the material, for example, less than 40%, less than 20% or intermediate values.

Other tissue and general

While the above application has focussed on the spine, other tissue can be treated as well, for example, compacted tibia plate and other bones with compression fractures and for tightening implants, for example, hip implants or other bone implants that loosened, or during implantation. Optionally, for tightening an existing implant, a small hole is drilled to a location where there is a void in the bone and material is extruded into the void.

It should be noted that while the use in bones of the above methods and devices provide particular advantages for bone and vertebras in particular, optionally, non-bone tissue is treated, for example, cartilage or soft tissue in need of tightening. Optionally, the delivered material includes an encapsulated pharmaceutical and is used as a matrix to slowly release the pharmaceutical over time. Optionally, this is used as a means to provide anti-arthritis drugs to a joint, but forming a void and implanting an eluting material near the joint. A bone cement according to the invention may be injected into a bone void as a preventive therapy and/or as a treatment for a fracture, deformity, deficiency or other abnormality. Optionally, the bone is a vertebral body and/or a long bone. In an embodiment of the invention, the cement is inserted into the medullary canal of a long bone. Optionally, the cement is moulded into a rod. In an embodiment of the invention, the rod serves as an intra-medular nail.

It will be appreciated that the above described methods of implanting and treating may be varied in many ways, including, changing the order of steps, which steps are performed more often and which less often, the arrangement of elements, the type and magnitude of forces applied and/or the particular shapes used.. In particular, various tradeoffs may be desirable, for example, between applied forces, degree of resistance and forces that can be withstood. Further, the location of various elements may be switched, for example, the location of the power source. In addition, a multiplicity of various features, both of method and of devices have been described. It should be appreciated that different features may be combined in different ways. In particular, not all the features shown above in a particular embodiment are necessary in every similar embodiment of the invention. Further, combinations of the above features are also considered to be embodiments of the invention. In addition, some of the features of the invention described herein may be adapted for use with prior art devices, in accordance with other embodiments of the invention. The particular geometric forms used to illustrate the invention should not be considered limiting the invention in its broadest aspect to only those forms, for example, where a cylindrical tube is shown, in other embodiments a rectangular tube may be used. Although some limitations are described only as method or apparatus limitations, the invention also includes apparatus programmed and/or designed to carry out the methods.

Also within the invention are surgical kits which include sets of medical devices suitable for implanting a device or material and such a device. Section headers are provided only to assist in navigating the application and should not be construed as necessarily limiting the contents described in a certain section, to that section. Measurements are provided to serve only as exemplary measurements for particular cases, the exact measurements applied will vary depending on the application. When used in the following claims, the terms "comprises", "comprising", "includes", "including" or the like means "including but not limited to".

**Claims**

1. A bone cement comprising:

a first powder component which contains pre-polymerised beads having a first average molecular weight and including a high molecular weight population of beads having a second average molecular weight that is higher than the first average molecular weight, and
a second liquid component which contains a monomer, **characterised in that** the first average molecular weight, the second average molecular weight, and the percentage of beads having the second average molecular weight, are selected so that contacting the first powder component and the second liquid component produces a mixture having a viscosity profile which includes an initial period, a working period, and a hardening period, in which:

(a) during the initial period, the viscosity increases over time until the mixture attains a viscosity greater than 200 Pascal.seconds within 1 minute from mixing the first and second components,
(b) during the working period of at least 5 minutes, the viscosity of the mixture remains between 200 and 2000 Pascal.seconds and changes by less than 10% in a period of 2 minutes within said working time,

the viscosity being measured using a capillary extrusion rheometer for measuring an applied force acting on a piston to displace the mixture in a capillary tube at a constant displacement rate, the measurements being performed at an average temperature of 22.3°C and a constant displacement rate of 3 mm.min$^{-1}$, and in which the mixture is suitable for *in vivo* use.

2. The bone cement of claim 1, in which the first powder component includes an acrylic polymer, preferably poly(methyl methacrylate) (PMMA).

3. The bone cement of claim 1 or 2, in which the second liquid component includes MMA.

4. The bone cement of claim 3, in which the first powder component includes PMMA, barium sulfate, and benzoyl peroxide (BPO), and in which the second liquid component includes MMA, DMPT, and hydroquinone.

5. The bone cement of any of claims 2 to 4, in which the first average molecular weight of PMMA is at least 80,000 Dalton.

6. The bone cement of any of claims 2 to 4, in which the second average molecular weight of PMMA in the high molecular weight population is at least 600,000 Dalton.

7. The bone cement of claim 1, further comprising processed bone and/or synthetic bone that is mixed together with the first component and the second component.


**Patentansprüche**

1. Knochenzement, der Folgendes umfasst:

eine erste Pulverkomponente, welche pre-polymerisierte Kügelchen mit einem ersten mittleren Molekulargewicht enthält und einen hohe Molekulargewichtsbestand von Kügelchen mit einem zweiten mittleren Molekulargewicht aufweist, welches höher ist als das erste mittlere Molekulargewicht, und
eine zweite flüssige Komponente, die ein Monomer enthält,

**dadurch gekennzeichnet, dass**
das erste mittlere Molekulargewicht, das zweite mittlere Molekulargewicht und der Anteil von Kügelchen mit dem zweiten mittleren Molekulargewicht so ausgewählt sind, dass ein Kontakt der ersten Komponente und der zweiten Komponente eine Mischung hervorbringt mit einem Viskositätsprofil, welches eine anfängliche Zeitdauer, eine Verarbeitungszeitdauer und eine Aushärtungszeitdauer aufweist, wobei:

(a) während der anfänglichen Zeitdauer die Viskosität über die Zeit anwächst bis die Mischung eine Viskosität von mehr als 200 Pa.s innerhalb einer Minute vom Mischen der ersten und zweiten Komponente erreicht,
(b) während der Verarbeitungszeitdauer von wenigstens fünf Minuten die Viskosität der Mischung zwischen 200 und 2.000 Pa.s bleibt und sich um weniger als 10% verändert während einer Zeitdauer von 2 Minuten innerhalb der Verarbeitungszeit,

wobei die Viskosität unter Verwendung eines kapillaren Extrusionsrheometers zum Messen einer angewandten Kraft gemessen wird, die auf einen Kolben wirkt, um die Mischung in einer Kapillarröhre mit einer konstanten Verschiebungsrate zu verschieben, wobei die Messungen bei einer mittleren Temperatur von 22,3°C und einer konstanten Verschiebungsrate von 3 mm.min$^{-1}$ ausgeführt werden, und wobei die Mischung zur Verwendung *in vivo* geeignet ist.

2. Knochenzement nach Anspruch 1, in welchem die erste Pulverkomponente ein Acrylpolymer enthält, vorzugsweise Poly(Methyl-Methacrylat) (PMMA) .

3. Knochenzement nach Anspruch 1 oder 2, in welchem die zweite flüssige Komponente MMA enthält.

**4.** Knochenzement nach Anspruch 3, in welchem die erste Pulverkomponente PMMA, Bariumsulfat und Benzylperoxid (BPO) enthält, und in welchem die zweite flüssige Komponente MMA, DMPT und Hydroquinon enthält.

**5.** Knochenzement nach Anspruch 2 bis 4, in welchem das erste mittlere Molekulargewicht von PMMA wenigstens 80.000 Dalton aufweist.

**6.** Knochenzement nach Anspruch 2 bis 4, in welchem das zweite mittlere Molekulargewicht von PMMA in der hohen Molekulargewichtspopulation wenigstens 600.000 Dalton aufweist.

**7.** Knochenzement nach Anspruch 1, der weiterhin bearbeitete Knochen und/oder synthetische Knochen umfasst, der mit der ersten und der zweiten Komponente vermischt ist.

**Revendications**

**1.** Ciment osseux comprenant :

un premier composant poudreux qui contient des perles pré-polymérisées présentant un premier poids moléculaire moyen et comprenant une population de perles à poids moléculaire élevé présentant un second poids moléculaire moyen qui est supérieur au premier poids moléculaire moyen, et
un second composant liquide qui contient un monomère,

**caractérisé en ce que**
le premier poids moléculaire moyen, le second poids moléculaire moyen, et le pourcentage de perles présentant le second poids moléculaire moyen sont sélectionnés de sorte que l'entrée en contact entre le premier composant poudreux et le second composant liquide produise un mélange présentant un profil de viscosité qui comprend une période initiale, une période de travail, et une période de durcissement, dans lesquelles :

(a) pendant la période initiale, la viscosité augmente avec le temps jusqu'à ce que le mélange atteigne une viscosité supérieure à 200 Pa.s moins d'une minute à partir du mélange des premier et second composants,
(b) pendant la période de travail d'au moins 5 minutes, la viscosité du mélange reste comprise entre 200 et 2 000 Pa.s, et change de moins de 10% dans une période de 2 minutes au cours dudit temps de travail,

la viscosité étant mesurée au moyen d'un rhéomètre à extrusion capillaire permettant de mesurer une force appliquée agissant sur un piston pour déplacer le mélange dans un tube capillaire à une vitesse de déplacement constante, les mesures étant effectuées à une température de 22.3°C et à une vitesse de déplacement constante de 3 mm.min$^{-1}$, et dans lequel le mélange est adapté à une utilisation *in vivo*.

**2.** Ciment osseux selon la revendication 1, dans lequel
le premier composant poudreux comprend un polymère acrylique, de préférence du poly(méthyl méthacrylate) (PMMA).

**3.** Ciment osseux selon la revendication 1 ou 2, dans lequel le second composant liquide comprend du MMA.

**4.** Ciment osseux selon la revendication 3, dans lequel le premier composant poudreux comprend du PMMA, du sulfate de barium, et du peroxyde de benzoyl (BPO), et dans lequel le second composant liquide comprend du MMA, du DMPT, et de l'hydroquinone.

**5.** Ciment osseux selon l'une quelconque des revendications 2 à 4, dans lequel la première masse moléculaire moyenne du PMMA est au moins 80,000 Dalton.

**6.** Ciment osseux selon l'une quelconque des revendications 2 à 4, dans lequel la deuxième masse moléculaire moyenne du PMMA, dans la population de poids moléculaire élevé, est au moins 600,000 Dalton.

**7.** Ciment osseux selon la revendication 1, comprenant de plus de l'os traité ou de l'os synthétique qui est mélangé avec le premier composant et le deuxième composant.

100

```
        ┌──────────────────────┐
        │    IDENTIFY  BONE     │───102
        └──────────────────────┘
                   │
        ┌──────────────────────┐
        │     BONE  ACCESS      │───104
        └──────────────────────┘
                   │
        ┌──────────────────────┐
        │   INJECT  MATERIAL    │───106
        └──────────────────────┘
                   │
        ┌──────────────────────┐
        │      (OPTIONAL)       │───108
        │   RESTORE  HEIGHT     │
        └──────────────────────┘
                   │
        ┌──────────────────────┐
        │    END  PROCEDURE     │───110
        └──────────────────────┘
```

FIG.1A

*122* — FORM PASSAGE TO BONE

*124* — PENETRATE CORTEX WITH GUIDE WIRE

*126* — RETRACT GUIDE WIRE (OPTIONAL)

*128* — ADVANCE DRILL INTO BONE

*130* — ADVANCE CANULA TO BONE

*132* — REMOVE GUIDE WIRE, DRILL

*134* — INSERT DELIVERY SYSTEM

*136* — INJECT

*138* — FEEDBACK

*140* — REFILL (OPTIONAL)

*142* — CHANGE DIRECTION

*144* — TOP OFF (OPTIONAL)

*146* — COMPLETE PROCEDURE

*120*

FIG.1B

FIG.2

FIG.3A

FIG.3B

FIG.3C

FIG.3D

FIG.3E

EP 2 319 439 B1

400

410

412

404 402 406

408

FIG.4A

EP 2 319 439 B1

FIG.4B

FIG.5A

FIG.5B

FIG.5C

FIG.6A

FIG.6B

FIG.6C

EP 2 319 439 B1

FIG.7A

EP 2 319 439 B1

744

742

720'

714'

740

FIG.7B

FIG.7C

FIG.7D

FIG.7E

FIG.7F

FIG.7G

FIG.7H

FIG.7I

FIG.7J

FIG.7K

FIG.7L

FIG.7M

FIG.7N

FIG.7O

FIG.7P

FIG.7Q

FIG.7R

2508

2410

2411

2400

2003

FIG.7S

2500

2508

2410

2411

2400

2003

FIG.7S1

FIG.7T

FIG.7U

FIG.7V

EP 2 319 439 B1

FIG.7W

FIG.7X

FIG.7Y

EP 2 319 439 B1

FIG.8A

*822*

*842*

*822*

*840*

FIG.8B

FIG.9A

FIG.9B

EP 2 319 439 B1

FIG.10A

FIG.10B

70

FIG.10C

EP 2 319 439 B1

FIG.10D

EP 2 319 439 B1

FIG.10E

EP 2 319 439 B1

FIG.10F

FIG.11A

FIG.11B

FIG.12A

FIG.12B

FIG.12C

FIG.13

4012

4030

4010

4008

4009

4006C

4006A

4006B

4000

4000

4005

4003

4012

4004

4025

4010

4020

4025

4002

4002

FIG.14A          FIG.14B

EP 2 319 439 B1

Fig.14C-1

Fig.14C-2

Fig.14C-3

Fig.14C-4

FIG.14C

FIG.15

FIG.16

FIG.17

FIG.18

FIG.19

5031

5030

5032

5000

5000

5030

2003

2003

5025

5020

5011

FIG.20

FIG.21

EP 2 319 439 B1

FIG.22

FIG.23

FIG.24

FIG.25

FIG.26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4969888 A
- US 5108404 A
- US 6383188 B
- US 20030109883 A
- US 2002068974 A
- US 6348055 B
- US 6383190 B
- US 4494535 A
- US 4653489 A
- US 4653487 A
- US 6241734 B
- US 6613054 B
- US 2004260303 A
- US 4341691 A
- WO 0044319 A
- WO 2004110300 A
- IL 000458 W
- IL 000058 W
- IL 000056 W
- IL 000055 W
- IL 000471 W
- IL 020077 W
- IL 030052 W
- IL 200400508 W
- IL 200400527 W
- IL 200400923 W

### Non-patent literature cited in the description

- **NUSSBAUM DA et al.** The Chemistry of Acrylic Bone Cement and Implication for Clinical Use in Image-guided Therapy. *J Vase Interv Radiol,* 2004, vol. 15, 121-126
- **G BAROUD et al.** Injection biomechanics of bone cements used in vertebroplasty. *Bio-Medical Materials and Engineering,* 2004, vol. 00, 1-18
- The viscosity of acrylic bone cements. *Journal of Biomedical Materials Research,* 1982, vol. 16, 219-243
- **ISHIKAWA et al.** Non-decay fast setting Calcium phosphate cement: Hydroxyapatite putty containing an increased amount of sodium alginate. *J Biomed Mater Res,* 1997, vol. 36, 393-399
- **KUNIO ISHIKAWA ; YOUJI MIYAMOTO ; MASAAKI TAKECHI ; YOSHIYA UEYAMA ; KAZUOMI SUZUKI ; MASARU NAGAYAMA ; TOMOHIRO MATSUMURA.** Effects of neutral sodium hydrogen phosphate on setting reaction and mechanical strength of hydroxyapatite putty. *J Biomed Mater Res,* 1999, vol. 44, 322-329